(19) Europäisches Patentamt / European Patent Office / Office européen des brevets

(11) **EP 4 763 838 A1**

(12) **EUROPEAN PATENT APPLICATION**
published in accordance with Art. 153(4) EPC


(43) Date of publication:
**24.06.2026 Bulletin 2026/26**

(21) Application number: **24853544.5**

(22) Date of filing: **29.07.2024**

(51) International Patent Classification (IPC):
***C07D 401/04*** *(2006.01)* ***C07D 401/14*** *(2006.01)*
***C07D 487/10*** *(2006.01)* ***A61K 31/501*** *(2006.01)*
***C07D 495/14*** *(2006.01)* ***A61K 31/551*** *(2006.01)*
***A61K 31/506*** *(2006.01)* ***A61P 19/08*** *(2006.01)*
***A61P 7/06*** *(2006.01)* ***A61P 37/02*** *(2006.01)*
***A61P 31/00*** *(2006.01)* ***A61P 29/00*** *(2006.01)*
***A61P 13/12*** *(2006.01)* ***A61P 3/14*** *(2006.01)*

(86) International application number:
**PCT/CN2024/108163**

(87) International publication number:
**WO 2025/036132 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.08.2023 CN 202311015401**



(71) Applicant: **Ocean University of China**
**Qingdao, Shandong 266100 (CN)**

(72) Inventors:
- **QIN, Chong**
  **Qingdao, Shandong 266101 (CN)**
- **WANG, Xiao**
  **Qingdao, Shandong 266101 (CN)**
- **ZHONG, Tengjiang**
  **Qingdao, Shandong 266101 (CN)**
- **WANG, Tao**
  **Qingdao, Shandong 266101 (CN)**

(74) Representative: **Huang, Liwei**
**Cäcilienstraße 12**
**40597 Düsseldorf (DE)**


(54) **COMPOUND AND USE THEREOF**


(57) The present disclosure relates to a cereblon (CRBN) small molecule ligand compound based on a pyridazine-glutarimide (PDG) core scaffold, as well as a composition thereof and use thereof, and further relates to a protein degrader of a CRBN small molecule ligand compound based on a pyridazine-glutarimide (PDG) core scaffold and use thereof. The CRBN small molecule ligand compound is represented by (I):

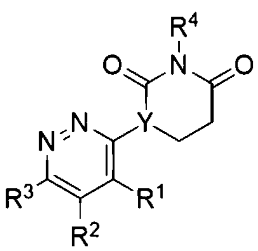

(I),

wherein $R^1$and $R^2$ are one or more selected from a condensed ring fused to the pyridazine parent core, H, alkyl, OR, F, CN, $CF_3$, $NR_2$, Ph and 4-Py; Y is one or more selected from CH, CD, CF and N; $R^3$ is one or more selected from OR, $NR_2$, $CHR_2$, and C≡CR; $R^4$ is selected from H or alkyl.


EP 4 763 838 A1

## Description

### Technical Field

The present disclosure belongs to the technical field of medical chemistry, particularly relates to a cereblon (CRBN) small molecule ligand compound based on a pyridazine-glutarimide (PDG) core scaffold and a composition and use thereof, and further relates to a protein degrader of a cereblon (CRBN) small molecule ligand compound based on a pyridazine-glutarimide (PDG) core scaffold and use thereof.

### Background

Cereblon (CRBN) is a substrate receptor protein of an E3 ubiquitin ligase complex of a CRL4CRBN subtype. It together with a DDB1 protein (Damage-specific DNA Binding protein 1), a zinc finger domain protein RBX1 and a Cullin4 (Cul4) scaffold protein forms a DDB1-Cul4-Rbx1-CRBN E3 ubiquitin ligase complex, and determines the substrate specificity of the CRL4 E3 ubiquitin ligase. It is widely expressed in the cytoplasm, nucleus and plasma membrane of prostate, liver, pancreas, placenta, kidney, lung, peripheral blood leukocytes and brain. The E3 ubiquitin ligase is capable of specifically recognizing a substrate protein and inducing the polyubiquitination of the substrate protein, so as to cause the ubiquitinated substrate protein to be degraded via a ubiquitin-proteasome pathway, thereby affecting various physiological activities such as cellular energy metabolism, membrane potential regulation and transcription factor degradation.

In 2010, Ito et al. first confirmed that the direct target of thalidomide is CRBN, a substrate receptor protein of an E3 ubiquitin ligase. After increasing evidence indicates that an immunomodulator plays its anti-tumor role by targeting CRBN, three papers [34-36] published in the same issue of Science in 2014 elucidated at a molecular level that the immunomodulators play their anti-multiple myeloma roles through a protein ubiquitination degradation mechanism. The CRL4CRBN (CUL4-Roc1-DDB1-CRBN) E3 ubiquitin ligase consists of a Cullin-4A (Cul4A) protein, a regulator of cullins 1 (Roc1) protein, a damaged DNA binding protein 1 (DDB1) and a CRBN (cereblon) protein. Cul4A has a scaffold function therein, while Roc1 has a ring finger domain associated with an E2 ubiquitin-conjugating enzyme. As a substrate receptor, CRBN directly binds to specific substrates and mediates their ubiquitination. Such ubiquitin ligase labels specific proteins through ubiquitin (Ub). The ubiquitin-labeled proteins are recognized by proteasomes as damaged or defective proteins so as to be hydrolyzed. The action of an immunomodulator on the E3 ubiquitin ligase activates the activity of the E3 ubiquitin ligase. Such activity is a basis for the immunomodulator to exert cytotoxic and immunomodulatory effects.

The ubiquitin-proteasome degradation pathway of the protein is a universal and important endogenous protein degradation method. This pathway is achieved through several sequential processes: 1, ATP provides energy so that the C-terminus of the ubiquitin covalently binds to the cysteine residue of the E1 ubiquitin-activating enzyme; 2, the E1 ubiquitin-activating enzyme transfers the ubiquitin to an E2 ubiquitin-conjugating enzyme which can transfer the ubiquitin to the $-NH_2$ of the lysine residue of the target protein; 3, an E3 ubiquitin ligase recruits the substrate protein and catalyzes the binding of the ubiquitin on the E2 ubiquitin-conjugating enzyme to the substrate protein; and 4, a 26S proteasome specifically recognizes and hydrolyzes the ubiquitin-labeled substrate protein.

The immunomodulator such as thalidomide binds to CRBN to activate the activity of the E3 ubiquitin ligase, thereby prompting the E3 ubiquitin ligase to recruit substrate proteins IKZF1/IKZF3 (Ikaros/Aiolos are zinc finger transcription factors that play an important role in blood cell differentiation). Subsequently, the E3 ubiquitin ligase, the substrate proteins IKZF1/IKZF3 and the immunomodulator will form a stable ternary complex to transfer the ubiquitin to the substrate proteins IKZF1/IKZF3, thereby causing IKZF1/IKZF3 to be degraded by the 26S proteasome. IKZF1 and IKZF3 are transcription regulatory factors that are essential for the proliferation and development of B cells and T cells [John LB, Ward AC. The Ikaros gene family: transcriptional regulators of hematopoiesis and immunity [J]. Molecular Immunology, 2011, 48(9):1272-1278. and Dijon M, Bardin F, Murati A, et al. The role of Ikaros in human erythroid differentiation [J]. Blood, 2008, 111(3):1138-1146]. Under normal conditions, IKZF3 inhibits a gene encoding IL-2 in T cells to stimulate the expression of IRF4 (a transcription factor responsive to infection). Therefore, on the one hand, the degradation of IKZF1 and IKZF3 directly reduces the expression of transcription factors such as IRF4 and Myc, thereby generating a cytotoxic effect on myeloma cells; on the other hand, the degradation of IKZF1 and IKZF3 improves the expression of IL-2 in T cells, thereby activating the immune response of T cells and suppressing the function of B cells, achieving tumor-killing effects and inhibiting tumor proliferation, while reducing the expression of tumor necrosis factor TNF and promoting human peripheral blood mononuclear cells to secrete an anti-inflammatory factor IL-10. Such imbalance leads to the apoptosis of multiple myeloma cells. In view of above, protein degradation targeting chimeras (PROTACs) designed based on the chemical structures of thalidomide-based immunomodulators such as thalidomide, lenalidomide and pomalidomide have continuously emerged in recent years.

A proteolysis targeting chimera (PROTAC), a protein degradation targeting chimera, is a bifunctional molecule which is composed of an E3 ligand structural unit capable of binding to a specific E3 ubiquitin ligase, a target protein ligand

structural unit capable of binding to a target protein and a linker that links the above two ligands. PROTAC can bind to a specific target protein by recruiting a specific E3 ubiquitin ligase to form an [E3 ubiquitin ligase-PROTAC-target protein] ternary complex. The complex can induce the target protein to be polyubiquitinated by the recruited E3 ubiquitin ligase, thereby causing the degradation of the target protein via the ubiquitin-proteasome pathway.

At present, more than 100 proteins have been successfully degraded by PROTAC. These targets include (1) kinases, such as RIPK2, BCR-ABL, EGFR, HER2, c-Met, TBK1, CDK2/4/6/9, ALK, Akt, CK2, ERK1/2, FLT3, PI3K, BTK and Fak; (2) BET proteins, such as BRD2/4/6/9; (3) nuclear receptors, such as AR and ER; and (4) other proteins, such as MetAp-2, Bcl-xL, Sirt2, HDAC6, Pirin, SMAD3, ARNT, PCAF/GCN5, Tau and FRS2. These targets even further include transcription factor regulatory protein pirin, epigenetic-related proteins PCAF/GCN5, KRAS-G12C and the like which are considered as "undruggable targets".

In the development of PROTACs, the CRBN ligand is the most widely used E3 ubiquitin ligase ligand. The designed PROTAC molecules can achieve different effects by selecting different target protein ligands and CRBN ligands. However, in practice, too few CRBN ligands can be selected, which cannot meet the demands for PROTACs development. Faced with the issues of immunomodulator tolerance, an inadequate number of CRBN ligands with a lack of structural diversity, and the commonly used thalidomide-derived CRBN ligands suffering from poor chemical stability, ready epimerization in vivo, a relatively large number of hydrogen bond donors and acceptors, poor solubility, and suboptimal binding activity, there is an urgent need to develop more CRBN ligands to address the aforementioned problems.

## Summary

In a first aspect, the present disclosure provides a compound capable of binding to a CRBN protein, wherein the compound is represented by Formula (I):

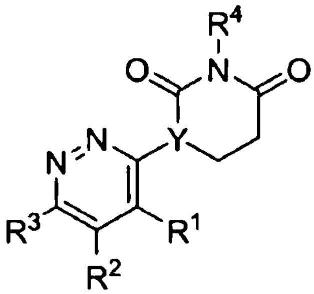

(I)

wherein $R^1$ and $R^2$ are one or more selected from a condensed ring fused to a pyridazine parent core, H, alkyl, OR, F, CN, $CF_3$, $NR_2$, Ph and 4-Py.

Further, the condensed ring may be selected from a fused ring, cyclopentane, cyclohexane, benzene and/or pyrazine.

Further, both $R^1$ and $R^2$ in the compound may be selected from alkyl.

Further, $R^1$ and $R^2$ in the compound cannot be at the same time selected from H, alkyl, OR, F, CN, $CF_3$, $NR_2$, Ph or 4-Py.

Further, when $R^1$ is H, $R^2$ is one or more selected from alkyl, OR, F, CN, $CF_3$, $NR_2$, Ph and 4-Py.

Further, when $R^2$ is H, $R^1$ is one or more selected from alkyl, OR, F, CN, $CF_3$, $NR_2$, Ph and 4-Py.

Y is one or more selected from CH, CD, CF, $CCH_3$ and N;

$R^3$ is one or more selected from OR, $NR_2$, $CHR_2$ and C≡CR; and

$R^4$ is selected from H or alkyl.

In a second aspect, the present disclosure further provides a pharmaceutically acceptable salt of the compound of Formula (I) according to the first aspect.

Further, the pharmaceutically acceptable salt refers to a non-toxic acid or base addition salt prepared from the parent compound.

In a third aspect, the present disclosure provides a protein degrader which is a target protein ligand (POI) + a Linker + a CRBN protein ligand, wherein the CRBN protein ligand is the compound according to the first aspect of the present disclosure, and the structural formula of the protein degrader is (II):

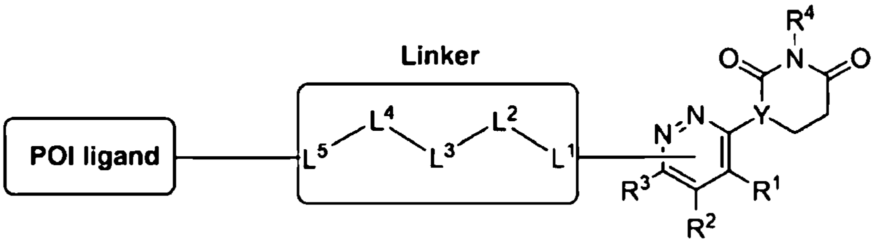

(II);

in the Formula II, the moiety $L^1$ of the Linker may be attached to C to which $R^2$ is attached, wherein $R^2$ is $L^1$; and $R^3$ is one or more selected from OR, $NR_2$, $CHR_2$ and C≡CR; and

in the Formula II, the moiety $L^1$ of the Linker is attached to C to which $R^3$ is attached, wherein $R^3$ is $L^1$, and $R^2$ is one or more selected from a condensed ring fused to the pyridazine parent core, H, alkyl, OR, F, CN, $CF_3$, $NR_2$, Ph and 4-Py.

In the Formula II, $R^1$ is one or more selected from a condensed ring fused to the pyridazine parent core, H, alkyl, OR, F, CN, $CF_3$, $NR_2$, Ph and 4-Py.

$R^4$ is selected from H or alkyl.

Y is one or more selected from CH, CD, CF, $CCH_3$ and N.

In the Formula II, the moieties $L^1$, $L^3$ and $L^5$ of the Linker may be one or more selected from:

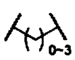 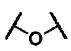 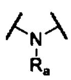 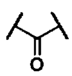 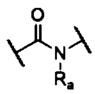 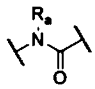 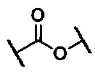 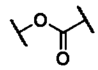 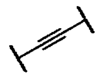

Ra = H, alkyl, $SO_2$(alyl). $SO_2$(aryl)

wherein, $R_a$ is one or more selected from H, alkyl, $SO_2$(alkyl) or $SO_2$(aryl).

In the Formula II, the moieties $L^2$ and $L^4$ of the Linker may be selected from one of:

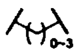

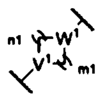

**m1, n1* 1, 2, 3,**

**m1+n1<=4**

**m1 = n1 = 2: W1, V1 = CH or N**

**m1 x n1 <= 3, W1, V1 = CH or N, but cannot be N at the same time**

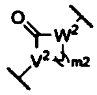

**m2 = 1, 2, 3;**

**W2, V2 = CH or N**

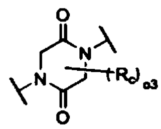

**Rc = alkyl**

**o3 = 0 - 4**

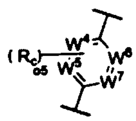

**W4, W5, W6, W7 = C or CH or N, and N count <3**

**Rc = alkyl or halo or CN or $CF_3$, o5 = 0 - 4**

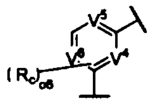

**V4, V5, V6 = C or CH or N, and N count <3**

**Rc = alkyl or halo or CN or $CF_3$, o6 = 0 - 4**

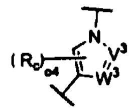 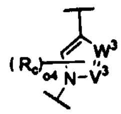

**W3, V3 = C or CH or N**

**Rc = alkyl**

**o4 = 0 ~ 3**

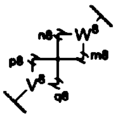 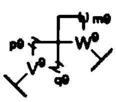 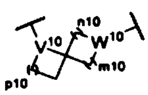 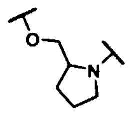 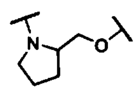

(continued)

W8, V8 = CH or N
m8, n8, p8, q8 = 1, 2, 3
and m8 + n8 < 5
and p8 + q8 < 5

W9, V9 = CH or N
m9, p9, q9 = 1, 2, 3
and p9 + q9 < 5

W10, V10 = CH or N
m10, n10, p10 = 1, 2, 3
and m10 + n10 < 5

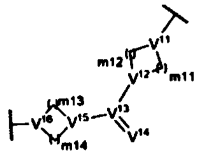

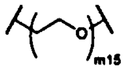 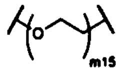

m15 = 1-20

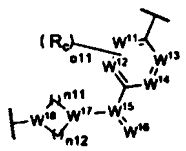 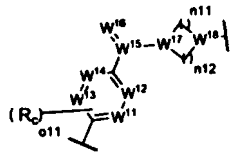

V11, V12, V15, V16 = CH or N
V13 = none, C, CH2, O, NR.
V14 = O, none
Ra = H, alkyl, $SO_2$(alyl), $SO_2$(aryl)
m11, m12, m13, m14 = 1, 2, 3
and m11 + m12 < 5
and m13 + m14 < 5
unless m11 = m12 = 2, V11 and V12 cannot be N at the same time

unless m13 = m14 = 2, V15 and V16 cannot be N at the same time

when V13 = C and V14 = O, V12, V15 = CH, N
when V13 = CH2 and V14 = none, V12 and V15 cannot be N at the same time
when V13 = O and V14 = none, V12 = V15 = CH
when V13 = V14 = none, V12 and V15 cannot be N at the same time

W11, W12, W13, W14 = C, CH, N
W17, W18 = CH, N
W15 = none, C, CH2, O, NR,
W16 = O, none
Ra = H, alkyl, $SO_2$(alyl), $SO_2$(aryl)
Rc = alkyl or halo or CN or $CF_3$
o11 = 0 ~ 4
Of the positions W11, W12 W13 and W14, only two may be N

n11, n12 = 1, 2, 3
and n11 + n12 < 5
unless n11 = n12 = 2, V11 and V12 cannot be N at the same time
when W15 = C, W16 = O
when W15 = CH2, W16 = none

when W15 = O and W16 = none, W17 = CH

Further, the target protein ligand is a ligand of a target protein that causes a related disease.

Further, the disease includes, but is not limited to, cancers or diseases such as prostate cancer, breast cancer, non-small cell lung cancer, chronic myeloid leukemia, acute myeloid leukemia, T-cell acute lymphoblastic leukemia, Alzheimer's disease, gout, autoimmune diseases, inflammatory bowel disease, B-cell lymphoma, androgenetic alopecia, acne, synovial sarcoma, solid tumors, lung cancer, multiple myeloma, lymphoma, tumor metastasis, cervical cancer, neuroblastoma, hepatocellular carcinoma, colorectal cancer, pancreatic cancer, malignant rhabdoid tumor, oral squamous cell carcinoma, etc.

Further, the target protein ligand, i.e., the POI ligand in the Formula II, may be one or more selected from:

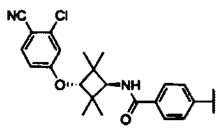 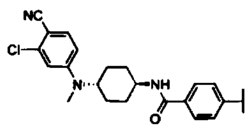 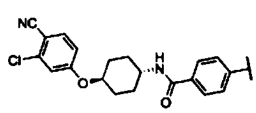 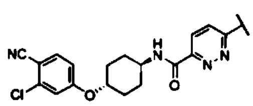

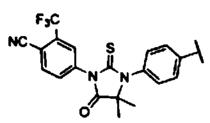 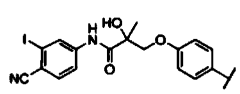 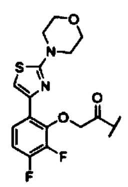 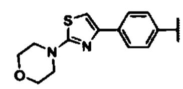

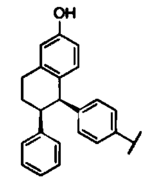
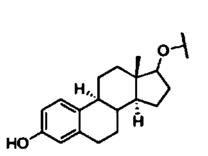
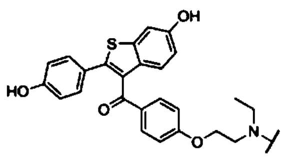
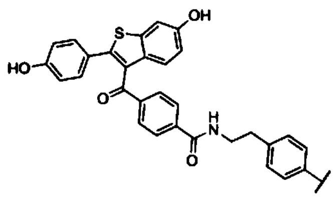

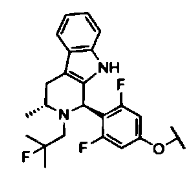
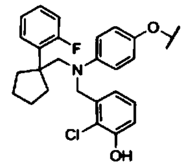

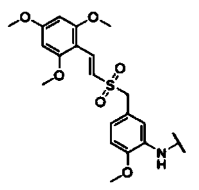
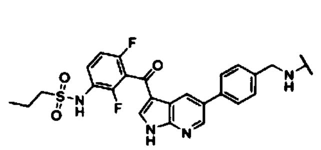
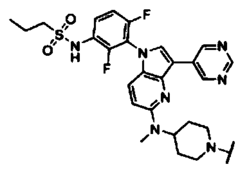
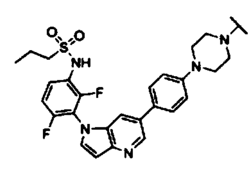

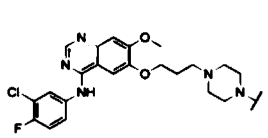
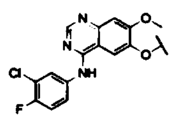
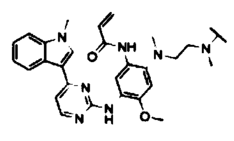
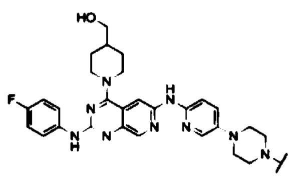

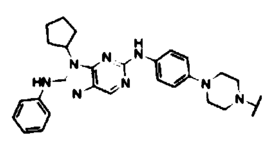
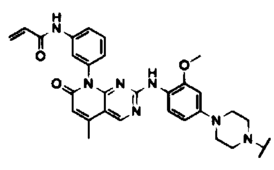
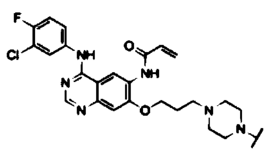

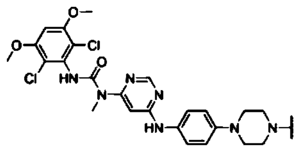
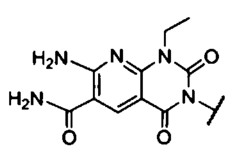
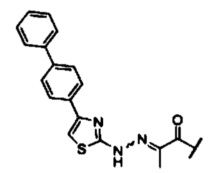
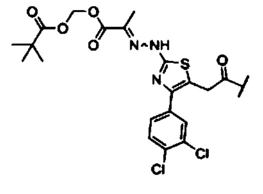

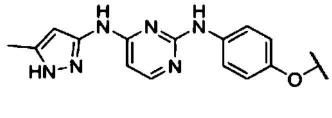
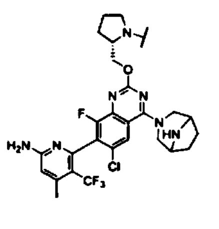
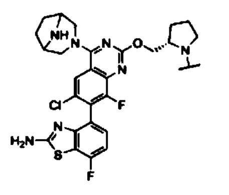

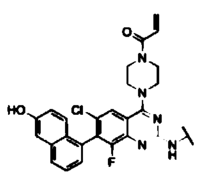
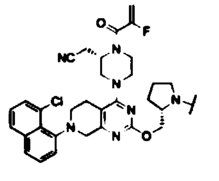
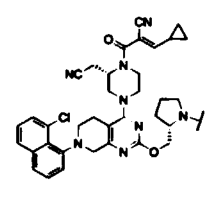
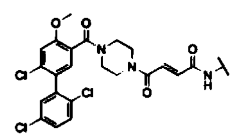

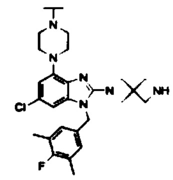
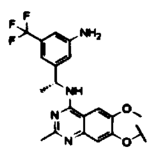
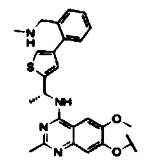
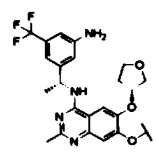
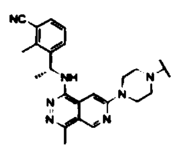

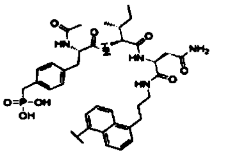
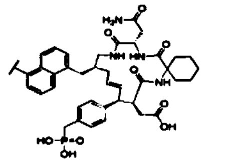
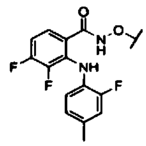
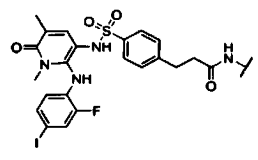

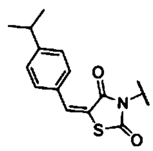
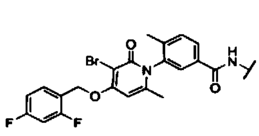
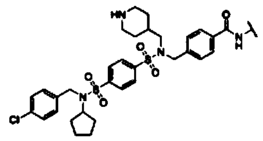
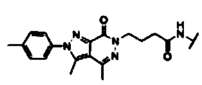

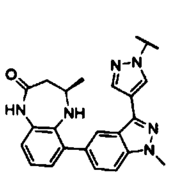
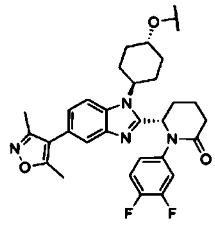
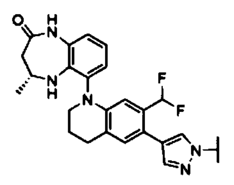
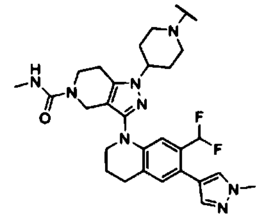

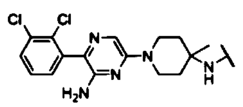
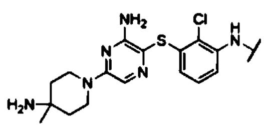
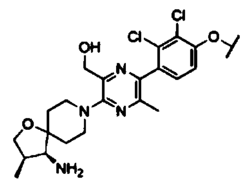
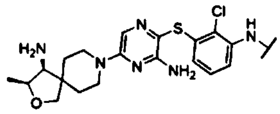

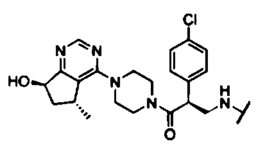
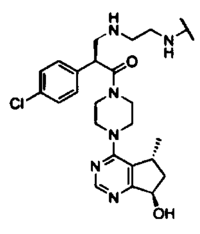

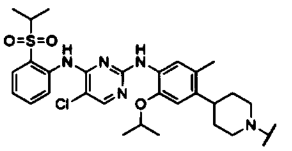
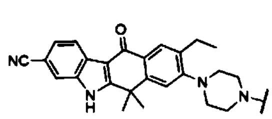
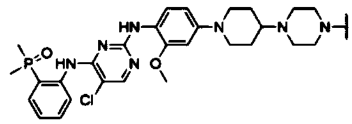

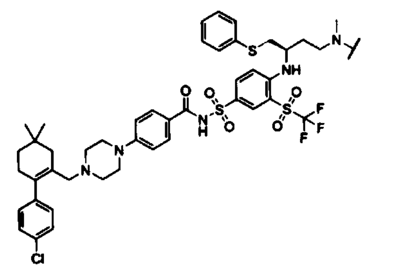

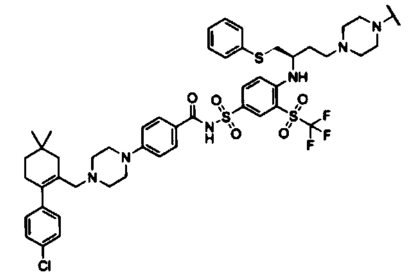

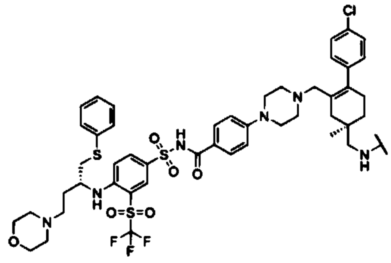

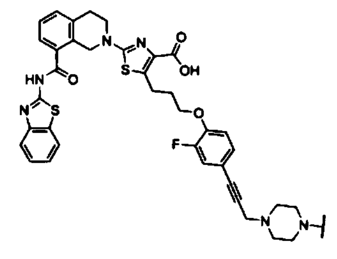

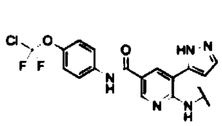

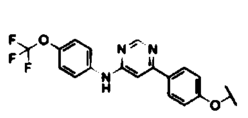

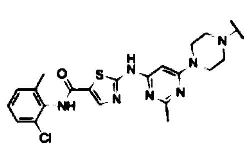

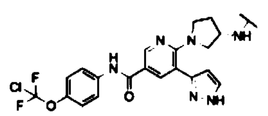

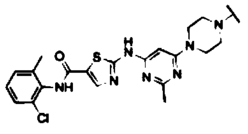

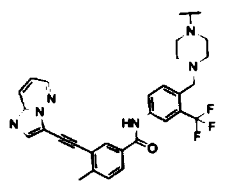

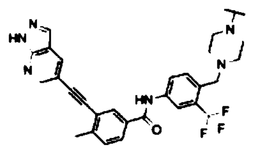

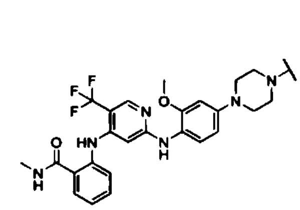

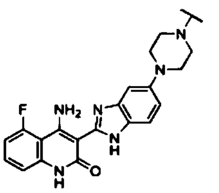

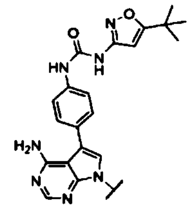

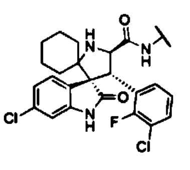

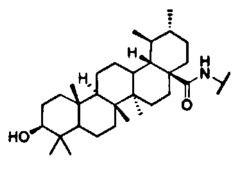

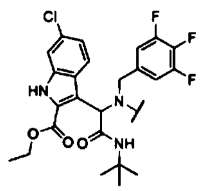

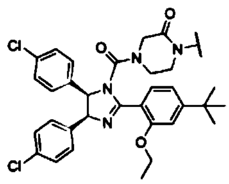

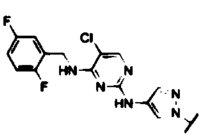

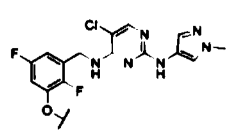

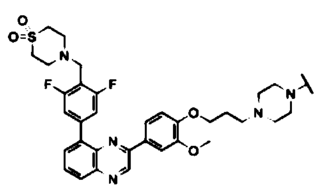

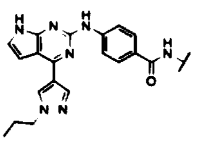

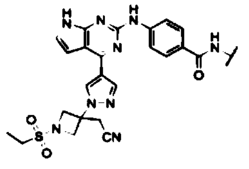

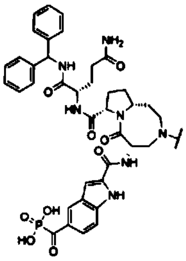

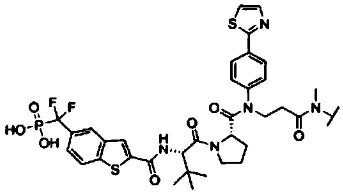

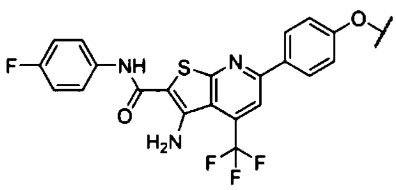

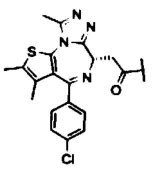

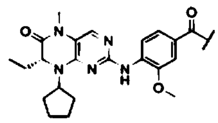

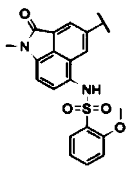

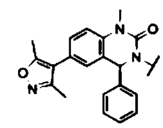

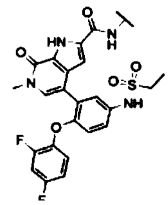

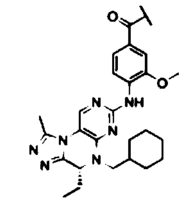

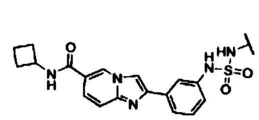

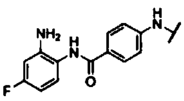

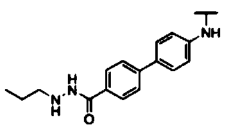

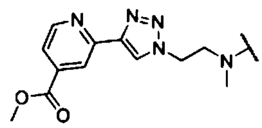

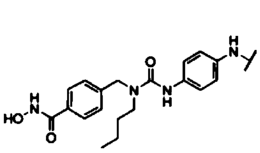

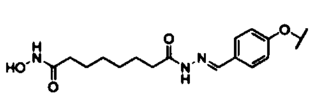

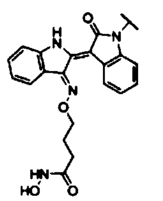

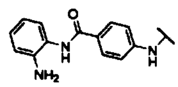

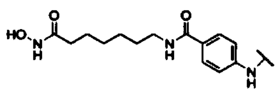

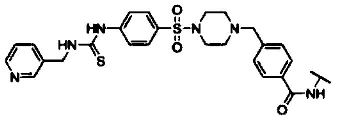

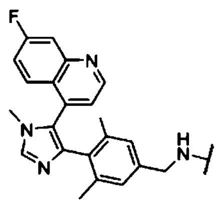

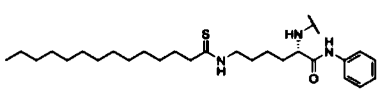

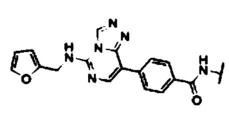 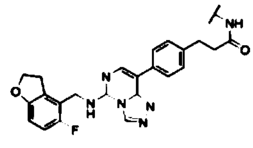 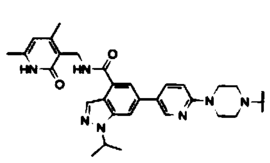 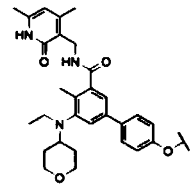

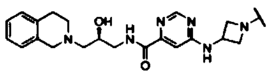

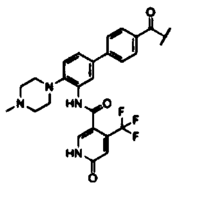 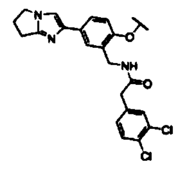 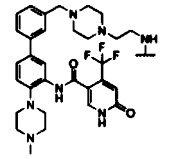 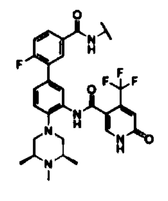

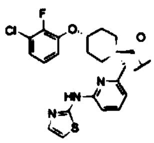 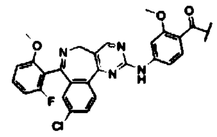 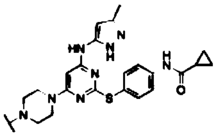 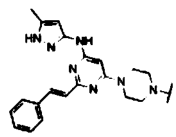

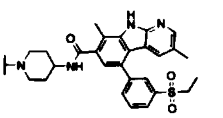 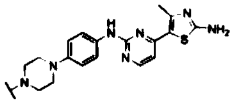 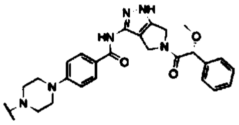 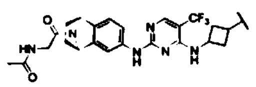

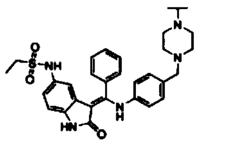 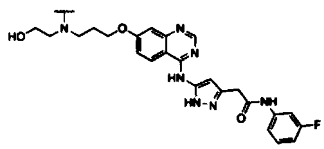 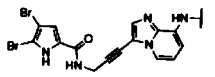

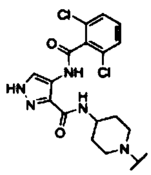 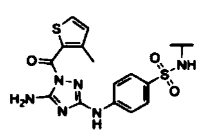 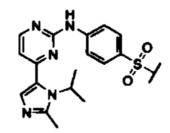

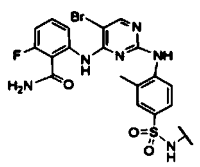 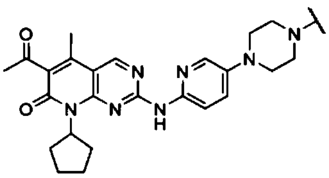 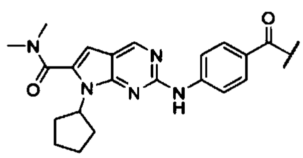

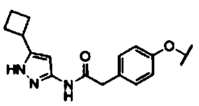 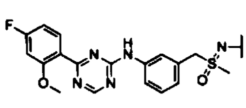 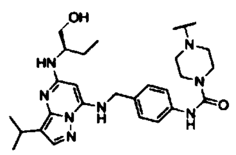 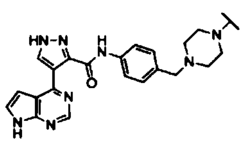

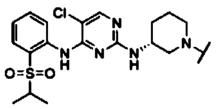
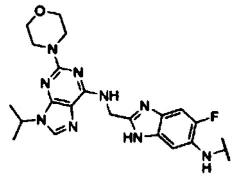
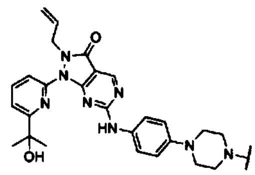

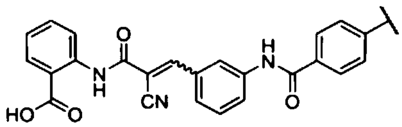
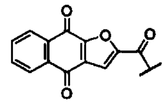

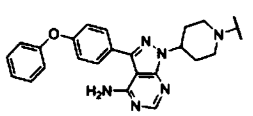
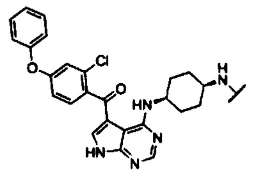
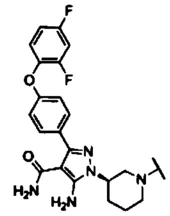
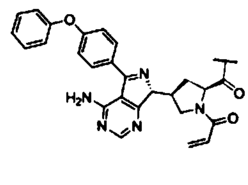

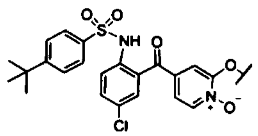
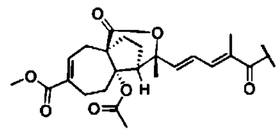
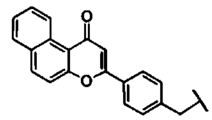

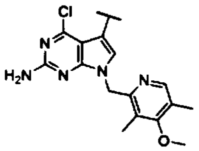
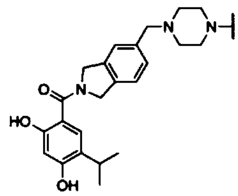
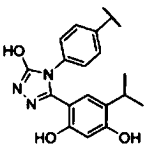

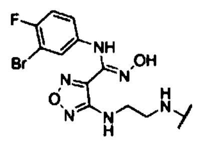
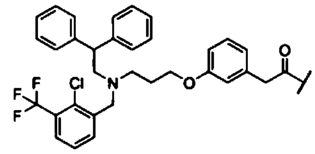
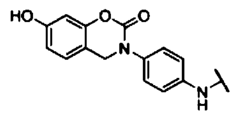

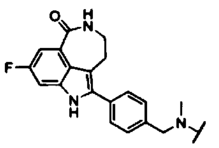
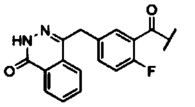
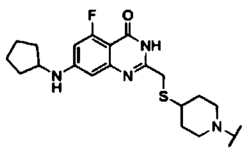

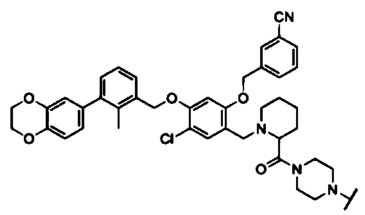
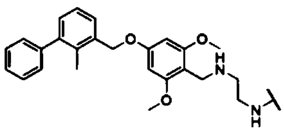
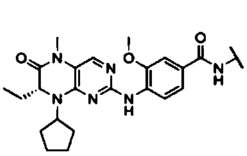

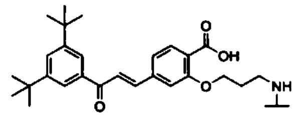 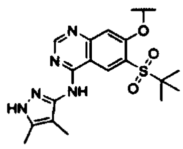 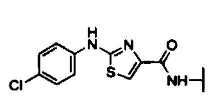 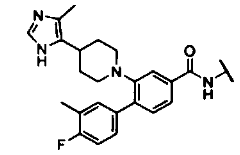

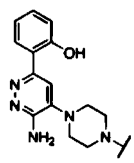 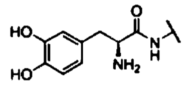 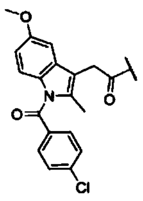 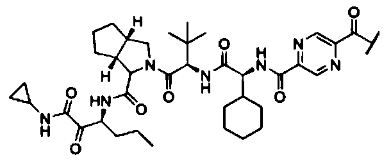

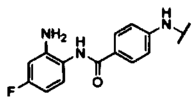 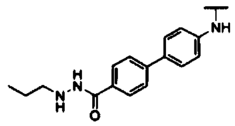 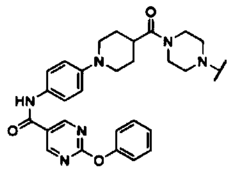 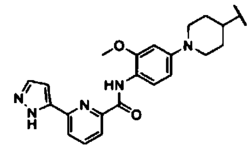

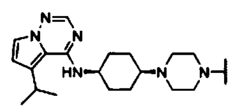

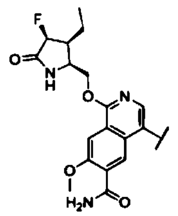 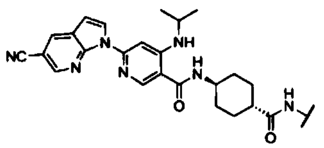 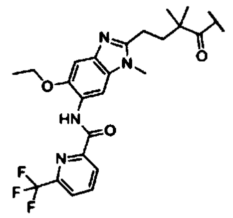

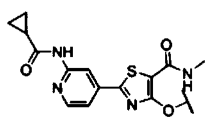 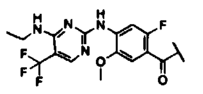 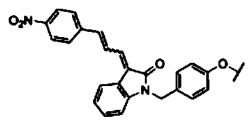 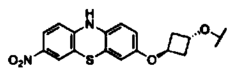

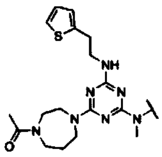 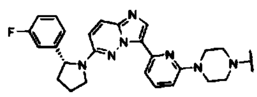 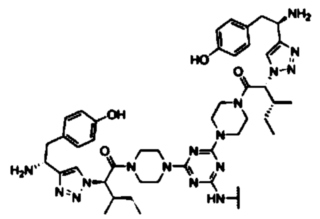

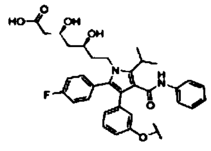 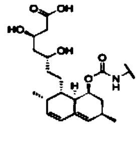 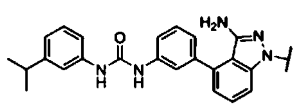 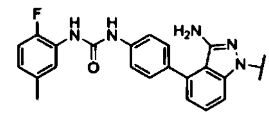 .

In a fourth aspect, the present disclosure provides a pharmaceutical composition comprising a combination of the compound of Formula (I) or the pharmaceutically acceptable salt thereof according to the first aspect and a pharmaceutically acceptable diluent or carrier.

Further, the pharmaceutical composition further comprises a combination of an enantiomer, a diastereomer and a stereoisomer of the compound of Formula (I) or the pharmaceutically acceptable salt thereof and a pharmaceutically

acceptable diluent or carrier.

In a fifth aspect, the present disclosure provides a pharmaceutical composition for the targeted degradation of a protein, the pharmaceutical composition comprising a combination of the protein degrader (II) or the pharmaceutically acceptable salt thereof according to the second aspect of the present disclosure and a pharmaceutically acceptable diluent or carrier.

Further, the pharmaceutical composition for the targeted degradation of the protein further comprises a combination of an enantiomer, a diastereomer, a stereoisomer or a pharmaceutically acceptable salt of the compound of Formula (I) and a pharmaceutically acceptable diluent or carrier.

In a sixth aspect, the present disclosure provides a regulator for regulating a transcription regulatory factor, the regulator comprising a compound of Formula (1) or a pharmaceutically acceptable salt or protein degrader (II) thereof, or a pharmaceutically acceptable salt, a hydrate, a solvate, a prodrug, a stereoisomer or a tautomer thereof.

Further, the transcription regulatory factor is a regulatory factor that is essential for proliferation and development of B cells and T cells.

Further, the regulation can be positive regulation and/or negative regulation.

Further, the transcription factor includes, but is not limited to, IRF4 and Myc.

In a seventh aspect, the present disclosure provides a method for treating or preventing pathological conditions or symptoms of a disease caused by limiting or inhibiting the expression of IRF4 and Myc, comprising administering to a patient in need thereof an effective amount of at least one of a compound of Formula I or a compound of Formula II, or a pharmaceutically acceptable salt, a hydrate, a solvate, a prodrug, a stereoisomer or a tautomer thereof.

Further, the pathological conditions or symptoms include, but are not limited to, bone marrow failure, anemia, immune paresis and infection, fractures and bone pain, high calcium levels, renal failure, etc.

## Detailed Description of the Embodiments

The specific embodiments of the present disclosure will be further described below. It is to be noted that the description of these embodiments is used to help understand the present disclosure, but not limit the present disclosure. In addition, the technical features involved in the embodiments described below can be combined with each other as long as they do not conflict with each other.

The present disclosure will be described below through specific embodiments. However, the present disclosure is not limited thereto. Any modifications, equivalent substitutions and improvements made within the spirit and principles of the present disclosure shall be included within the protection scope of the present disclosure.

The experimental methods used in the following embodiments, unless otherwise specified, are all conventional methods.

The materials, reagents and the like used in the following embodiments, unless otherwise specified, can be commercially available.

Provided is a compound of a CRBN protein, wherein the compound is represented by Formula (I):

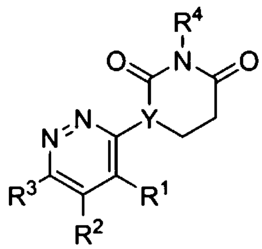

(I).

If the compound contains fatty amine, the depicted structure includes, but is not limited to, its free amine form, hydrochloride salt form or trifluoroacetate salt form.

In some embodiments, $R^1$ is one of the following structures:

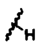, 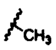, 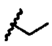, 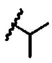, 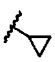, 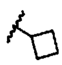, 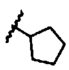, 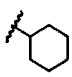, 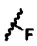, 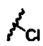, 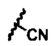, 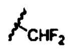,

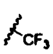, 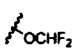, 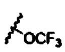, 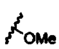, 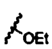, 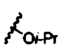, 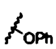, 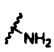, 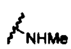, 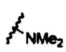, 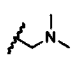,

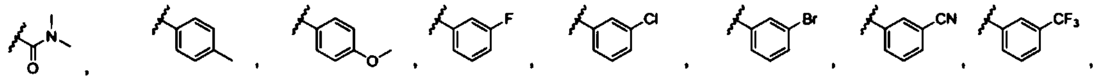
, , , , , , , ,

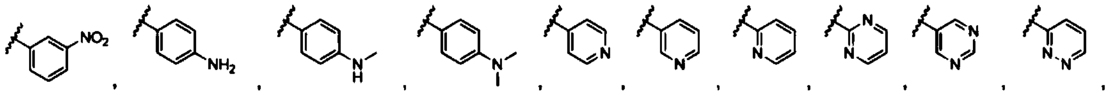
, , , , , , , , , ,

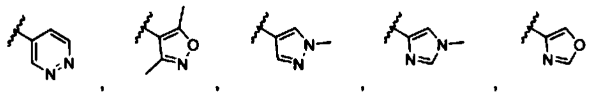
, , , ,

When $R^2$ is one of the following structures:

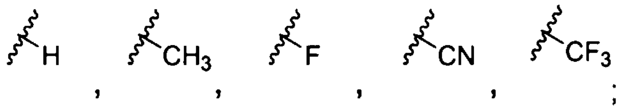
, , , , ;

$R^3$ is selected from any one of the following structures:

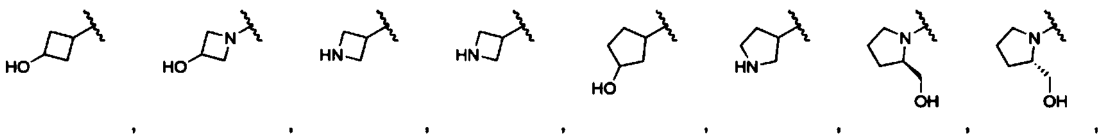
, , , , , , , ,

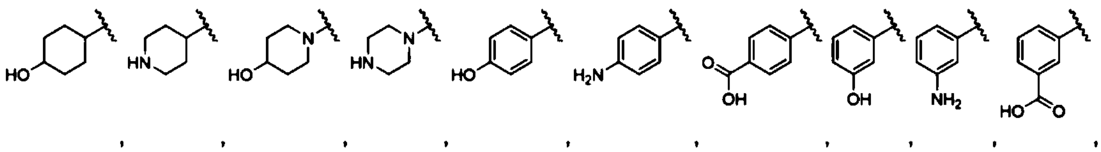
, , , , , , , , , ,

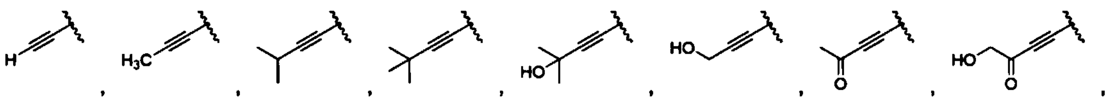
, , , , , , , ,

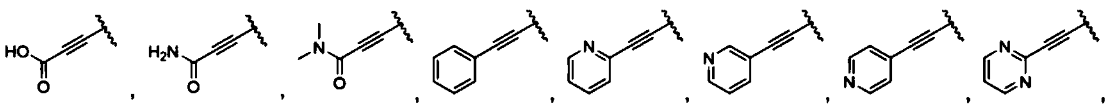
, , , , , , , ,

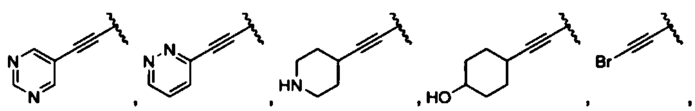
, , , , ,

When $R^2$ is one of the following structures:

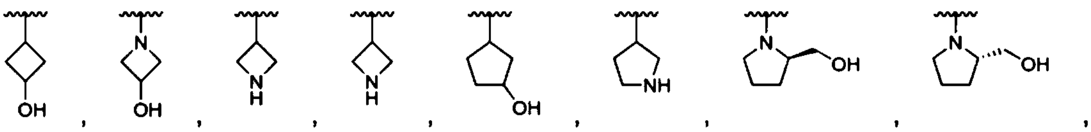
, , , , , , , ,

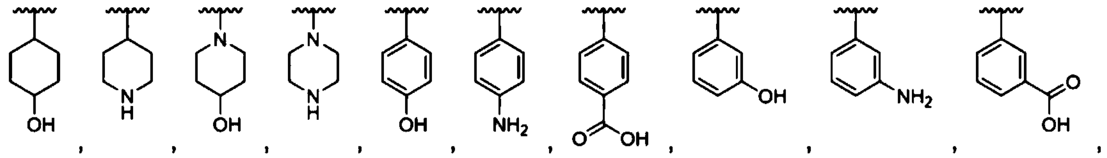

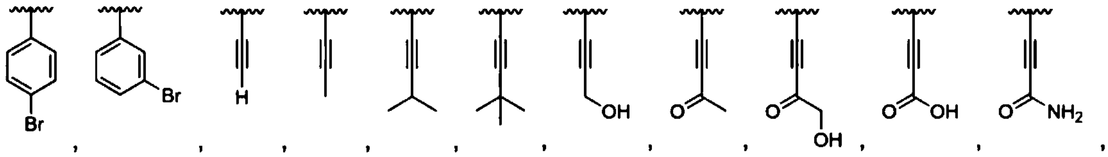

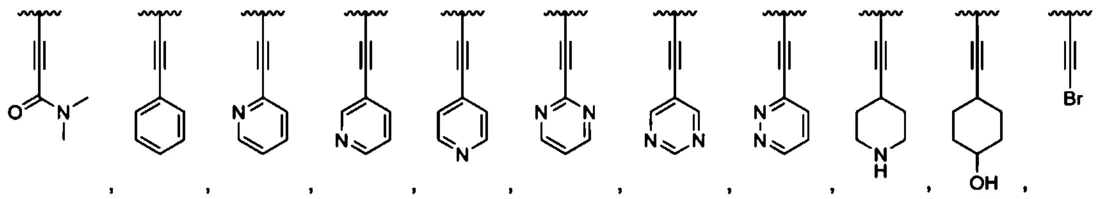

$R^3$ is any one of the following structures:

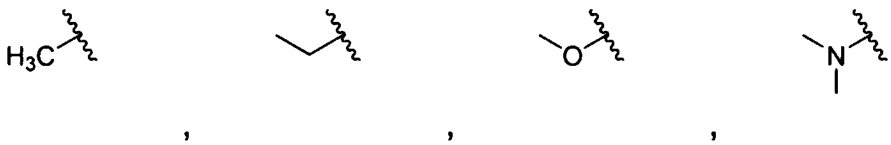

Y is one of the following structures: $CCH_3$, CD, CF and N; and

$R^4$ is one of the following structures: H, $CH_3$, Boc, Cbz and Ph.

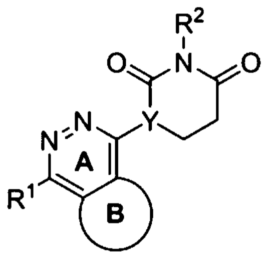

In the above-mentioned structure, if it contains the fatty amine, the depicted structure includes, but is not limited to, its free amine form, hydrochloride salt form or trifluoroacetate form;

The AB condensed ring is one of the following structures:

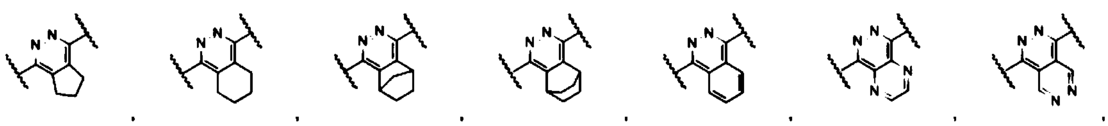

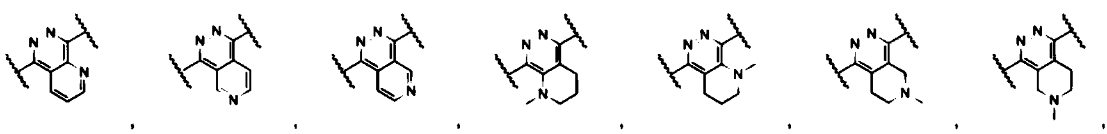

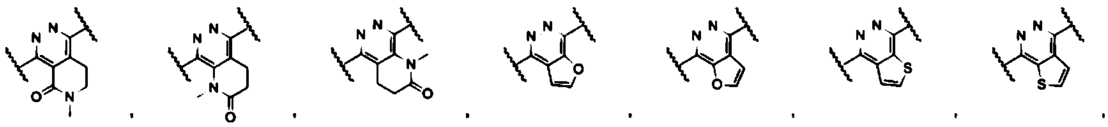
, , , , , , ,

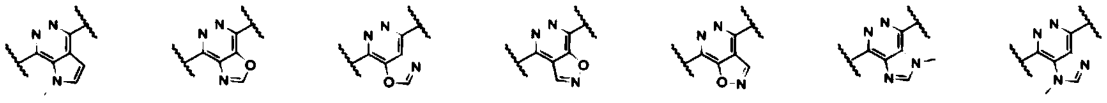
, , , , , , ,

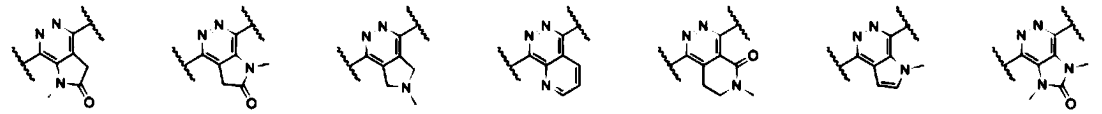
, , , , , , ,

$R^1$ is one of the following structures:

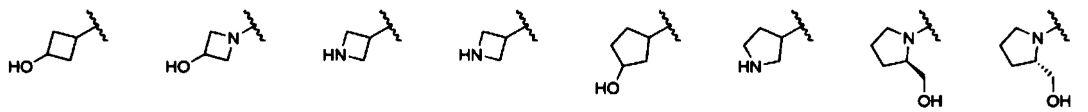
, , , , , , , ,

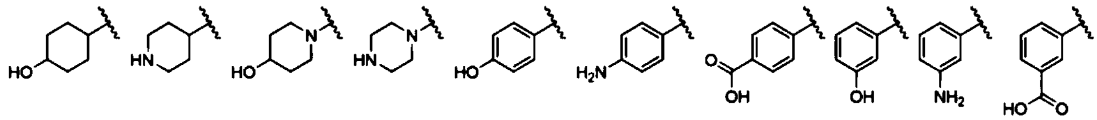
, , , , , , , , , ,

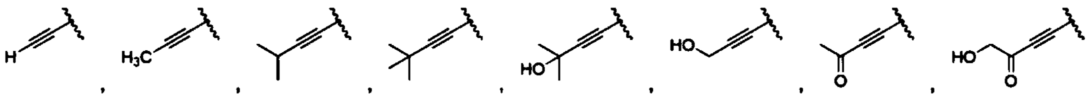
, , , , , , , ,

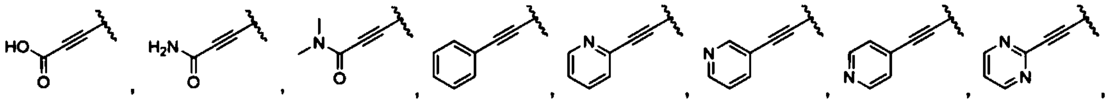
, , , , , , , ,

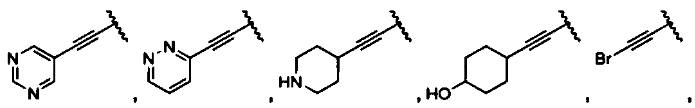
, , , , ,

Y is one of the following structures: $CCH_3$, CD, CF, N; and $R^4$ is one of the following structures: H, $CH_3$, Boc, Cbz and Ph.

Table 1 Exemplary compound structures binding to CRBN small molecules

| ID | IUPAC Name | Structure | $^1$H NMR | UPLC-MS m/z, $t_R$ |
|---|---|---|---|---|
| 1 | 3-(6-(prop-1-yn-1-yl)pyrid azin-3-yl)piperidine-2,6-di one | 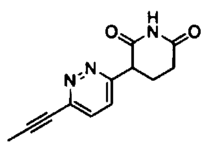 | $^1$H NMR (400 MHz, Chloroform-*d*) δ 7.69 (dd, *J* = 7.8, 0.6 Hz, 1H), 7.64 (d, *J* = 7.8 Hz, 1H), 4.05 - 3.91 (m, 1H), 2.68 - 2.45 (m, 2H), 2.26 - 2.01 (m, 5H) ppm. | [M - H]⁻ 230.1 4.516 min. |

(continued)

| ID | IUPAC Name | Structure | $^1$H NMR | UPLC-MS m/z, $t_R$ |
|---|---|---|---|---|
| 2 | 3-(6-(azetidin-3-yl)pyridaz in-3-yl)piperidine-2,6-d ion ehydrochloride salt | 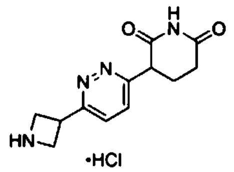 | $^1$H NMR (400 MHz, $D_2O$) δ 7.67 (dd, *J* = 8.3, 0.6 Hz, 1H), 7.57 (dd, *J* = 8.2, 0.5 Hz, 1H), 4.03 - 3.92 (m, 1H), 3.55 - 3.38 (m, 5H), 2.67 - 2.52 (m, 2H), 2.25 - 2.03 (m, 2H) ppm. | [M - H]$^-$ 247.1 0.972 min. |
| 3 | 3-(6-(piperazin-1-yl)pyridazin-3-yl)piperidine-2,6-di one hydrochloride salt azin-3-yl)piperidine-2,6-di | 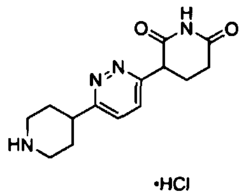 | $^1$H NMR (400 MHz, $D_2O$) δ 7.92 - 7.82 (m, 2H), 4.04 - 3.90 (m, 4H), 3.41 (dd, *J* = 7.0, 3.4 Hz, 5H), 2.78 - 2.70 (m, 2H), 2.41 - 2.22 (m, 2H) ppm. | [M - H]-275.2 0.941 min. |
| 4 | 3-(6-(3-hydroxyazetidin-1 -yl)pyridazin-3-yl) piperidin e-2,6-dione -yl) pyridazin-3-yl)piperidin | 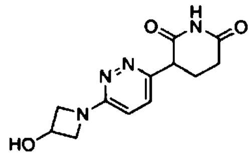 | $^1$H NMR (400 MHz, Methanol-$d_4$) δ 7.51 - 7.36 (m, 1H), 6.95 (d, *J* = 8.6 Hz, 1H), 4.20 (q, *J* = 4.3 Hz, 1H), 4.07 - 4.01 (m, 1H), 3.93 (dd, *J* = 12.3, 4.4 Hz, 2H), 3.83 (dd, *J* = 12.4, 4.4 Hz, 2H), 2.65 - 2.51 (m, 2H), 2.20 - 2.05 (m, 2H) ppm. | [M - H]-263.1 2.689 min. |
| 5 | 3-(6-((R)-2-(hydroxymeth yl)pyrrolidin-1-yl) pyridazin -3-yl)piperidine-2,6-dione | 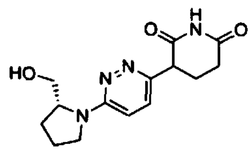 | $^1$H NMR (400 MHz, Methanol-$d_4$) δ 7.66 - 7.46 (m, 1H), 6.92 (d, *J* = 8.1 Hz, 1H), 4.07 - 3.67 (m, 6H), 2.69 - 2.49 (m, 2H), 2.22 - 1.71 (m, 6H) ppm. | [M - H]-291.2 2.323 min. |
| 6 | 3-(6-((S)-2-(hydroxymeth yl) pyrrolidin-1-yl) pyridazin-3 -yl)piperidine-2,6-dione | 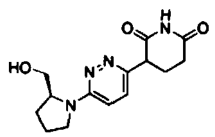 | $^1$H NMR (400 MHz, Methanol-$d_4$) δ 7.66 - 7.36 (m, 1H), 6.92 (d, *J* = 8.1 Hz, 1H), 4.12 - 3.55 (m, 6H), 2.69 - 2.40 (m, 2H), 2.25 - 1.75 (m, 6H) ppm. | [M - H]$^-$ 291.2 2.323 min. |
| 7 | 3-(6-(piperazin-1-yl)pyridazin-3-yl)piperidine-2,6-di one hydrochloride salt | 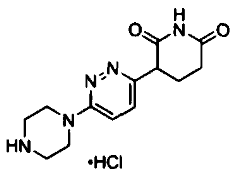 | $^1$H (d, 12.9 1H), 2.60 4H), 1H), 2.60 4H), $^1$H NMR (400 MHz, $D_2O$) δ 7.97 (d, *J* = 1.3 Hz, 2H), 3.52 (d, *J* = 12.9 Hz, 2H), 3.41 - 3.30 (m, 1H), 3.21 - 3.09 (m, 2H), 2.81-2.60 (m, 2H), 2.52 - 2.15 (m, 4H), 2.09 - 1.93 (m, 2H) ppm. | [M - H]-276.1 0.982 min. |
| 8 | 3-(6-methoxypyridazin-3-yl) piperidine-2,6-dione | 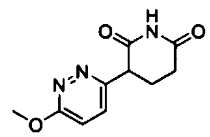 | $^1$H NMR (400 MHz, Methanol-$d_4$) δ 7.58 - 7.52 (m, 1H), 7.03 (d, *J* = 8.1 Hz, 1H), 4.07 - 3.99 (m, 1H), 3.90 (s, 3H), 2.65 - 2.50 (m, 2H), 2.23 - 2.05 (m, 2H) ppm. | [M - H]$^-$ 222.1 3.567 min. |
| 9 | 3-(4-methyl-6-(piperazin-1-yl)pyridazin-3-yl)piperidi ne- 2,6-dione hydrochloride salt | 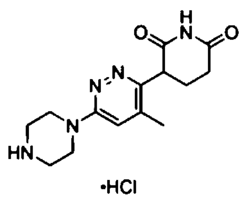 | $^1$H NMR (400 MHz, Methanol-$d_4$) δ 6.94 (s, 1H), 4.12 (t, *J* = 6.0 Hz, 1H), 3.66 - 3.58 m, 5H), 3.04 - 2.89 (m, 5H), 2.41 (d, *J* = 0.7 Hz, 3H), 2.28 - 2.09 (m, 2H) ppm. | [M - H]$^-$ 290.2 0.944 min. |

(continued)

| ID | IUPAC Name | Structure | $^1$H NMR | UPLC-MS m/z, $t_R$ |
|---|---|---|---|---|
| 10 | 3-(4-ethyl-6-(piperazin-1-yl) pyridazin-3-yl) piperidine-2,6-dione hy-drochloride salt | 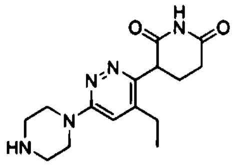 •HCl | $^1$H NMR (400 MHz, Methanol-*d*4) δ 6.66 (s, 1H), 4.13 (t, *J* = 6.5 Hz, 1H), 3.66 - 3.55 (m, 5H), 3.01 - 2.68 (m, 7H), 2.30 - 1.97 (m, 2H), 1.22 (t, *J* = 7.7 Hz, 3H) ppm. | [M - H]-304.2 0.938 min. |
| 11 | 3-(4-isopropyl-6-(piperazi n-1-yl)pyridazin-3-yl) piper idine-2,6-dione hy-drochloride salt | 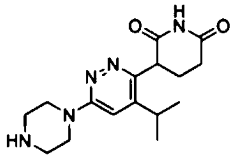 •HCl | $^1$H NMR (400 MHz, Methanol-$d_4$) δ 6.67 (s, 1H), 4.18 (t, *J* = 6.3 Hz, 1H), 3.65 -3.58 (m, 5H), 3.40 - 3.18 (m, 1H), 2.96 - 2.87 (m, 5H), 2.34 - 2.07 (m, 2H), 1.39 - 1.29 (m, 6H) ppm. | $[M - H]^-$ 318.2 0.956 min. |
| 12 | 3-(4-cyclopropyl-6-(pi-pera zin-1-yl)pyrida-zin-3-yl)pip eridine-2,6-dione hydrochloride salt | 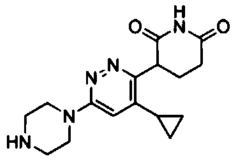 •HCl | 2.32 - 2.09 2.32 - 2.09 $^1$H NMR (400 MHz, Methanol-$d_4$) δ 6.99 (d, *J* = 0.6 Hz, 1H), 4.19 (t, *J* = 6.3 Hz, 1H), 3.65 - 3.59 (m, 4H), 3.11 - 2.82 (m, 5H), 2.64 - 2.52 (m, 2H), 2.32 - 2.09 (m, 2H), 1.31 - 1.10 (m, 2H), 0.97 - 0.74 (m, 2H) ppm. | $[M - H]^-$ 316.2 0.944 min. |
| 13 | 3-(4-fluoro-6-(piperazin-1-yl)pyridazin-3-yl)piperidin e-2,6-dione hy-drochloride salt | 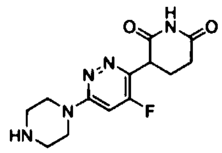 •HCl | $^1$H NMR (400 MHz, Methanol-$d_4$) δ 6.79 (d, *J* = 7.9 Hz, 1H), 4.18 (t, *J* = 6.0 Hz, 1H), 3.64 - 3.57 (m, 4H), 2.98 - 2.87 (m, 4H), 2.71 - 2.51 (m, 2H), 2.32 - 2.09 (m, 2H) ppm. | $[M - H]^-$ 294.1 0.911 min. |
| 14 | 3-(2,6-dioxopiperidin-3-yl) -6-(piperazin-1-yl) pyridazi ne-4-carbonitrile hydrochloride salt | 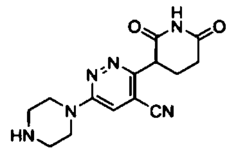 •HCl | $^1$H NMR (400 MHz, Methanol-$d_4$) δ 7.32 (s, 1H), 4.16 (t, *J* = 5.4 Hz, 1H), 3.65 - 3.58 (m, 4H), 2.97 - 2.88 (m, 4H), 2.67 - 2.53 (m, 2H), 2.28 - 2.11 (m, 2H) ppm. | [M - H]-301.1 0.921 min. |
| 15 | 3-(6-(piperazin-1-yl)-4-(trif luoromethyl) pyridazin-3-yl )piperi-dine-2,6-dione hydro-chloride salt | 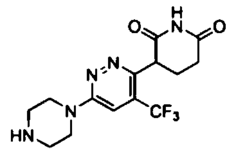 •HCl | $^1$H NMR (400 MHz, Methanol-$d_4$) δ 7.39 (s, 1H), 3.76 (t, *J* = 5.9 Hz, 1H), 3.64 - 3.58 (m, 4H), 2.97 - 2.87 (m, 4H), 2.71 - 2.54 (m, 2H), 2.28 - 2.09 (m, 2H) ppm. | $[M - H]^-$ 344.1 0.923 min. |
| 16 | 3-(4-methoxy-6-(piperazi n-1-yl)pyridazin-3-yl) piper idine-2,6-dione hy-drochloride salt | 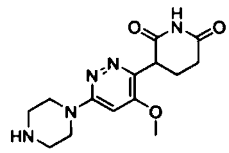 •HCl | $^1$H NMR (400 MHz, Methanol-*d*4) δ 6.54 (s, 1H), 4.14 (t, *J* = 6.4 Hz, 1H), 3.86 (s, 3H), 3.65 - 3.60 (m, 4H), 2.97 - 2.89 (m, 4H), 2.62 - 2.54 (m, 2H), 2.27 - 2.08 (m, 2H) ppm. | $[M - H]^-$ 306.2 0.911 min. |
| 17 | 3-(4-amino-6-(piperazin-1 -yl)pyridazin-3-yl) piperidin e-2,6-dione hy-drochloride salt | 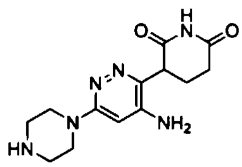 •HCl | 4.13 $^1$H NMR (400 MHz, Methanol-$d_4$) δ 6.44 (s, 1H), 4.13 (t, *J* = 6.6 Hz, 1H), 3.65 - 3.57 (m, 4H), 2.92 (m, 4H), 2.62 - 2.53 (m, 2H), 2.22 - 2.07 (m, 2H) ppm. | [M - H]-291.2 0.758 min. |

(continued)

| ID | IUPAC Name | Structure | $^1$H NMR | UPLC-MS m/z, $t_R$ |
|---|---|---|---|---|
| 18 | 3-(4-((dimethylamino) met hyl)-6-(piperazin-1-yl)pyri dazin-3-yl)piperi-dine-2,6-dionebis hydro-chloride salt | 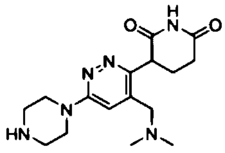 •2HCl | $^1$H NMR (400 MHz, Methanol-$d_4$) δ 6.74 (s, 1H), 4.12 (t, *J* = 6.4 Hz, 1H), 3.84 (s, 2H), 3.64 - 3.58 (m, 4H), 2.96 - 2.88 (m, 4H), 2.62 - 2.57 (m, 2H), 2.41 (s, 6H), 2.26 - 2.09 (m, 2H) ppm. | $[M + 2H]^{2+}$ 167.1 0.781 min. |
| 19 | 3-(4-phenyl-6-(piperazin-1-yl)pyridazin-3-yl)piperidi ne- 2,6-dione hy-drochloride salt | 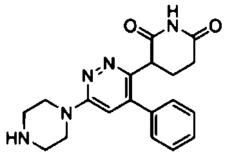 •HCl | $^1$H NMR (400 MHz, Methanol-$d_4$) δ 7.66 - 7.59 (m, 2H), 7.50 - 7.38 (m, 3H), 7.25 (s, 1H), 3.79 (t, *J* = 6.2 Hz, 1H), 3.66 - 3.57 (m, 4H), 2.96 - 2.87 (m, 4H), 2.65 - 2.56 (m, 2H), 2.26 - 2.12 (m, 2H) ppm. | $[M - H]^-$ 352.2 0.972 min. |
| 20 | 3-(6-(piperazin-1-yl)-4-(py ridin-4-yl)pyri-dazin-3-yl)pi peri-dine-2,6-dione hydro-chloride salt | 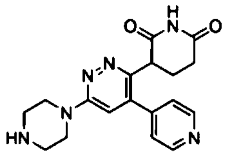 •HCl | $^1$H NMR (400 MHz, Methanol-$d_4$) δ 8.71 - 8.61 (m, 2H), 7.63 - 7.55 (m, 2H), 7.29 (s, 1H), 3.79 (t, *J* = 6.3 Hz, 1H), 3.64 - 3.58 (m, 4H), 2.96 - 2.88 (m, 4H), 2.61 - 2.53 (m, 2H), 2.27 - 2.10 (m, 2H) ppm. | $[M - H]^-$ 353.2 0.850 min. |
| 21 | 3-(5-methyl-6-(piperazin-1-yl)pyridazin-3-yl)piperidi ne- 2,6-dione hy-drochloride salt | 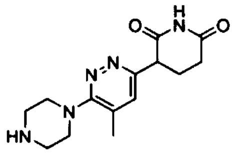 •HCl | $^1$H NMR (400 MHz, Methanol-$d_4$) δ 7.17 (s, 1H), 4.02 (t, *J* = 6.1 Hz, 1H), 3.95 - 3.85 (m, 4H), 3.32 - 3.19 (m, 4H), 2.65 - 2.48 (m, 2H), 2.27 (s, 3H), 2.23 - 2.02 (m, 2H) ppm. | $[M - H]^-$ 290.2 0.932 min. |
| 22 | 3-(5-ethyl-6-(piperazin-1-yl) pyridazin-3-yl) piperidine-2,6-dione hy-drochloride salt | 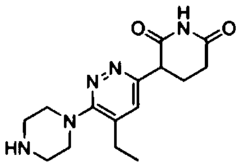 •HCl | $^1$H NMR (400 MHz, Methanol-$d_4$) δ 7.18 (d, *J* = 0.6 Hz, 1H), 4.05 (td, *J* = 6.1, 0.7 Hz, 1H), 4.00 (dd, *J* = 5.9, 3.1 Hz, 2H), 3.90 (dd, *J* = 5.7, 3.1 Hz, 2H), 3.31 - 3.23 (m, 4H), 2.78 - 2.51 (m, 4H), 2.26 - 2.03 (m, 2H), 1.23 (t, *J* = 7.9 Hz, 3H) ppm. | $[M - H]^-$ 304.2 0.955 min. |
| 23 | 3-(5-isopropyl-6-(piperazi n-1-yl)pyridazin-3-yl) piper idine-2,6-dione hy-drochloride salt | 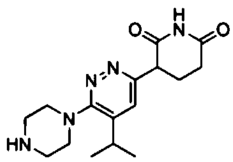 •HCl | $^1$H NMR (400 MHz, Methanol-$d_4$) δ 7.29 (s, 1H), 4.02 (t, *J* = 6.2 Hz, 1H), 4.00 - 3.89 (m, 4H), 3.30 - 3.22 (m, 4H), 3.18 (h, *J* = 7.1 Hz, 1H), 2.67 - 2.46 (m, 2H), 2.29 - 1.95 (m, 2H), 1.36 (d, *J* = 7.3 Hz, 3H), 1.31 (d, *J* = 7.2 Hz, 3H) ppm. | $[M - H]^-$ 318.2 0.970 min. |
| 24 | 3-(5-cyclopropyl-6-(pi-pera zin-1-yl)pyrida-zin-3-yl)pip eridine-2,6-dione hydrochloride salt | 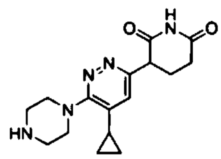 •HCl | $^1$H NMR (400 MHz, Methanol-$d_4$) δ 7.33 (s, 1H), 4.02 (t, *J* = 6.1 Hz, 1H), 4.00 - 3.89 (m, 4H), 3.33 - 3.20 (m, 4H), 3.04 - 2.88 (m, 1H), 2.66 - 2.47 (m, 2H), 2.25 - 2.01 (m, 2H), 1.20 - 1.01 (m, 2H), 0.93 - 0.74 (m, 2H) ppm. | $[M - H]^-$ 316.2 0.965 min. |
| 25 | 3-(5-fluoro-6-(piperazin-1-yl)pyridazin-3-yl)piperidin e-2,6-dione hy-drochloride salt | 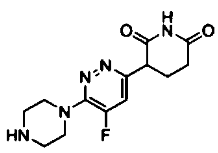 •HCl | $^1$H NMR (400 MHz, Methanol-$d_4$) δ 7.11 (d, *J* = 7.9 Hz, 1H), 4.07 (t, *J* = 6.1 Hz, 1H), 3.98 - 3.88 (m, 4H), 3.29 - 3.18 (m, 4H), 2.63 - 2.49 (m, 2H), 2.23 - 2.09 (m, 2H) ppm. | $[M - H]^-$ 294.1 0.915 min. |

(continued)

| ID | IUPAC Name | Structure | $^1$H NMR | UPLC-MS m/z, $t_R$ |
|---|---|---|---|---|
| 26 | 6-(2,6-dioxopiperidin-3-yl) -3-(piperazin-1-yl) pyridazi ne-4-carbonitrile hydrochloride salt | 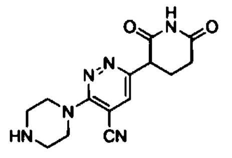 •HCl | 4.06 4H), 2.07 4H), 2.07 $^1$H NMR (400 MHz, Methanol-$d_4$) δ 7.67 (s, 1H), 4.06 (t, $J$ = 6.2 Hz, 0H), 4.01 - 3.90 (m, 4H), 3.28 - 3.19 (m, 4H), 2.65 - 2.48 (m, 2H), 2.24 - 2.07 (m, 2H) ppm. | $[M - H]^-$ 301.1 0.932 min. |
| 27 | 3-(6-(piperazin-1-yl)-5-(trif luoromethyl) pyridazin-3-yl )piperidine-2,6-dione hydrochloride salt 3-(6-(piperazin-1-yl)-5-(trif | 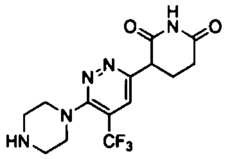 •HCl | $^1$H NMR (400 MHz, Methanol-$d_4$) δ 7.62 (s, 1H), 4.05 (t, $J$ = 6.2 Hz, 1H), 4.00 - 3.89 (m, 4H), 3.32 - 3.20 (m, 4H), 2.67 - 2.48 (m, 2H), 2.24 - 2.04 (m, 2H) ppm. | $[M - H]^-$ 344.1 0.942 min. |
| 28 | 3-(5-methoxy-6-(piperazi n-1-yl)pyridazin-3-yl) piper idine-2,6-dione hydrochloride salt | 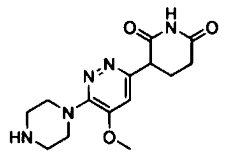 •HCl | $^1$H NMR (400 MHz, Methanol-$d_4$) δ 6.99 (s, 1H), 4.04 - 3.86 (m, 8H), 3.29 - 3.20 (m, 4H), 2.65 - 2.49 (m, 2H), 2.22 - 2.11 (m, 2H) ppm. | $[M - H]^-$ 306.2 0.921 min. |
| 29 | 3-(5-amino-6-(piperazin-1 -yl)pyridazin-3-yl) piperidin e-2,6-dione hydrochloride salt | 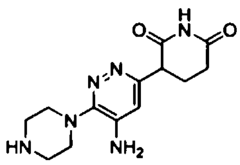 •HCl | $^1$H NMR (400 MHz, Methanol-$d_4$) δ 6.84 (s, 1H), 4.03 - 3.87 (m, 5H), 3.30 - 3.15 (m, 4H), 2.70 - 2.46 (m, 2H), 2.24 - 2.04 (m, 2H) ppm. | $[M - H]^-$ 291.2 0.788 min. |
| 30 | 3-(5-((dimethylamino) met hyl)-6-(piperazin-1-yl)pyri dazin-3-yl)piperidine-2,6-dione bis-hydrochloride salt | 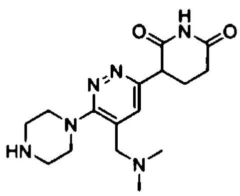 •2HCl | $^1$H NMR (400 MHz, Methanol-$d_4$) δ 7.64 (s, 1H), 4.41 (d, $J$ = 1.3 Hz, 2H), 4.05 (t, $J$ = 6.2 Hz, 1H), 4.01 - 3.89 (m, 4H), 3.29 - 3.20 (m, 4H), 2.71 (s, 6H), 2.63 - 2.52 (m, 2H), 2.24 - 2.09 (m, 2H) ppm. | $[M + 2H]^{2+}$ 167.1 0.741 min. |
| 31 | 3-(5-phenyl-6-(piperazin-1-yl)pyridazin-3-yl)piperidi ne-2,6-dione hydrochloride salt | 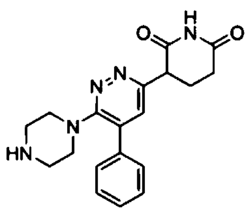 •HCl | $^1$H NMR (400 MHz, Methanol-$d_4$) δ 7.68 - 7.59 (m, 3H), 7.51 - 7.45 (m, 2H), 7.43 - 7.37 (m, 1H), 4.09 (t, $J$ = 6.2 Hz, 1H), 4.03 - 3.89 (m, 4H), 3.30 - 3.19 (m, 4H), 2.65 - 2.48 (m, 2H), 2.24 - 2.08 (m, 2H) ppm. | $[M - H]^-$ 352.2 0.986 min. |
| 32 | 3-(6-(piperazin-1-yl)-5-(py ridin-4-yl)pyridazin-3-yl)pi peridine-2,6-dione hydrochloride salt | 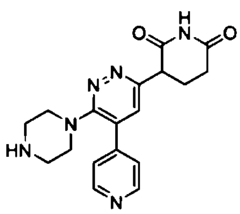 •HCl | $^1$H NMR (400 MHz, Methanol-$d_4$) δ 8.66 (d, $J$ = 4.6 Hz, 2H), 7.66 (s, 1H), 7.57 (d, $J$ = 4.6 Hz, 2H), 4.09 (t, $J$ = 6.2 Hz, 1H), 4.02 - 3.91 (m, 4H), 3.32 - 3.18 (m, 4H), 2.68 - 2.49 (m, 2H), 2.26 - 2.08 (m, 2H) ppm. | $[M - H]^-$ 353.2 0.880 min. |

(continued)

| ID | IUPAC Name | Structure | $^1$H NMR | UPLC-MS m/z, $t_R$ |
|---|---|---|---|---|
| 33 | 3-(4,5-dimethyl-6-(pi-pera zin-1-yl)pyrida-zin-3-yl)pip eridine-2,6-dione hydrochloride salt | 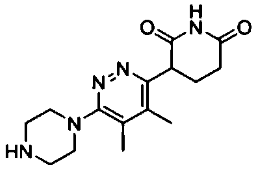 •HCl | $^1$H NMR (400 MHz, Methanol-$d_4$) δ 4.05 (t, *J* = 6.0 Hz, 1H), 3.93 - 3.79 (m, 4H), 3.32 - 3.18 (m, 4H), 2.69 - 2.52 (m, 2H), 2.43 (s, 3H), 2.24 - 2.10 (m, 5H) ppm. | $[M - H]^-$ 304.2 1.103 min |
| 34 | 3-(4-(piperazin-1-yl)-6,7-d ihydro-5H-cyclopenta [d]p yridazin-1-yl)piperi-dine-2, 6-dione hydro-chloride salt | 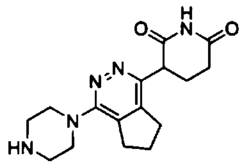 •HCl | $^1$H NMR (400 MHz, Methanol-$d_4$) δ 4.06 (t, *J* = 6.4 Hz, 1H), 3.95 - 3.84 (m, 4H), 3.33 - 3.22 (m, 4H), 2.99 - 2.82 (m, 4H), 2.67 - 2.52 (m, 2H), 2.38 - 2.10 (m, 4H) ppm. | $[M - H]^-$ 316.2 1.116 min |
| 35 | 3-(4-(piperazin-1-yl)-5,6,7 ,8-tetrahy-drophthalazin-1-yl)piper-idine-2,6-dione hydro-chloride salt | 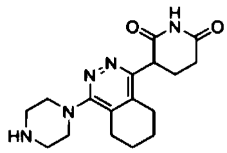 •HCl | $^1$H NMR (400 MHz, Methanol-$d_4$) δ 4.06 (t, *J* = 6.4 Hz, 1H), 3.96 - 3.84 (m, 4H), 3.34 - 3.21 (m, 4H), 3.14 - 2.87 (m, 4H), 2.67 - 2.51 (m, 2H), 2.26 - 2.09 (m, 2H), 1.93 - 1.78 (m, 4H) ppm. | $[M - H]^-$ 330.2 1.120 min |
| 36 | 3-(4-(piperazin-1-yl) phtha lazin-1-yl)piperidine-2,6-d ione hydro-chloride salt | 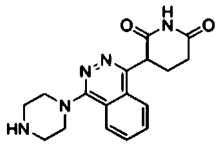 •HCl | $^1$H NMR (400 MHz, Methanol-$d_4$) δ 8.31 (dd, *J* = 8.8, 1.3 Hz, 1H), 8.10 (dd, *J* = 9.1, 1.4 Hz, 1H), 7.86 (ddd, *J* = 9.1, 7.8, 1.3 Hz, 1H), 7.72 (ddd, *J* = 9.0, 7.9, 1.3 Hz, 1H), 4.05 - 3.95 (m, 4H), 3.79 (t, *J* = 6.2 Hz, 1H), 3.28 - 3.21 (m, 4H), 2.70 - 2.49 (m, 2H), 2.26 - 2.11 (m, 2H) ppm. | $[M - H]^-$ 326.1 1.019 min |
| 37 | 3-(8-(piperazin-1-yl)pyrazi n o[2,3-d]pyridazin-5-yl)pip eridine-2,6-dione hydrochloride salt | 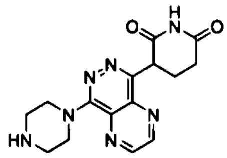 •HCl | $^1$H NMR (400 MHz, Methanol-$d_4$) δ 8.73 (d, *J* = 3.5 Hz, 1H), 8.72 (d, *J* = 3.5 Hz, 1H), 4.03 - 3.91 (m, 4H), 3.84 (t, *J* = 6.6 Hz, 1H), 3.29 - 3.17 (m, 4H), 2.69 - 2.50 (m, 2H), 2.38 - 2.17 (m, 2H) ppm. | $[M - H]^-$ 328.1 0.874 min |
| 38 | 3-fluoro-3-(6-(piperazin-1-yl)pyridazin-3-yl)piperidin e-2,6-dione hy-drochloride salt | 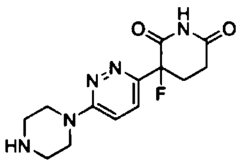 •HCl | $^1$H NMR (400 MHz, Methanol-*d*4) δ 7.45 (d, *J* = 7.2 Hz, 1H), 6.99 (d, *J* = *7.3* Hz, 1H), 4.01 - 3.82 (m, 5H), 3.34 - 3.17 (m, 4H), 2.74 - 2.49 (m, 2H), 2.44 - 2.08 (m, 2H) ppm. | $[M - H]^-$ 294.1 0.965 min |
| 39 | 3-(6-(piperazin-1-yl)pyrid azin-3-yl)piperidine-2,6-di one-3-dhydrochloride salt | 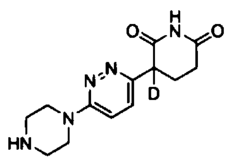 •HCl | $^1$H NMR (400 MHz, Methanol-$d_4$) δ 7.28 (d, *J* = 8.6 Hz, 1H), 6.93 (d, *J* = 8.6 Hz, 1H), 3.96 - 3.83 (m, 4H), 3.41 - 3.15 (m, 4H), 2.78 - 2.47 (m, 2H), 2.28 - 2.06 (m, 2H) ppm. | $[M - H]^-$ 277.2 0.977 min |

(continued)

| ID | IUPAC Name | Structure | $^1H$ NMR | UPLC-MS m/z, $t_R$ |
|---|---|---|---|---|
| 40 | 1-(6-(piperazin-1-yl)pyridazin-3-yl)dihydropyrimidin e-2,4(1*H*,3*H*)-dione hydrochloride salt | 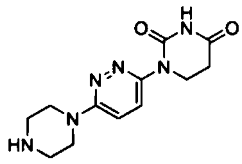 •HCl | 4.12 $^1H$ NMR (400 MHz, Methanol-$d_4$) δ 7.52 (d, *J* = 7.9 Hz, 1H), 7.09 (d, *J* = 7.9 Hz, 1H), 4.12 - 3.82 (m, 6H), 3.47 - 3.14 (m, 4H), 2.74 (dd, *J* = 7.0, 4.2 Hz, 2H) ppm. | [M - H]-277.1 0.768 min |
| 41 | 3-(6-methoxy-5-(piperazi n-1-yl)pyridazin-3-yl) piper idine-2,6-dione hydrochloride salt | 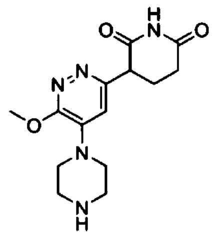 •HCl | $^1H$ NMR (400 MHz, Methanol-$d_4$) δ 7.02 (d, *J* = 0.6 Hz, 1H), 4.02 - 3.94 (m, 4H), 3.49 - 3.38 (m, 9H), 2.62 - 2.51 (m, 2H), 2.16 (dtd, *J* = 9.7, 6.2, 2.1 Hz, 2H) ppm. | $[M - H]^-$ 306.2 0.682 min |
| 42 | 3-(6-(dimethylamino)-5-(p iperazin-1-yl) pyridazin-3-yl)piperidine-2,6-dione hydrochloride salt | 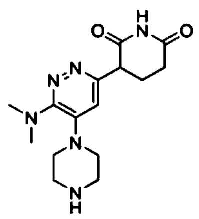 •HCl | $^1H$ NMR (400 MHz, Methanol-$d_4$) δ 6.79 (d, *J* = 0.6 Hz, 1H), 3.98 (td, *J* = 6.2, 0.7 Hz, 1H), 3.80 - 3.71 (m, 2H), 3.54 - 3.48 (m, 2H), 3.46 - 3.36 (m, 4H), 3.15 (s, 6H), 2.63 - 2.52 (m, 2H), 2.16 (dtd, *J* = 9.7, 6.2, 2.1 Hz, 2H) ppm. | $[M - H]^-$ 319.2 0.595 min |
| 43 | 1-methyl-3-(6-(piperazin-1-yl)pyridazin-3-yl)piperidi ne- 2,6-dione hydrochloride salt | 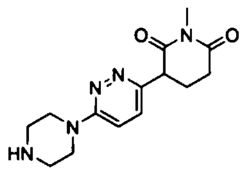 •HCl | $^1H$ NMR (400 MHz, Methanol-$d_4$) δ 7.47 (dd, *J* = 8.6, 0.6 Hz, 1H), 6.95 (d, *J* = 8.3 Hz, 1H), 4.03 (td, *J* = 6.5, 0.7 Hz, 1H), 3.94 - 3.81 (m, 4H), 3.31 - 3.18 (m, 4H), 3.12 (s, 3H), 2.68 - 2.51 (m, 2H), 2.26 - 2.09 (m, 2H) ppm. | $[M - H]^-$ 290.2 1.021 min. |

Table 2 Exemplary compound structures of PROTAC protein degraders

| Compound | Structure | 1H NMR |
|---|---|---|
| **PRTB-01** | 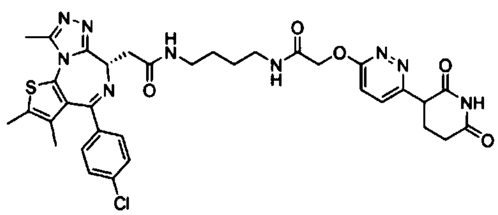 | $^1H$ NMR (400 MHz, DMSO-$d_6$) δ 11.07 (s, 1H), 7.74 - 7.62 (m, 4H), 7.62 - 7.55 (m, 1H), 7.55 - 7.48 (m, 2H), 7.09 (d, *J* = 8.1 Hz, 1H), 5.78 (t, *J* = 9.3 Hz, 1H), 4.69 (d, *J* = 0.6 Hz, 2H), 3.73 - 3.65 (m, 1H), 3.24 - 2.98 (m, 6H), 2.63 (s, 3H), 2.59 - 2.48 (m, 2H), 2.21 - 2.04 (m, 2H), 1.45 (p, *J* = 2.8 Hz, 4H) ppm. |
| **PRTB-02** | 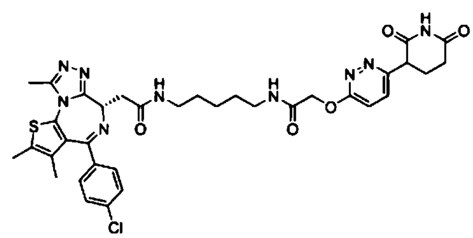 | $^1H$ NMR (400 MHz, DMSO-$d_6$) δ 11.07 (s, 1H), 7.71 (t, *J* = 4.8 Hz, 1H), 7.71 - 7.62 (m, 3H), 7.62 - 7.55 (m, 1H), 7.55 - 7.48 (m, 2H), 7.09 (d, *J* = 8.1 Hz, 1H), 5.78 (t, *J* = 9.3 Hz, 1H), 4.69 (d, *J* = 0.6 Hz, 2H), 3.73 - 3.65 (m, 1H), 3.22 - 2.98 (m, 7H), 2.63 (s, 3H), 2.59 - 2.48 (m, 2H), 2.23 - 2.04 (m, 2H), 1.53 - 1.41 (m, 4H), 1.39 - 1.27 (m, 2H) ppm. |

(continued)

| Compound | Structure | 1H NMR |
|---|---|---|
| **PRTB-03** | 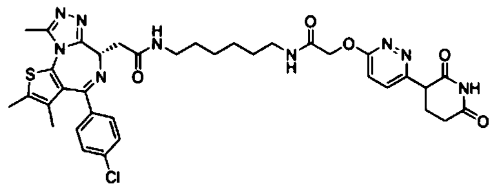 | $^1$H NMR (400 MHz, DMSO-$d_6$) δ 11.07 (s, 1H), 7.70 (t, *J* = 4.7 Hz, 1H), 7.71 - 7.62 (m, 3H), 7.62 - 7.55 (m, 1H), 7.55 - 7.48 (m, 2H), 7.09 (d, *J* = 8.1 Hz, 1H), 5.78 (t, *J* = *9.3* Hz, 1H), 4.69 (d, *J* = 0.7 Hz, 2H), 3.73 - 3.65 (m, 1H), 3.23 - 3.10 (m, 4H), 3.13 - 3.04 (m, 1H), 3.08 - 2.98 (m, 1H), 2.63 (s, 3H), 2.59 - 2.48 (m, 2H), 2.23 - 2.04 (m, 2H), 1.58 - 1.45 (m, 4H), 1.40 - 1.27 (m, 4H) ppm. |
| **PRTB-04** | 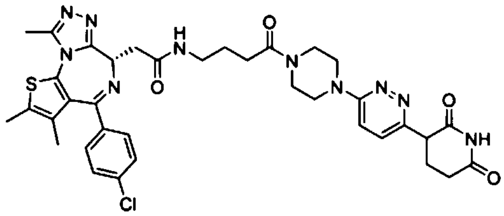 | 2H), $^1$H NMR (400 MHz, DMSO-$d_6$) δ 11.07 (s, 1H), 7.78 (d, *J* = 9.5 Hz, 1H), 7.78 (s, 1H), 7.69 - 7.62 (m, 2H), 7.55 - 7.46 (m, 2H), 7.46 (d, *J* = 0.7 Hz, 1H), 6.92 (d, *J* = 3.3 Hz, 1H), 5.78 (t, *J* = 9.3 Hz, 1H), 3.72 - 3.52 (m, 11H), 3.13 (td, *J* = 6.0, 4.7 Hz, 2H), 3.07 (d, *J* = 5.9 Hz, 1H), 3.08 - 2.98 (m, 1H), 2.63 (s, 3H), 2.59 - 2.48 (m, 2H), 2.29 (d, *J* = 0.9 Hz, 1H), 2.21 - 2.04 (m, 2H), 1.76 (tt, *J* = 7.7, 5.9 Hz, 2H) ppm. |
| **PRTB-05** | 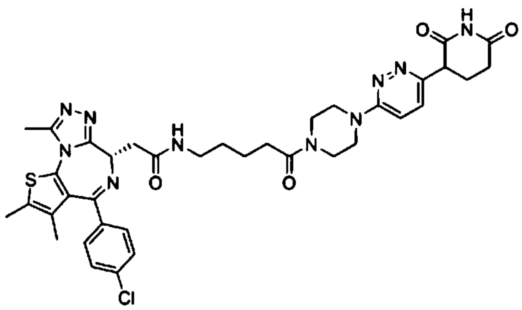 | $^1$H NMR (400 MHz, DMSO-$d_6$) δ 11.07 (s, 1H), 7.71 - 7.62 (m, 2H), 7.55 - 7.44 (m, 2H), 6.92 (d, *J* = *8.3* Hz, 1H), 5.78 (t, *J* = 9.3 Hz, 1H), 3.72 - 3.52 (m, 7H), 3.20 - 3.12 (m, 1H), 3.16 - 3.06 (m, 1H), 3.09 - 2.98 (m, 1H), 2.63 (s, 2H), 2.58 - 2.49 (m, 1H), 2.28 - 2.21 (m, 1H), 2.25 - 2.04 (m, 2H), 1.60 -1.46 (m, 3H) ppm. |
| **PRTB-06** | 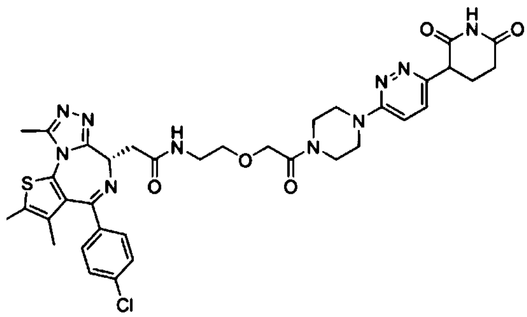 | $^1$H NMR (400 MHz, DMSO-$d_6$) δ 11.07 (s, 1H), 7.76 (t, *J* = 4.8 Hz, 1H), 7.69 - 7.62 (m, 2H), 7.55 - 7.48 (m, 2H), 7.51 - 7.44 (m, 1H), 6.92 (d, *J* = 8.3 Hz, 1H), 5.78 (t, *J* = 9.3 Hz, 1H), 4.13 (s, 2H), 3.72 - 3.58 (m, 9H), 3.62 - 3.55 (m, 2H), 3.40 - 3.24 (m, 2H), 3.14 - 2.98 (m, 2H), 2.63 (s, 3H), 2.59 - 2.48 (m, 2H), 2.21 - 2.04 (m, 2H) ppm. |
| **PRTB-07** | 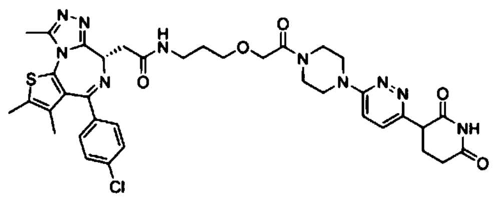 | $^1$H NMR (400 MHz, DMSO-$d_6$) δ 11.07 (s, 1H), 7.69 (t, *J* = 4.6 Hz, 1H), 7.69 - 7.62 (m, 2H), 7.55 - 7.44 (m, 3H), 6.92 (d, *J* = 8.3 Hz, 1H), 5.78 (t, *J* = 9.3 Hz, 1H), 4.14 (s, 2H), 3.72 - 3.51 (m, 11H), 3.19 (td, *J* = 6.1, 4.6 Hz, 2H), 3.14 - 2.98 (m, 2H), 2.59 - 2.48 (m, 2H), 2.22 - 2.04 (m, 2H), 1.80 (pd, *J* = 6.0, 2.5 Hz, 2H) ppm. |

(continued)

| Compound | Structure | 1H NMR |
|---|---|---|
| **PRTB-08** | 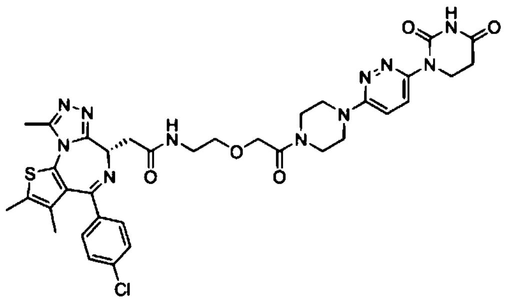 | $^1$H NMR (400 MHz, DMSO-$d_6$) δ 10.64 (s, 1H), 7.76 (t, *J* = 4.8 Hz, 1H), 7.69 - 7.62 (m, 2H), 7.55 - 7.48 (m, 2H), 7.06 (d, *J* = 7.9 Hz, 1H), 7.01 (d, *J*= *7.9* Hz, 1H), 5.78 (t, *J* = 9.3 Hz, 1H), 4.13 (s, 2H), 3.95 (ddd, *J* = 16.5, 7.0, 4.2 Hz, 2H), 3.71 - 3.55 (m, 11H), 3.39 - 3.25 (m, 2H), 3.14 - 2.98 (m, 2H), 2.71 (ddd, *J* = 7.3, 4.2, 3.3 Hz, 2H), 2.31 (d, *J* = 1.5 Hz, 6H) ppm. |
| **PRTB-09** | 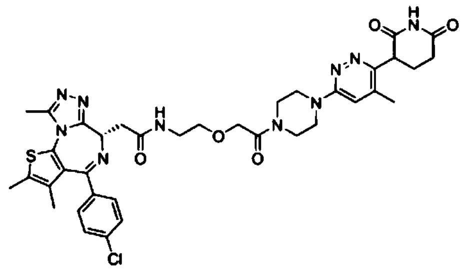 | 2H), $^1$H NMR (400 MHz, DMSO-$d_6$) δ 11.13 (s, 1H), 7.76 (t, *J* = 4.8 Hz, 1H), 7.69 - 7.62 (m, 2H), 7.55 - 7.48 (m, 2H), 6.67 (d, *J* = 0.9 Hz, 1H), 5.78 (t, *J* = 9.3 Hz, 1H), 4.15 - 4.07 (m, 3H), 3.72 (dd, *J* = 6.7, 3.8 Hz, 2H), 3.69 - 3.63 (m, 4H), 3.60 (ddd, *J* = 11.9, 6.7, 3.8 Hz, 4H), 3.39 - 3.25 (m, 2H), 3.14 - 2.98 (m, 2H), 2.64 - 2.48 (m, 2H), 2.40 (d, *J* = 0.7 Hz, 3H), 2.31 (d, *J* = 1.5 Hz, 6H), 2.22 - 2.05 (m, 2H). |
| **PRTB-10** | 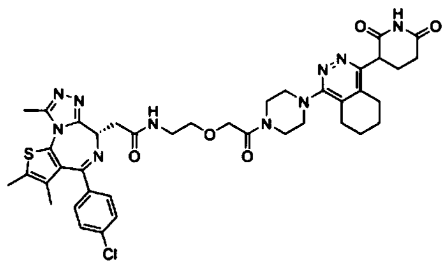 | $^1$H NMR (400 MHz, DMSO-$d_6$) δ 11.12 (s, 1H), 7.76 (t, *J* = 4.8 Hz, 1H), 7.69 - 7.62 (m, 2H), 7.55 - 7.48 (m, 2H), 5.78 (t, *J* = 9.3 Hz, 1H), 4.13 (s, 2H), 4.06 (t, *J* = 6.4 Hz, 1H), 3.75 - 3.54 (m, 11H), 3.39 - 3.25 (m, 2H), 3.14 - 2.96 (m, 4H), 2.95 - 2.85 (m, 2H), 2.64 - 2.48 (m, 2H), 2.31 (d, *J* = 1.5 Hz, 6H), 2.25 - 2.07 (m, 2H), 1.90 - 1.78 (m, 4H) ppm. |
| **PRTB-11** | 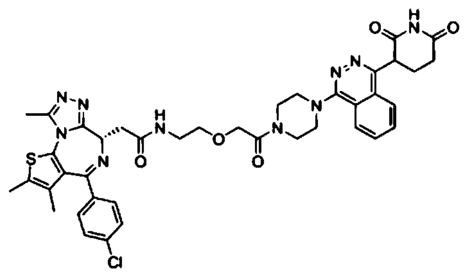 | $^1$H NMR (400 MHz, DMSO-$d_6$) δ 11.17 (s, 1H), 8.33 (dd, *J* = 8.8, 1.3 Hz, 1H), 8.13 (dd, *J* = 8.6, 1.3 Hz, 1H), 7.83 (ddd, *J* = 9.1, 7.8, 1.3 Hz, 1H), 7.76 (t, *J* = 4.8 Hz, 1H), 7.71 (ddd, *J* = 9.0, 7.7, 1.3 Hz, 1H), 7.69 - 7.62 (m, 2H), 7.55 - 7.48 (m, 2H), 5.78 (t, *J* = 9.3 Hz, 1H), 4.13 (s, 2H), 3.83 - 3.70 (m, 5H), 3.67 (t, *J* = 4.3 Hz, 2H), 3.63 - 3.54 (m, 4H), 3.40 - 3.24 (m, 2H), 3.14 - 2.98 (m, 2H), 2.63 (s, 3H), 2.62 - 2.48 (m, 2H), 2.31 (s, 3H), 2.25 - 2.08 (m, 2H) ppm. |
| **PRTB-12** | 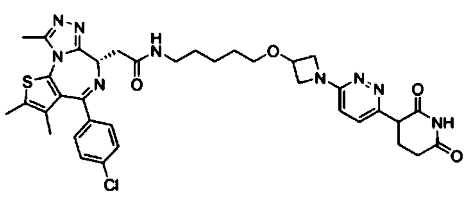 | $^1$H NMR (400 MHz, DMSO-$d_6$) δ 11.07 (s, 1H), 7.71 - 7.62 (m, 3H), 7.55 - 7.48 (m, 2H), 7.51 - 7.44 (m, 1H), 6.94 (d, *J* = 8.6 Hz, 1H), 5.78 (t, *J* = 9.3 Hz, 1H), 4.21 (p, *J* = 3.7 Hz, 1H), 3.93 (dd, *J* = 12.3, 3.7 Hz, 2H), 3.79 (dd, *J* = 12.5, 3.8 Hz, 2H), 3.68 (td, *J* = 6.2, 0.7 Hz, 1H), 3.48 (t, *J* = 6.2 Hz, 2H), 3.13 (qd, *J* = 5.4, 1.6 Hz, 2H), 3.12 - 3.04 (m, 1H), 3.08 - 2.98 (m, 1H), 2.63 (s, 3H), 2.59 - 2.48 (m, 2H), 2.22 - 2.04 (m, 2H), 1.62 - 1.48 (m, 2H), 1.53 - 1.33 (m, 4H) ppm. |

(continued)

| Compound | Structure | 1H NMR |
|---|---|---|
| **PRTB-13** | 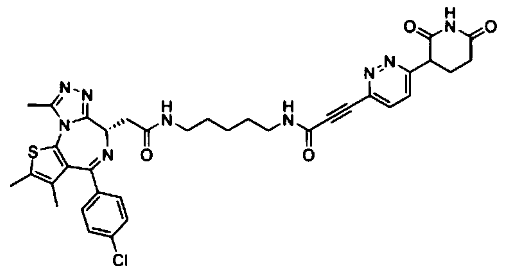 | $^1$H NMR (400 MHz, DMSO-$d_6$) δ 11.07 (s, 1H), 7.97 (t, *J* = 4.6 Hz, 1H), 7.74 - 7.62 (m, 5H), 7.55 - 7.48 (m, 2H), 5.78 (t, *J* = 9.3 Hz, 1H), 3.69 (t, *J* = 6.2 Hz, 1H), 3.25 - 2.98 (m, 6H), 2.63 (s, 3H), 2.59 - 2.48 (m, 2H), 2.22 - 2.04 (m, 2H), 1.60 - 1.41 (m, 4H), 1.38 - 1.26 (m, 2H). |
| **PRTB-14** | 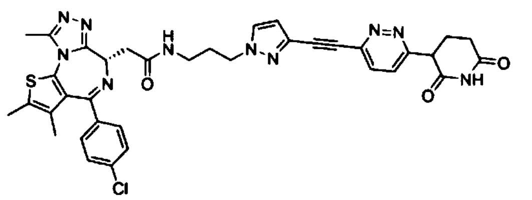 | $^1$H NMR (400 MHz, DMSO-$d_6$) δ 11.07 (s, 1H), 7.75 (t, *J* = 3.8 Hz, 1H), 7.72 - 7.62 (m, 4H), 7.56 (d, *J* = 3.3 Hz, 1H), 7.54 - 7.48 (m, 2H), 6.37 (d, *J* = 3.1 Hz, 1H), 5.78 (t, *J = 9.3* Hz, 1H), 4.10 (td, J = 5.3, 0.7 Hz, 2H), 3.72 - 3.65 (m, 1H), 3.20 - 2.98 (m, 4H), 2.63 (s, 3H), 2.59 - 2.48 (m, 2H), 2.22 - 2.04 (m, 2H), 1.96 (p, *J* = 5.5 Hz, 2H) ppm. |
| **PRTB-15** | 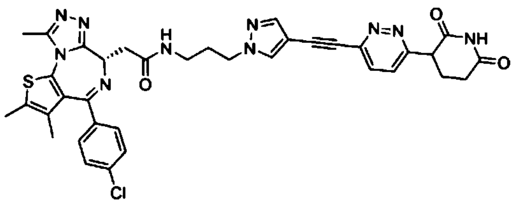 | $^1$H NMR (400 MHz, DMSO-$d_6$) δ 11.07 (s, 1H), 7.75 (t, *J* = 3.8 Hz, 1H), 7.73 - 7.65 (m, 2H), 7.69 - 7.61 (m, 4H), 7.55 - 7.48 (m, 2H), 5.78 (t, *J* = 9.3 Hz, 1H), 4.10 (td, *J* = 5.4, 1.2 Hz, 2H), 3.72 - 3.65 (m, 1H), 3.16 (td, *J* = 5.7, 3.8 Hz, 2H), 3.15 - 2.98 (m, 2H), 2.59 - 2.48 (m, 2H), 2.22 - 2.04 (m, 2H), 1.96 (p, *J* = 5.5 Hz, 2H) ppm. |
| **PRTB-16** | 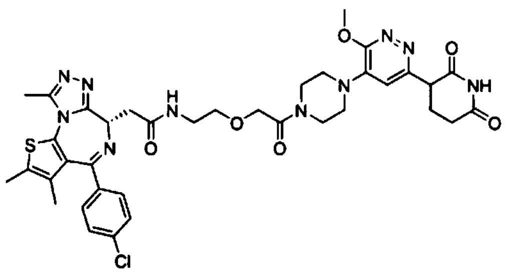 | $^1$H NMR (400 MHz, DMSO-$d_6$) δ 11.08 (s, 1H), 7.76 (t, *J* = 4.8 Hz, 1H), 7.69 - 7.62 (m, 2H), 7.55 - 7.48 (m, 2H), 7.02 (d, *J* = 0.6 Hz, 1H), 5.78 (t, *J* = 9.3 Hz, 1H), 4.13 (s, 2H), 4.03 - 3.95 (m, 1H), 3.98 (s, 3H), 3.70 - 3.55 (m, 6H), 3.40 - 3.18 (m, 7H), 3.14 - 2.98 (m, 2H), 2.63 (s, 3H), 2.59 - 2.49 (m, 2H), 2.31 (s, 3H), 2.22 - 2.05 (m, 2H) ppm. |
| **PRTA-01** | 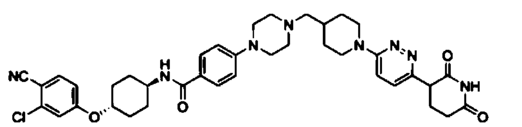 | $^1$H NMR (400 MHz, DMSO-$d_6$) δ 11.07 (s, 1H), 7.83 (d, *J* = 8.8 Hz, 1H), 7.71 (d, *J* = 3.9 Hz, 1H), 7.68 - 7.62 (m, 2H), 7.51 - 7.44 (m, 1H), 7.16 (d, *J* = 2.2 Hz, 1H), 7.09 - 7.02 (m, 2H), 6.98 - 6.86 (m, 2H), 4.02 - 3.89 (m, 3H), 3.82 (ddd, *J* = 12.4, 8.1, 6.9 Hz, 2H), 3.75 - 3.65 (m, 2H), 3.21 (ddd, *J* = 7.5, 5.7, 3.0 Hz, 5H), 2.65 - 2.45 (m, 9H), 2.22 - 2.04 (m, 2H), 2.02 - 1.63 (m, 16H) ppm. |

(continued)

| Compound | Structure | 1H NMR |
|---|---|---|
| PRTA-02 | 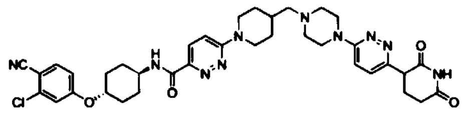 | $^1$H NMR (400 MHz, DMSO-$d_6$) δ 11.07 (s, 1H), 7.93 - 7.82 (m, 2H), 7.71 (d, *J* = 8.8 Hz, 1H), 7.47 (dd, *J* = 8.5, 0.6 Hz, 1H), 7.23 - 7.14 (m, 2H), 6.98 - 6.88 (m, 2H), 4.02 - 3.92 (m, 2H), 3.96 - 3.89 (m, 1H), 3.88 - 3.77 (m, 2H), 3.81 - 3.72 (m, 1H), 3.68 (td, *J* = 6.2, 0.6 Hz, 1H), 3.65 - 3.51 (m, 4H), 2.73 - 2.64 (m, 2H), 2.67 - 2.61 (m, 2H), 2.64 - 2.57 (m, 1H), 2.53 (ddd, *J* = 8.2, 7.4, 0.9 Hz, 2H), 2.49 (dd, *J* = 11.5, 4.9 Hz, 1H), 2.22 -2.04 (m, 2H), 2.02 - 1.62 (m, 13H) ppm. |
| PRTA-03 | 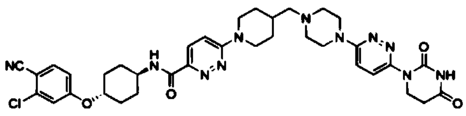 | $^1$H NMR (400 MHz, DMSO-$d_6$) δ 10.64 (s, 1H), 7.93 - 7.82 (m, 2H), 7.71 (d, *J* = 8.8 Hz, 1H), 7.23 - 7.14 (m, 2H), 7.06 (d, *J* = 3.0 Hz, 1H), 7.00 (d, *J* = 8.0 Hz, 1H), 6.95 (dd, *J* = 8.8, 2.2 Hz, 1H), 4.02 - 3.95 (m, 1H), 3.99 - 3.89 (m, 4H), 3.84 (d, *J* = 7.8 Hz, 1H), 3.84 - 3.78 (m, 1H), 3.82 - 3.72 (m, 1H), 3.66 - 3.51 (m, 4H), 2.75 - 2.57 (m, 7H), 2.49 (dd, *J* = 11.4, 4.9 Hz, 1H), 2.02 - 1.62 (m, 13H) ppm. |
| PRTA-04 | 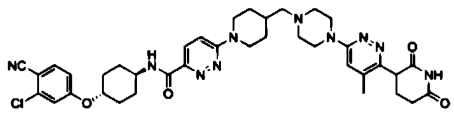 | $^1$H NMR (400 MHz, DMSO-$d_6$) δ 11.13 (s, 1H), 7.93 - 7.82 (m, 2H), 7.71 (d, *J* = 8.8 Hz, 1H), 7.23 - 7.14 (m, 2H), 6.95 (dd, J = 8.8, 2.3 Hz, 1H), 6.67 (d, *J* = 0.6 Hz, 1H), 4.11 (t, *J* = 6.1 Hz, 1H), 4.02 - 3.92 (m, 2H), 3.96 - 3.89 (m, 1H), 3.88 - 3.77 (m, 2H), 3.81 - 3.71 (m, 1H), 3.67 - 3.57 (m, 2H), 3.61 - 3.52 (m, 2H), 2.73 - 2.45 (m, 8H), 2.23 -2.04 (m, 2H), 2.02 - 1.62 (m, 14H) ppm. |
| PRTA-05 | 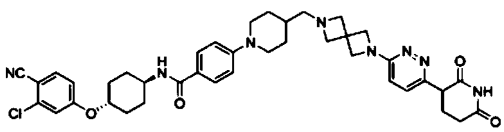 | $^1$H NMR (400 MHz, DMSO-$d_6$) δ 11.07 (s, 1H), 7.83 (d, *J* = 8.8 Hz, 1H), 7.71 (d, *J* = 8.9 Hz, 1H), 7.68 - 7.61 (m, 2H), 7.51 - 7.44 (m, 1H), 7.16 (d, *J* = 2.2 Hz, 1H), 7.09 - 7.02 (m, 2H), 6.95 (dd, *J* = 8.8, 2.2 Hz, 1H), 6.89 (d, *J* = 8.6 Hz, 1H), 3.98 - 3.90 (m, 1H), 3.93 (s, 2H), 3.87 (s, 2H), 3.75 - 3.64 (m, 2H), 3.42 (ddd, *J* = 12.3, 8.3, 6.1 Hz, 2H), 3.09 (ddd, *J* = 12.3, 8.2, 6.2 Hz, 2H), 2.60 (dd, *J* = 11.2, 3.7 Hz, 1H), 2.59 - 2.48 (m, 3H), 2.22 - 2.04 (m, 2H), 2.02 - 1.88 (m, 2H), 1.88 - 1.63 (m, 11H) ppm. |
| PRTA-06 | 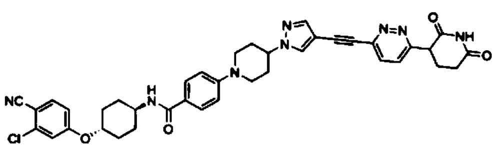 | $^1$H NMR (400 MHz, DMSO-$d_6$) δ 11.07 (s, 1H), 7.87 (d, *J* = 1.8 Hz, 1H), 7.83 (d, *J* = 8.8 Hz, 1H), 7.74 - 7.65 (m, 3H), 7.68 - 7.61 (m, 3H), 7.16 (d, *J* = 2.2 Hz, 1H), 7.09 - 7.02 (m, 2H), 6.95 (dd, *J* = 8.8, 2.2 Hz, 1H), 4.47 (pd, *J* = 3.1, 0.7 Hz, 1H), 3.93 (tt, *J* = 6.2, 3.6 Hz, 1H), 3.75 - 3.56 (m, 6H), 2.59 - 2.48 (m, 2H), 2.22 - 1.63 (m, 15H) ppm. |

As used herein, the term "alkyl" refers to branched and linear saturated aliphatic hydrocarbyl groups having a specified number of carbon atoms, generally 1 to about 12 carbon atoms. Examples of alkyl include, but are not limited to, methyl, ethyl, n-propyl, isopropyl, n-butyl, 3-methylbutyl, tert-butyl, n-pentyl and sec-pentyl.

As used herein, the term "stereoisomer" refers to a compound having the same chemical compositions but

different spatial arrangements of atoms or groups, comprising a "diastereomer" and an "enantiomer".

As used herein, the term "diastereomer" refers to a stereoisomer that has two or more chiral centers and whose molecules are not mirror images of each other. The diastereomer has different physical properties such as melting point, boiling point, spectral characteristics and reactivity. In the presence of a resolving agent or chromatography, a mixture of diastereomers can be separated under high-resolution analytical steps such as electrophoresis and crystallization by using for example a chiral high performance liquid chromatography (HPLC) column.

As used herein, the term "enantiomer" refers to two stereoisomers of a compound that have no superimposable mirror images of each other. A mixture of enantiomers in a ratio of 50:50 is referred to as a racemic mixture or a racemate, which may occur in a situation that there has been no stereoselectivity or stereospecificity during the chemical reaction or treatment.

As used herein, the term "pharmaceutically acceptable salt" and " the salt of the compound" can be interchangeable, both of which are derivatives of the disclosed compound, wherein non-toxic acid or base addition salts are prepared from the parent compound; examples of the pharmaceutically acceptable salts include, but are not limited to, non-toxic acid addition salts: salts formed by using inorganic acids such as hydrochloric acid, hydrobromic acid, phosphoric acid, sulfuric acid and perchloric acid, or organic acids such as acetic acid, maleic acid, tartaric acid, citric acid, succinic acid or malonic acid. Other pharmaceutically acceptable salts include, but are not limited to, adipates, alginates, ascorbates, aspartates, benzenesulfonates, benzoates, bisulfates, borates, butyrates, camphorates, camphorsulfonates, citrates, cyclopentanepropionates, digluconates, dodecylsulfates, ethanesulfonates, formates, fumarates, glucoheptonates, glycerophosphates, gluconates, hemisulfates, heptanoates, hexanoates, hydroiodides, 2-hydroxy-ethanesulfonates, lactobionates, lactates, laurates, lauryl sulfates, malates, maleates, malonates, methanesulfonates, 2-naphthalenesulfonates, nicotinates, nitrates, oleates, oxalates, palmitates, pamoates, pectinates, persulfates, 3-phenylpropionates, phosphates, picrates, pivalates, propionates, stearates, succinates, sulfates, tartrates, thiocyanates, tosylates, undecanoates, valerates, etc. Representative alkali metal or alkaline earth metal salts comprise sodium, lithium, potassium, calcium, magnesium, etc. Where appropriate, other pharmaceutically acceptable salts comprise non-toxic ammonium, quaternary ammonium and amine cations formed by using counterions such as halide ions, hydroxy radicals, carboxylate radicals, sulfate radicals, phosphate radicals, nitrate radicals, alkyl having 1-6 carbon atoms, sulfonate radicals and aryl sulfonate radicals.

## Examples

### Example 1 Synthesis of compound 1

[Step A] Raw materials 1-(6-chloropyridazin-3-yl)ethan-1-one (1.50 g, 9.58 mmol, 1.0 equiv.), 2,6-bis(benzyloxy)-3-(4,4,5,5-tetramethyl-1,3,2-dioxaborolan-2-yl)pyridine (4.80 g, 11.50 mmol, 1.2 equiv.), $K_3PO_4$ (6.22 g, 28.74 mmol, 3.0 equiv.), DavePhos (1.131 g, 2.87 mmol, 30 mol%) and $Pd(OAc)_2$ (215 mg, 0.96 mmol, 10 mol%) were sequentially added to a round-bottom flask. 50 mL of isopropanol-water (2:1, v/v) mixed solution was injected. The reaction system was stirred for 9 h at 80°C (oil bath) under an argon atmosphere. No remaining materials were detected by thin-layer chromatography (TLC). After being cooled to room temperature, the reaction system was diluted with 40 mL of water and then extracted with EtOAc. The organic phases were combined, washed with saturated brine and dried. The dried organic phase was subjected to suction filtration and underwent rotary evaporation under reduced pressure to remove the solvent. The resulting solid was purified by chromatography to obtain 2.68 g of compound 44, with the yield of 68%. MS (ESI) m/z 412.2.

[Step B] The compound 44 (1.00 g, 2.43 mmol), 20 mL of anhydrous ethanol and Pd/C (200 mg, 20 wt%, 10% on carbon) were added to a round-bottom flask. The reaction solution was stirred for 8 h under the pressure of a hydrogen balloon. No remaining materials were detected by TLC, and no unreacted intermediate was detected by liquid chromatography-mass spectrum (LC-MS). The reaction solution was subjected to suction filtration and underwent rotary evaporation to dryness under reduced pressure to obtain 612 mg of crude product compound 45. The crude product was directly used for the next step without purification. MS (ESI) m/z 234.1.

[Step C] p-methoxybenzyl chloride (PMBCl) (360 μL, 2.62 mmol, 1.0 equiv.), $K_2CO_3$ (724 mg, 5.24 mmol, 2.0 equiv.) and tetra-n-butylammonium iodide (TBAI) (193 mg, 0.52 mmol, 0.2 equiv.) were added to a solution of the crude product 45 (612 mg, about 2.62 mmol, about 1.0 equiv.) in acetone (15 mL). The reaction mixed solution was stirred for 12 h until no remaining materials were detected by TLC. The above reaction mixed solution was diluted with 20 mL of water and then extracted with EtOAc. The organic phases were combined, washed with saturated brine and dried. The dried organic phase was subjected to suction filtration and underwent rotary evaporation under reduced pressure to remove the solvent. The resulting solid was purified by chromatography to obtain 515 mg of compound 46, with the yield of 60% for two steps. MS (ESI) m/z 412.2.

[Step D] (Trimethylsilyl) diazomethane (TMSCHN2) (360 μL, 1.90 M in hexanes, 0.68 mmol, 1.2 equiv.) was dropwise added to a solution of lithium diisopropylamide (LDA) in anhydrous tetrahydrofuran (THF) (about 0.68 mmol, 7.0

mL, about 1.2 equiv.) at -78°C under an argon atmosphere. The reaction solution continued to be stirred for 30 min at the same temperature, and then a solution of compound 46 (200 mg, 0.57 mmol, 1.0 equiv.) in anhydrous THF (5 mL) was dropwise added. The reaction solution continued to be stirred for 1 h at the same temperature, then warmed to room temperature and heated to reflux for 3 h. No remaining materials were detected by TLC. After being cooled to room temperature, the reaction system was diluted with10 mL of ice water and then extracted with EtOAc. The organic phases were combined, washed with saturated brine and dried. The dried organic phase was subjected to suction filtration and underwent rotary evaporation under reduced pressure to remove the solvent. The resulting solid was purified by chromatography to obtain 103 mg of compound 47, with the yield of 52%. MS (ESI) m/z 350.1.

[Step E] Ceric ammonium nitrate (CAN) (941 mg, 1.72 mmol, 10.0 equiv.) was added in batch to a solution of compound 47 (60 mg, 0.17 mmol, 1.0 equiv.) in MeCN-$H_2O$ (20 mL, 10:1, v/v) at 0°C under an argon atmosphere. The reaction mixed solution was stirred for 15 min at 0°C and then stirred for 2 h at room temperature. No remaining materials were detected by TLC. A saturated aqueous sodium bicarbonate solution (10 mL) was added, and then the reaction mixture was extracted with EtOAc. The organic phases were combined, washed with saturated brine and dried. The dried organic phase was subjected to suction filtration and underwent rotary evaporation under reduced pressure to remove the solvent. The resulting solid was purified by chromatography to obtain 34 mg of compound 1, with the yield of 85% (see synthetic route 1).

UPLC-MS (ESI) m/z 230.1, $t_R$ 4.516min. $^1$H NMR (400MHz, Chloroform-d) δ 7.69 (dd, J=7.8, 0.6Hz, 1H), 7.64 (d, J=7.8Hz, 1H), 4.05-3.91 (m, 1H), 2.68-2.45 (m, 2H), 2.26-2.01 (m, 5H) ppm.

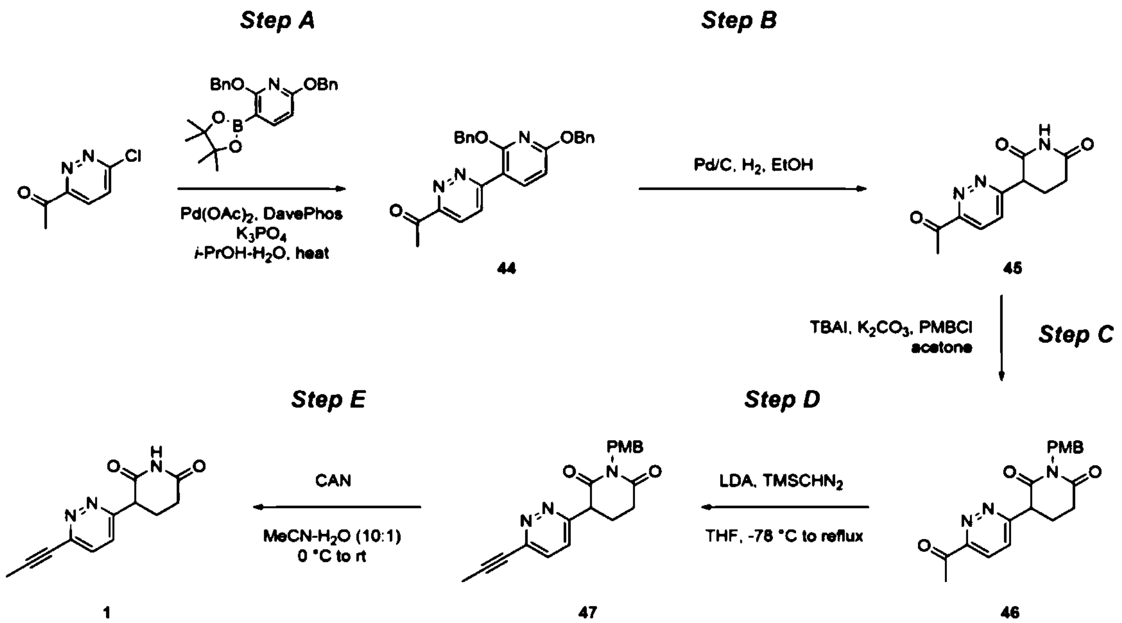


Synthetic route 1 Synthetic route of compound 1

Example 2 Preparation of compound 2-compound 43

[Step A]:

Negishi reaction (Rt. 2):

Diatomaceous earth (10 wt% of Zn) was added to a round-bottom flask, heated for 15 min with a heat gun (400°C) in high vacuum, and then cooled to room temperature, followed by adding zinc powder (200 mesh, 2.0 equiv.). Then, dry N,N-dimethylacetamide (DMA) (1 M) and 1,2-dibromoethane (0.12 equiv.) were sequentially injected. The reaction system was heated for 15 min at 70°C and then cooled to room temperature, followed by dropwise adding TMSCl (trimethylchlorosilane) (0.12 equiv.). The reaction system was stirred for 1 h at room temperature. Subsequently, a solution of tert-butyl 3-iodoazetidine-1-carboxylate (1-Boc-3-iodoazetidine) (1.5 equiv.) in dry DMA (0.4 M) was dropwise added and the temperature of the reaction system was maintained below 40°C. After the addition was completed, the reaction system was stirred for 2 h at 40°C. After the reaction system was cooled to room temperature and then subjected to standing for 30 min. The entire supernatant was transferred to a round-bottom flask, followed by sequentially adding $Pd_2(dba)_3$ (2 mol%) and $P(o\text{-}furyl)_3$ (4 mol%). After purged and backfilled with argon, a solution of 3,6-dichloropyridazine (1.0

equiv.) in dry DMA (0.4 M) was injected into the round-bottom flask. The reaction solution was stirred for 2 h at 70°C. No remaining materials were detected by TLC. After being cooled to room temperature, the reaction was quenched with a saturated ammonium chloride solution and extracted with EtOAc. The organic phases were combined, washed with brine and dried. The dried organic phase was subjected to suction filtration and underwent rotary evaporation under reduced pressure to remove the solvent. The resulting solid was purified by flash silica gel column chromatography to obtain a Negishi coupling product.

Suzuki reaction (Rt.3):

3,6-dichloropyridazine (1.0 equiv.), tert-butyl 4-(4,4,5,5-tetramethyl-1,3,2-dioxaborolan-2-yl)-5,6-dihydropyridine-1(2H)-carboxylate (1.1 equiv.) and $Pd(PPh_3)_4$ (5 mol%) were added to a round-bottom flask. The round-bottom flask was purged and backfilled with argon again. Dry dimethoxyethane (DME) (0.2 M) and a pre-deoxygenated sodium bicarbonate aqueous solution (50 v% of DME) were injected into the round-bottom flask. The reaction system was stirred for 8 h at 80°C. No remaining materials were detected by TLC. After being cooled to room temperature, the reaction system was diluted with water and extracted with EtOAc (3×). The organic phases were combined, washed with saturated brine and dried. The dried organic phase was subjected to suction filtration and underwent rotary evaporation under reduced pressure to remove the solvent. The resulting solid was purified by flash silica gel column chromatography to obtain a Suzuki coupling product.

[Step B] (Rt.2-6)

6-chloropyridazine derivative (1.0 equiv.), 2,6-bis(benzyloxy)-3-(4,4,5,5-tetramethyl-1,3,2-dioxaborolan-2-yl) pyridine (1.2 equiv.), $K_3PO_4$ (3.0 equiv.), DavePhos (30 mol%) and $Pd(OAc)_2$ (10 mol%) were added to a round-bottom flask. The round-bottom flask was purged and backfilled with argon again and tightly sealed with a rubber stopper, and then a deoxygenated isopropanol-water (0.2 M, 2:1, v/v) mixed solution was injected. Under stirring, the round-bottom flask was carefully purged and backfilled with argon. The reaction system was stirred for 9 h under argon atmosphere at 80°C. No remaining materials were detected by TLC. After being cooled to room temperature, the reaction system was diluted with water and then extracted with EtOAc. The organic phases were combined, washed with saturated brine and dried over anhydrous sodium sulfate. The dried organic phase was subjected to suction filtration and underwent rotary evaporation under reduced pressure to remove the solvent. The resulting solid was purified by flash silica gel column chromatography to obtain a Suzuki coupling product.

[Step C] (Rt. 2-6)

A dibenzyl-protected pyridinedione derivative was dissolved in MeOH-THF (0.1M, 1:1, v/v), and then 10% Pd/C (20wt%) was added. Argon replacement was conducted, followed by hydrogen replacement under stirring. The reaction system was vigorously stirred for 8-16 h at room temperature. No remaining materials were detected by TLC, and no unreacted intermediate was detected via LC-MS. The reaction solution was subjected to suction filtration via silica gel cake and underwent rotary evaporation under reduced pressure to remove the solvent. The resulting solid was purified by silica gel flash column chromatography to obtain a glutarimide derivative.

[Step D] (Rt.2, 3 and 5)

The glutarimide derivative obtained in the previous step was dissolved into DCM (1,2-dichloroethane) (0.2M), and then a HCl dioxane solution (4.0M, about 6equiv.) was slowly dropwise added at 0°C. The reaction system was stirred for 2 h at room temperature. No remaining materials were detected by TLC. An equal volume of ether was added to the reaction solution, followed by pulping for 0.5 h. The obtained slurry was subjected to suction filtration. The solid obtained by suction filtration was washed with PE. The obtained solid was dried in vacuum to remove the residual solvent to obtain a secondary amine hydrochloride final product as a white solid.

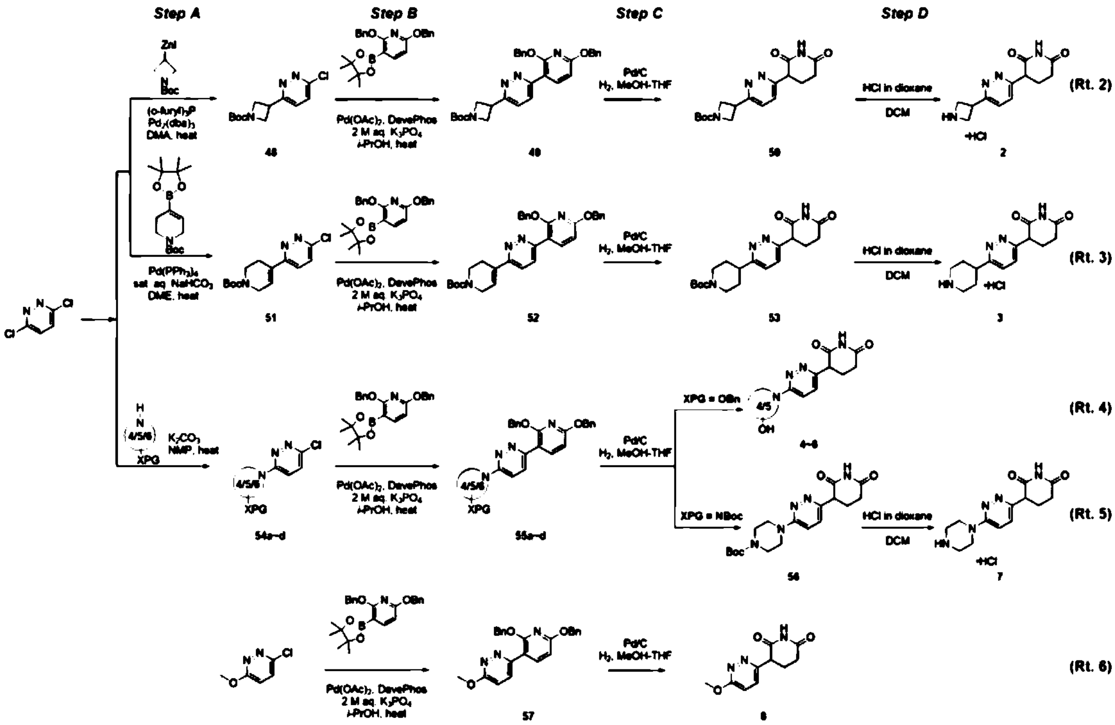


Synthetic route 2 Synthetic route of compound 2-compound 8

268 mg of Negishi coupling product 48 was obtained by using 3,6-dichloropyridazine (200 mg, 1.34 mmol) as an equivalent reference material in the Step A-Negishi reaction according to the synthetic route in the synthetic route Rt. 2, with a yield of 74%. MS (ESI) m/z 214.0 ([M+H-t-Bu]$^+$).

In Step B, 103 mg of Suzuki coupling product 49 was obtained by using compound 48 (80 mg, 0.30 mmol) as an equivalent reference material, with a yield of 66%. MS (ESI) m/z 469.2 ([M+H-t-Bu]$^+$).

In Step C, 31 mg of product 50 was obtained by using compound 49 (100 mg, 0.19 mmol) as an equivalent reference material, with a yield of 47%. MS (ESI) m/z 291.1 ([M+H-t-Bu]$^+$).

In Step D, 23 mg of target product 2 was obtained by using compound 50 (31 mg, 0.09 mmol) as an equivalent reference material, with a yield of 92%.

UPLC-MS (ESI) m/z 247.1, $t_R$ 0.972 min. $^1$H NMR (400 MHz, $D_2O$) δ 7.67 (dd, J = 8.3, 0.6 Hz, 1H), 7.57 (dd, J = 8.2, 0.5 Hz, 1H), 4.03-3.92 (m, 1H), 3.55-3.38 (m, 5H), 2.67-2.52 (m, 2H), 2.25-2.03 (m, 2H) ppm.

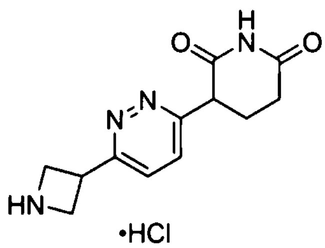

**2**

In Step A-Suzuki reaction, 301 mg of Suzuki coupling product 51 was obtained by using 3,6-dichloropyridazine (200 mg, 1.34 mmol) as an equivalent reference material according to the above operation and based on the synthetic route of Rt.3, with a yield of 76%. MS (ESI) m/z 240.1 ([M+H-t-Bu]$^+$).

In Step B, 224 mg of Suzuki coupling product 52 was obtained by using compound 51 (250 mg, 0.85 mmol) as an equivalent reference material, with a yield of 48%. MS (ESI) m/z 495.2 ([M+H-t-Bu]$^+$).

In Step C, 55 mg of product 53 was obtained by using compound 52 (200 mg, 0.36 mmol) as an equivalent reference material, with a yield of 40%. MS (ESI) m/z 319.1 ([M+H-t-Bu]$^+$).

In Step D, 43 mg of target product 3 was obtained by using compound 53 (55 mg, 0.15 mmol) as an equivalent reference material, with a yield of 95%. UPLC-MS (ESI) m/z 275.2, $t_R$ 0.941min. $^1$H NMR (400 MHz, $D_2O$) δ 7.92-7.82 (m,

2H), 4.04-3.90 (m, 4H), 3.41(dd, J =7.0, 3.4Hz, 5H), 2.78-2.70(m, 2H), 2.41-2.22 (m, 2H) ppm.

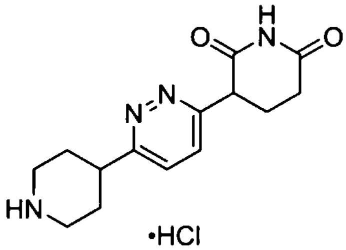

•HCl

**3**

(II) Aromatic nucleophilicity

Aromatic nucleophilic substitution reaction (Rts. 4 and 5):

Secondary amine (1.0 equiv.) and $K_2CO_3$ (3.0 equiv.) were added to a stirred solution of 3,6-dichloropyridazine (1.0 equiv.) in dry NMP (N-methylpyrrolidone) (0.2 M). After purged and backfilled with argon, the reaction system was stirred for 8 h at 110°C. No remaining materials were detected by TLC. After being cooled to room temperature, the reaction system was diluted with water and extracted with EtOAc (3×). The organic phases were combined, washed with saturated brine and dried. The dried organic phase was subjected to suction filtration and underwent rotary evaporation under reduced pressure to remove the solvent. The resulting solid was purified by chromatography to obtain an aromatic nucleophilic substitution product.

In Step A-aromatic nucleophilic substitution reaction, 303 mg of 3-(3-(benzyloxy)azetidin-1-yl)-6-chloropyridazine (54a) was obtained by using 3,6-dichloropyridazine (200 mg, 1.34 mmol) as an equivalent reference material and 3-(benzyloxy)azetidine hydrochloride (268 mg, 1.47 mmol, 1.1 equiv.) as a reactant according to the above operation and based on the synthetic route of Rt. 4, with a yield of 82%. MS(ESI) m/z 276.1.

In Step B, 289 mg of product 55a was obtained by using compound 54a (250 mg, 0.91 mmol) as an equivalent reference material, with a yield of 60%. MS (ESI) m/z 531.6.

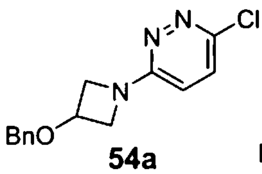

**54a**

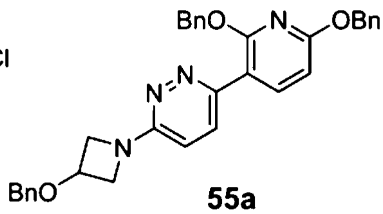

**55a**

In Step C, 43 mg of target product 4 was obtained by using compound 55a (200 mg, 0.36 mmol) as an equivalent reference material, with a yield of 44%. UPLC-MS (ESI) m/z 263.1, $t_R$ 2.689 min. $^1$H NMR (400 MHz, Methanol-$d_4$) δ 7.51-7.36 (m, 1H), 6.95 (d, J=8.6Hz, 1H), 4.20 (q, J = 4.3Hz, 1H), 4.07-4.01 (m, 1H), 3.93 (dd, J = 12.3, 4.4Hz, 2H), 3.83 (dd, J = 12.4, 4.4Hz, 2H), 2.65-2.51 (m, 2H), 2.20-2.05 (m, 2H) ppm.

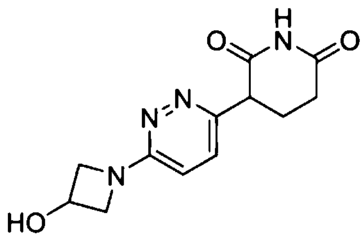

**4**

According to the above operation and based on the synthetic route of Rt. 4, 346 mg of product 54b was obtained by using 3,6-dichloropyridazine (200 mg, 1.34 mmol) as an equivalent reference material in Step A-aromatic nucleophilic substitution reaction and (R)-2-((benzyloxy)methyl)pyrrolidine (256 mg, 1.34 mmol, 1.0 equiv.) as a reactant, with a yield of 85%. MS (ESI) m/z 304.1; and 334 mg of product 54c(S)-2-((benzyloxy)methyl)pyrrolidine (256 mg, 1.34 mmol, 1.0 equiv.)

as a reactant, with a yield of 82%. MS (ESI) m/z 304.1.

In Step B, 267 mg of product 55b was obtained by using compound 54b (250 mg, 0.82 mmol) as an equivalent reference material, with a yield of 58%. MS (ESI) m/z 559.3; and 276 mg of product 55c was obtained by using compound 54c (250 mg, 0.82 mmol) as an equivalent reference material, with a yield of 60%. MS(ESI) m/z 559.3.

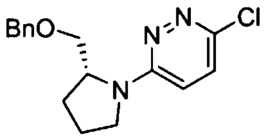

**54b**

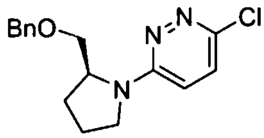

**54c**

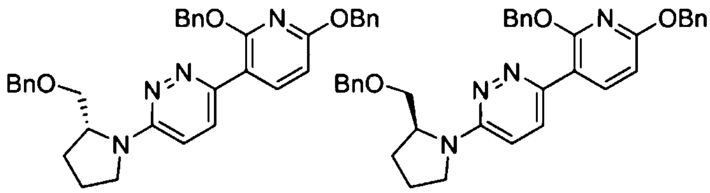

**55b** **55c**

In Step C, 54 mg of target product 5 was obtained by using compound 55b (200 mg, 0.36 mmol) as an equivalent reference material, with a yield of 52%. UPLC-MS (ESI) m/z 291.2, $t_R$ 2.323 min. $^1$H NMR (400 MHz, Methanol-$d_4$) δ 7.66-7.46 (m, 1H), 6.92 (d, J=8.1Hz, 1H), 4.07-3.67(m, 6H), 2.69-2.49 (m, 2H), 2.22-1.71 (m, 6H) ppm.

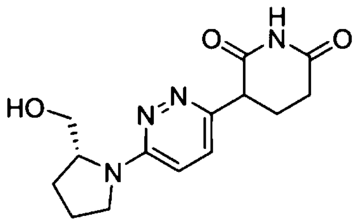

**5**

51 mg of target product 6 was obtained by using compound 55c (200 mg, 0.36 mmol) as an equivalent reference material, with a yield of 49%. UPLC-MS (ESI) m/z 291.2, $t_R$ 2.323 min. $^1$H NMR (400 MHz, Methanol-$d_4$) δ 7.66-7.36 (m, 1H), 6.92 (d, J =8.1Hz, 1H), 4.12-3.55 (m, 6H), 2.69-2.40 (m, 2H), 2.25-1.75 (m, 6H) ppm.

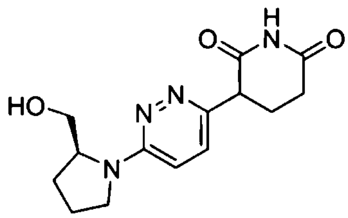

**6**

In Step A-aromatic nucleophilic substitution reaction, 344 mg of product 54d was obtained by using 3,6-dichloropyridazine (200 mg, 1.34 mmol) as an equivalent reference material and tert-butyl piperazine-1-carboxylate (250 mg, 1.34 mmol, 1.0 equiv.) as a reactant according to the above operation and based on the synthetic route of Rt. 5, with a yield of 86%. MS(ESI) m/z 199.1 ([M+H-Boc]$^+$).

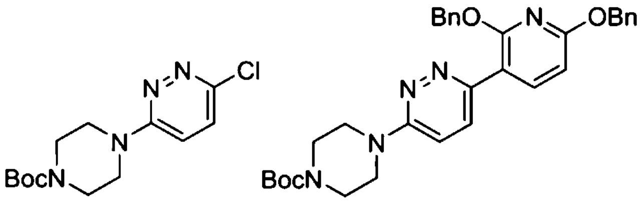

54d 55d

In Step B, 255 mg of product 55d was obtained by using compound 54d (250 mg, 0.84 mmol) as an equivalent reference material, with a yield of 55%. MS (ESI) m/z 454.2 ([M+H-Boc]$^+$).

In Step C, 70 mg of product 56 was obtained by using compound 55d (200 mg, 0.36 mmol) as an equivalent reference material, with a yield of 52%. MS(ESI) m/z 276.4 ([M+H-Boc]$^+$).

In Step D, 34 mg of target product 7 was obtained by using compound 56 (70 mg, 0.13 mmol) as an equivalent reference material, with a yield of 88%.

UPLC-MS (ESI) m/z 276.1, $t_R$ 0.982 min. $^1$H NMR (400 MHz, $D_2O$) δ 7.97(d, J = 1.3Hz, 2H), 3.52(d, J=12.9Hz, 2H), 3.41-3.30 (m, 1H), 3.21-3.09 (m, 2H), 2.81-2.60 (m, 2H), 2.52-2.15 (m, 4H), 2.09-1.93 (m, 2H) ppm.

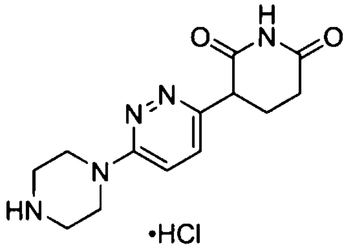

•HCl

7

In Step B, 133 mg of Suzuki coupling product 57 was obtained by using 3-chloro-6-methoxypyridazine (100 mg, 0.69 mmol) as an equivalent reference material according to the above operation and based on the synthetic route of Rt. 6, with a yield of 48%. MS(ESI) m/z 400.2.

In Step C, 18 mg of target product 8 was obtained by using compound 57 (100 mg, 0.25 mmol) as an equivalent reference material, with a yield of 32%.

UPLC-MS (ESI) m/z 222.1, $t_R$ 3.567 min. $^1$HNMR (400MHz, Methanol-d4) δ 7.58-7.52(m,1H), 7.03 (d, J = 8.1Hz, 1H), 4.07-3.99 (m, 1H), 3.90 (s, 3H), 2.65-2.50 (m, 2H), 2.23-2.05 (m, 2H) ppm.

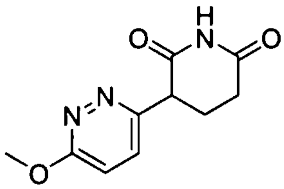

8

Compounds CMP-F, CMP-CN and CMP-$CF_3$ were synthesized from starting materials (1.0 equiv.) and MeONa (1.5 equiv.) according to the synthetic route 3 - Eq. 1 method.

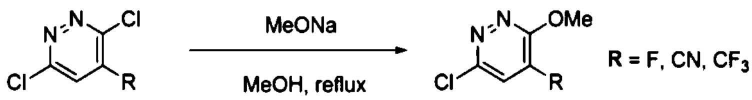


(Eq. 1)

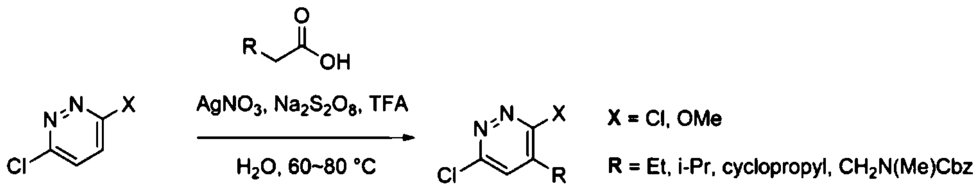


(Eq. 2)

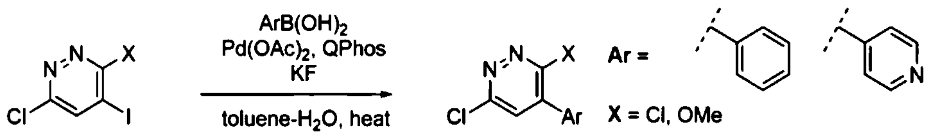


(Eq. 3)

Synthetic route 3 Synthesis reaction equation of 4-substituted pyridazine derivative

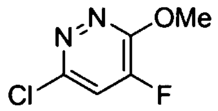

CMP-F

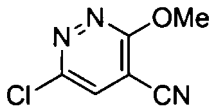

CMP-CN

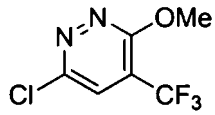

$CMP\text{-}CF_3$

Compounds CMP-Et, CMP-IP, CMP-Cp, CCP-Cp and CMP-NC were synthesized according to the synthetic route 3 - Eq. 2 method. A starting material (1.0 equiv.) was added to ultrapure water, and then carboxylic acid (1.2 equiv.), TFA (trifluoroacetic acid) (1.0 equiv.), $AgNO_3$ (0.2 equiv.) and $Na_2S_2O_8$ (1.2 equiv.) were added. After purged and backfilled with argon, the system reacted for 6-12 h under an argon atmosphere at 60°C. No remaining materials were detected by TLC. After the reaction system was cooled to room temperature, $Na_2CO_3$ was added to neutralize the reaction, and then the reaction was quenched with a saturated sodium thiosulfate solution. The reaction mixture was extracted with EtOAc. The organic phases were combined, washed with saturated brine and dried over anhydrous sodium sulfate. The dried organic phase was subjected to suction filtration and underwent rotary evaporation under reduced pressure to remove the solvent. The resulting solid was purified by silica gel flash column chromatography to obtain a desired compound.

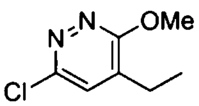

CMP-Et

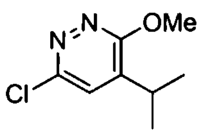

CMP-IP

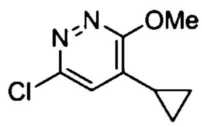

CMP-Cp

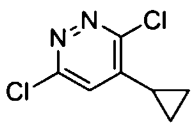

CCP-Cp

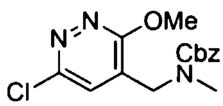

CMP-NC

Compounds CMP-Ph, CMP-Py, CCP-Ph and CCP-Py were synthesized according to the synthetic route 3 - Eq. 3 method. Starting materials (6-chloro-4-iodo-3-methoxypyridazine or 3,6-dichloro-4-iodopyridazine) (1.0 equiv.), arylboronic acid (1.05 equiv.), KF (2.5 equiv.), $Pd(OAc)_2$ (5 mol%) and QPhos (5 mol%) were added to a dry and argon-filled reaction tube with a side arm. After purged and backfilled with argon for three times, dry deoxygenated ultrapure water (4:1, v/v) was injected. After purged and backfilled with argon, the system reacted for 10-18 h under argon atmosphere at 70°C. No remaining materials were detected by TLC. After being cooled to room temperature, the reaction solution was diluted with EtOAc and subjected to suction filtration. Then, the resulting filtrate was diluted with water and then extracted with EtOAc. The organic phases were combined, washed with saturated brine and dried over anhydrous sodium sulfate. The dried organic phase was subjected to suction filtration and underwent rotary evaporation under reduced pressure to remove the solvent. The resulting solid was purified by silica gel flash column chromatography to obtain a desired compound.

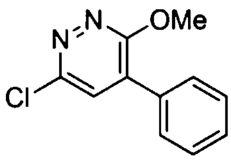

CMP-Ph

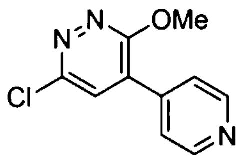

CMP-Py

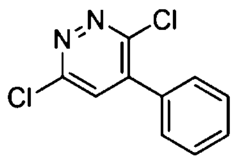

CCP-Ph

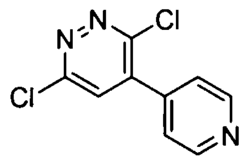

CCP-Py

[Rt. 7]

According to the synthetic route 4 - Rt. 7, Step 1, a 6-chloropyridazine derivative (1.0 equiv.), benzyl piperazine-1-carboxylate (1.2 equiv.), $Cs_2CO_3$ (1.0 equiv.), BINAP (10 mol%) and $Pd(OAc)_2$ (10 mol%) were sequentially added to an oven-dried and argon-filled reaction tube with a side arm. The reaction system was evacuated with an oil pump for 10 min and then purged and backfilled with argon again. Dry toluene (0.1M) was injected. The resulting mixed solution was stirred for 10 min at room temperature, and then heated for 8-15 h at 100°C under an argon atmosphere. No remaining materials were detected by TLC. After being cooled to room temperature, the reaction system was subjected to rotary evaporation under reduced pressure to remove the solvent. The mixture was dissolved in DCM (1,2-dichloroethane), filtered and subjected to rotary evaporation under reduced pressure to remove the solvent. The resulting solid was purified by silica gel flash column chromatography to obtain a series of intermediates CbzNN-PM-R.

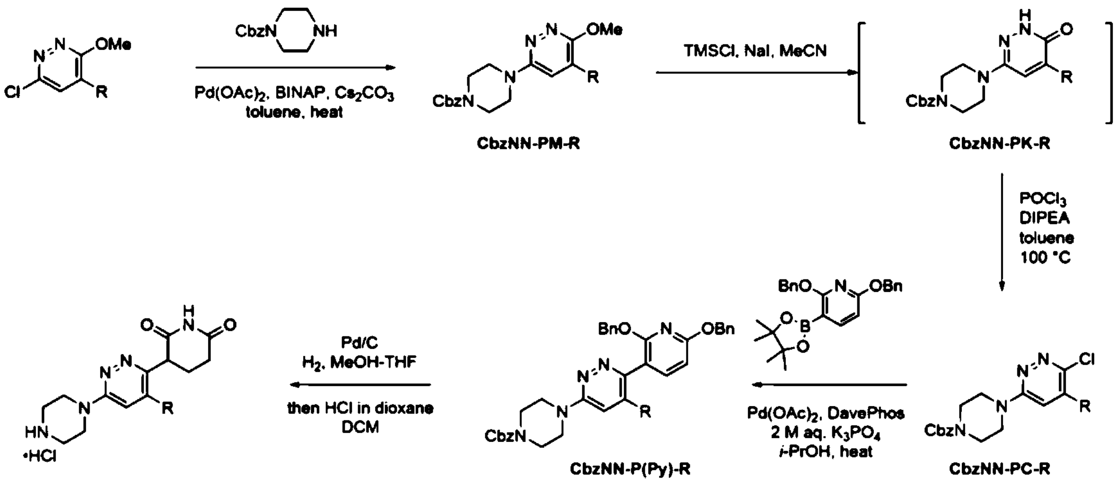


**(Rt. 7)**

Synthetic route 4 Synthetic route of compounds 9-15, 19 and 20

Table 3 A series of intermediates CbzNN-PM-R

| R | 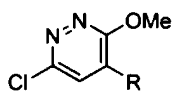 **Feed amount of material (mg)** | 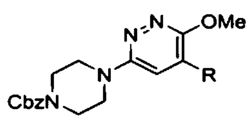 **Amount of product obtained (mg)** | Yield(%) | MS (ESI) m/z |
|---|---|---|---|---|
| Me | 500 | 864 | 80 | 343.2 |
| Et | 500 | 743 | 72 | 357.2 |
| *i*-Pr | 500 | 853 | 86 | 371.2 |
| Cyclopropyl | 500 | 778 | 78 | 369.2 |
| F | 500 | 906 | 85 | 347.2 |

(continued)

| R | 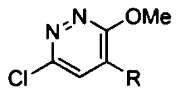 Feed amount of material (mg) | 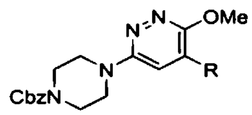 Amount of product obtained (mg) | Yield(%) | MS (ESI) m/z |
|---|---|---|---|---|
| CN | 500 | 802 | 77 | 354.2 |
| CF3 | 500 | 662 | 71 | 397.1 |
| Ph | 500 | 816 | 89 | 405.2 |
| 4-Py | 500 | 787 | 86 | 406.2 |

Step 2, NaI (3.0 equiv.) was added to a dry MeCN solution of CbzNN-PM-R (1.0 equiv.) under stirring. The suspension was placed in an ice-water bath at 0°C, and then TMSCl (trimethylchlorosilane) (3.0 equiv.) was slowly dropwise added to the reaction system. After the addition was completed, the reaction system was warmed to room temperature and then continued to be stirred for 6-14 h. No remaining materials were detected by TLC. The reaction was quenched with a sodium bicarbonate solution and a sodium thiosulfate solution and continued to be stirred for 30 min. The reaction mixture was extracted with DCM-MeOH (5-10:1, v/v). The organic phases were combined and then dried. The dried organic phase was subjected to suction filtration and underwent rotary evaporation under reduced pressure to remove the solvent to obtain a 3(2H)-pyridazinone derivative crude product. Anhydrous toluene (0.2M) was added to the round-bottom flask containing the above 3(2H)-pyridazinone derivative crude product in an ice-water bath at 0°C, then $POCl_3$ (1.5 equiv.) was slowly added, and subsequently DIPEA (diisopropylethylamine) (3.0 equiv.) was dropwise added. The reaction system continued to be stirred for 15 min at room temperature, and subsequently stirred for 4-8 h at 100°C. No remaining materials were detected by TLC. After being cooled to room temperature, the reaction system was subjected to rotary evaporation under reduced pressure to remove all volatile components. The resulting residue was dissolved with DCM (1,2-dichloroethane). The DCM phase was rapidly washed with ice water (<1 min). The obtained organic phase was washed with ice-cold saturated brine and dried over anhydrous sodium sulfate. The dried organic phase was subjected to suction filtration and underwent rotary evaporation under reduced pressure to remove the solvent. The resulting solid was purified by silica gel flash column chromatography to obtain a 3-chloropyridazine derivative.

Table 4 3-chloropyridazine derivatives

| R | 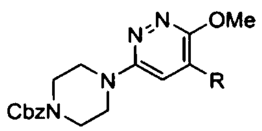 Feed amount of raw materials (mg) | 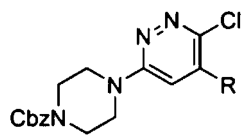 Amount of product obtained (mg) | Yield (%) | MS (ESI) m/z |
|---|---|---|---|---|
| Me | 250 | 129 | 51 | 347.1 |
| Et | 250 | 165 | 65 | 361.1 |
| *i*-Pr | 250 | 139 | 55 | 375.2 |
| Cyclopropyl | 250 | 172 | 68 | 373.1 |
| F | 250 | 167 | 66 | 351.1 |
| CN | 250 | 104 | 41 | 358.1 |
| CF3 | 250 | 177 | 70 | 401.1 |
| Ph | 250 | 167 | 66 | 409.1 |
| 4-Py | 250 | 159 | 63 | 410.1 |

Step 3, a series of intermediates CbzNN-P(Py)-R were synthesized from the 3-chloropyridazine derivative obtained in Step 2 as an equivalent reference material according to the operation of Step B in the synthetic route 2.

Table 5 A series of intermediates CbzNN-P(Py)-R

| R | 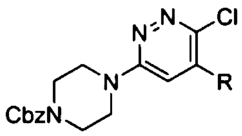 Feed amount of raw materials (mg) | 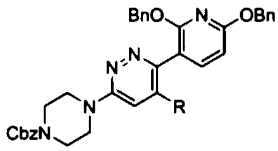 Amount of product obtained (mg) | Yield (%) | MS (ESI) m/z |
|---|---|---|---|---|
| Me | 200 | 198 | 57 | 602.3 |
| Et | 200 | 192 | 56 | 616.3 |
| *i*-Pr | 200 | 142 | 42 | 630.3 |
| Cyclopropyl | 200 | 178 | 53 | 628.3 |
| F | 200 | 180 | 52 | 606.3 |
| US | 200 | 184 | 54 | 613.3 |
| CF3 | 200 | 180 | 55 | 656.2 |
| pH | 200 | 178 | 55 | 664.3 |
| 4-Py | 200 | 130 | 40 | 665.3 |

Step 4, target products 9-15, 19 and 20 were synthesized from the CbzNN-P(Py)-R intermediates obtained in Step 3 as equivalent reference starting materials according to the operations of Steps C and D in synthetic route 2.

Table 6 Synthesis of products 9-15, 19, 20

| R | Target product number | 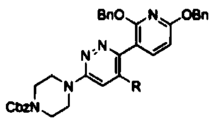 Feed amount of raw materials (mg) | 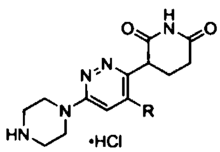 Amount of product obtained /mg (Yield) | $^1$H NMR | UPLC-MS (ESI) m/z, $t_R$ |
|---|---|---|---|---|---|
| Me | **9** | 150 | 31 (38%) | $^1$H NMR (400 MHz, Methanol-$d_4$) δ 6.94 (s, 1H), 4.12 (t, *J* = 6.0 Hz, 1H), 3.66 - 3.58 (m, 5H), 3.04 - 2.89 (m, 5H), 2.41 (d, *J* = 0.7 Hz, 3H), 2.28 -2.09 (m, 2H) ppm. | $[M + H]^+$ 290.2 0.944 min. |
| Et | **10** | 150 | 34 (41%) | $^1$H NMR (400 MHz, Methanol-$d_4$) δ 6.66 (s, 1H), 4.13 (t, *J* = 6.5 Hz, 1H), 3.66 - 3.55 (m, 5H), 3.01 - 2.68 (m, 7H), 2.30 - 1.97 (m, 2H), 1.22 (t, *J* = 7.7 Hz, 3H) ppm. | $[M + H]^+$ 304.2 0.938 min. |
| *i*-Pr | **11** | 150 | 32 (38%) | $^1$H NMR (400 MHz, Methanol-$d_4$) δ 6.67 (s, 1H), 4.18 (t, *J* = 6.3 Hz, 1H), 3.65 -3.58 (m, 5H), 3.40 - 3.18 (m, 1H), 2.96 - 2.87 (m, 5H), 2.34 - 2.07 (m, 2H), 1.39 - 1.29 (m, 6H) ppm. | $[M + H]^+$ 318.2 0.956 min. |

(continued)

| R | Target product number | 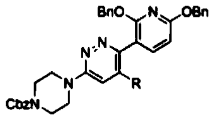 Feed amount of raw materials (mg) | 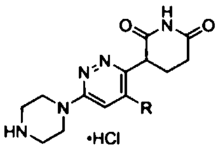 Amount of product obtained /mg (Yield) | $^1$H NMR | UPLC-MS (ESI) m/z, $t_R$ |
|---|---|---|---|---|---|
| Cyclopropyl | **12** | 150 | 44 (52%) | $^1$H NMR (400 MHz, Methanol-$d_4$) δ 6.99 (d, $J$ = 0.6 Hz, 1H), 4.19 (t, $J$ = 6.3 Hz, 1H), 3.65 - 3.59 (m, 4H), 3.11 - 2.82 (m, 5H), 2.64 - 2.52 (m, 2H), 2.32 - 2.09 (m, 2H), 1.31 - 1.10 (m, 2H), 0.97 - 0.74 (m, 2H) ppm. | $[M + H]^+$ 316.2 0.944 min. |
| F | **13** | 150 | 47 (57%) | $^1$H NMR (400 MHz, Methanol-$d_4$) δ 6.79 (d, $J$ = 7.9 Hz, 1H), 4.18 (t, $J$ = 6.0 Hz, 1H), 3.64 - 3.57 (m, 4H), 2.98 - 2.87 (m, 4H), 2.71 - 2.51 (m, 2H), 2.32 - 2.09 (m, 2H) ppm. | $[M + H]^+$ 294.1 0.911 min. |
| CN | **14** | 150 | 35 (42%) | $^1$H NMR (400 MHz, Methanol-$d_4$) δ 7.32 (s, 1H), 4.16 (t, $J$ = 5.4 Hz, 1H), 3.65 - 3.58 (m, 4H), 2.97 - 2.88 (m, 4H), 2.67 - 2.53 (m, 2H), 2.28 - 2.11 (m, 2H) ppm. | $[M + H]^+$ 301. 1 0.921 min. |
| CF3 | **15** | 150 | 45 (52%) | $^1$H NMR (400 MHz, Methanol-$d_4$) δ 7.39 (s, 1H), 3.76 (t, $J$ = 5.9 Hz, 1H), 3.64 - 3.58 (m, 4H), 2.97 - 2.87 (m, 4H), 2.71 - 2.54 (m, 2H), 2.28 - 2.09 (m, 2H) ppm. | $[M + H]^+$ 344.1 0.923 min. |
| Ph | **19** | 150 | 53 (60%) | $^1$H NMR (400 MHz, Methanol-$d_4$) δ 7.66 - 7.59 (m, 2H), 7.50 - 7.38 (m, 3H), 7.25 (s, 1H), 3.79 (t, $J$ = 6.2 Hz, 1H), 3.66 - 3.57 (m, 4H), 2.96 - 2.87 (m, 4H), 2.65 - 2.56 (m, 2H), 2.26 - 2.12 (m, 2H) ppm. | $[M + H]^+$ 352.2 0.972 min. |
| 4-Py | **20** | 150 | 39 (45%) | $^1$H NMR (400 MHz, Methanol-$d_4$) δ 8.71 - 8.61 (m, 2H), 7.63 - 7.55 (m, 2H), 7.29 (s, 1H), 3.79 (t, $J$ = 6.3 Hz, 1H), 3.64 - 3.58 (m, 4H), 2.96 - 2.88 (m, 4H), 2.61 - 2.53 (m, 2H), 2.27 - 2.10 (m, 2H) ppm. | $[M + H]^+$ 353.2 0.850 min. |

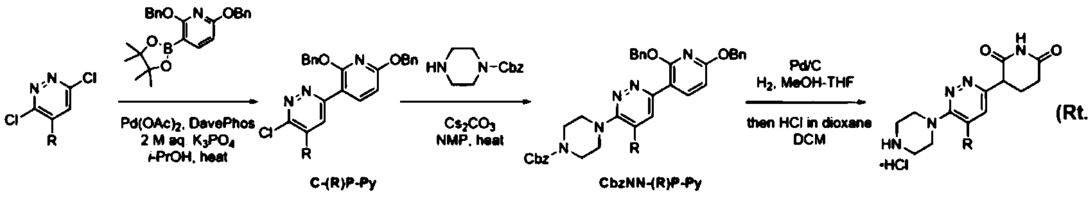

(Rt. 8)

Synthetic route 5 Synthetic route of compounds 21-24, 31 and 32

[Rt. 8]

In synthetic route 5 - Rt. 8, Step 1, a series of intermediates C-(R)P-Py were synthesized from a 3,6-dichloropyridazine derivative as an equivalent reference material according to the operation of Step B in the synthetic route 2.

Table 7 A series of intermediates C-(R)P-Py

| **R** | 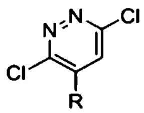 **Feed amount of raw materials (mg)** | 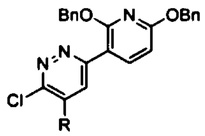 **Amount of product obtained (mg)** | **Yield (%)** | **MS (ESI) m/z** |
|---|---|---|---|---|
| Me | 500 | 449 | 35 | 418.1 |
| Et | 500 | 586 | 48 | 432.1 |
| *i*-Pr | 500 | 700 | 60 | 446.2 |
| Cyclopropyl | 500 | 681 | 58 | 444.1 |
| pH | 500 | 661 | 62 | 480.1 |
| 4-Py | 500 | 585 | 55 | 481.1 |

Step 2, benzyl piperazine-1-carboxylate (1.5 equiv.) and $Cs_2CO_3$ (3.0 equiv.) were added to a solution of C-(R)P-Py (1.0 equiv.) in dry NMP (N-methylpyrrolidone) under stirring, and then the suspension was stirred for 8-15 h at 110°C under argon atmosphere. No remaining materials were detected by TLC. After being cooled to room temperature, the reaction system was diluted with water. The reaction was quenched with a saturated sodium bicarbonate solution and a saturated sodium thiosulfate solution, and continued to be stirred for 30 min. The reaction mixture was extracted with DCM-MeOH (5-10:1, v/v) (4×). The organic phases were combined and dried. The dried organic phase was subjected to suction filtration and underwent rotary evaporation under reduced pressure to remove the solvent. The resulting solid was purified by flash silica gel column chromatography to obtain a series of CbzNN-(R)P-Py compounds.

Table 8 A series of CbzNN-(R)P-Py compounds

| **R** | 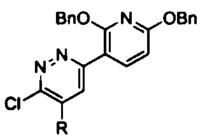 **Feed amount of raw materials (mg)** | 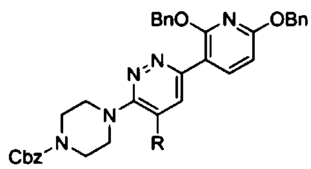 **Amount of product obtained (mg)** | **Yield (%)** | **MS (ESI) m/z** |
|---|---|---|---|---|
| Me | 500 | 562 | 78 | 602.3 |
| Et | 500 | 527 | 74 | 616.3 |
| *i*-Pr | 500 | 346 | 49 | 630.3 |
| Cyclopropyl | 500 | 403 | 57 | 628.3 |

(continued)

| R | 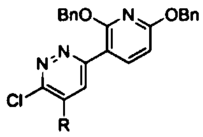 **Feed amount of raw materials (mg)** | 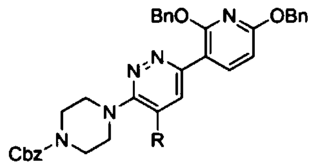 **Amount of product obtained (mg)** | **Yield (%)** | **MS (ESI) m/z** |
|---|---|---|---|---|
| pH | 500 | 463 | 67 | 664.3 |
| 4-Py | 500 | 428 | 62 | 665.3 |
| | | | | |

Step 3, target products 21-24, 31 and 32 were synthesized from a series of CbzNN-(R)P-Py compounds obtained in the second step as equivalent reference raw materials according to the operations of Steps C and D in the synthetic route 2.

Table 9 Synthesis of target products 21-24, 31 and 32

| **R** | **Target product number** | 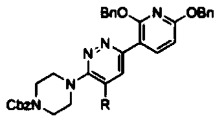 **Feed amount of raw materials (mg)** | 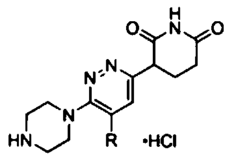 **Amount of product obtained /mg (Yield)** | **$^1H$ NMR** | **UPLC-MS (ESI) m/z, $t_R$** |
|---|---|---|---|---|---|
| Me | **21** | 150 | 37 (45%) | $^1H$ NMR (400 MHz, Methanol-$d_4$) δ 7.17 (s, 1H), 4.02 (t, $J$ = 6.1 Hz, 1H), 3.95 - 3.85 (m, 4H), 3.32 - 3.19 (m, 4H), 2.65 - 2.48 (m, 2H), 2.27 (s, 3H), 2.23 - 2.02 (m, 2H) ppm. | $[M + H]^+$ 290.2 0.932 min |
| Et | **22** | 150 | 35 (42%) | $^1H$ NMR (400 MHz, Methanol-$d_4$) δ 7.18 (d, $J$ = 0.6 Hz, 1H), 4.05 (td, $J$ = 6.1, 0.7 Hz, 1H), 4.00 (dd, $J$ = 5.9, 3.1 Hz, 2H), 3.90 (dd, $J$ = 5.7, 3.1 Hz, 2H), 3.31 - 3.23 (m, 4H), 2.78 - 2.51 (m, 4H), 2.26 - 2.03 (m, 2H), 1.23 (t, $J$ = 7.9 Hz, 3H) ppm. | $[M + H]^+$ 304.2 0.955 min. |

(continued)

| R | Target product number | 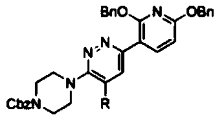 Feed amount of raw materials (mg) | 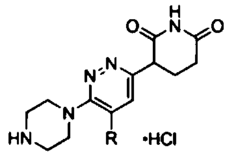 Amount of product obtained /mg (Yield) | $^1$H NMR | UPLC-MS (ESI) m/z, $t_R$ |
|---|---|---|---|---|---|
| i-Pr | **23** | 150 | 41 (49%) | $^1$H NMR (400 MHz, Methanol-$d_4$) δ 7.29 (s, 1H), 4.02 (t, *J* = 6.2 Hz, 1H), 4.00 - 3.89 (m, 4H), 3.30 - 3.22 (m, 4H), 3.18 (h, *J* = 7.1 Hz, 1H), 2.67 - 2.46 (m, 2H), 2.29 - 1.95 (m, 2H), 1.36 (d, *J* = 7.3 Hz, 3H), 1.31 (d, *J* = 7.2 Hz, 3H) ppm. | $[M + H]^+$ 318.2 0.970 min. |
| Cyclopropyl | **24** | 150 | 46 (46%) | $^1$H NMR (400 MHz, Methanol-$d_4$) δ 7.33 (s, 1H), 4.02 (t, *J* = 6.1 Hz, 1H), 4.00 - 3.89 (m, 4H), 3.33 - 3.20 (m, 4H), 3.04 - 2.88 (m, 1H), 2.66 - 2.47 (m, 2H), 2.25 - 2.01 (m, 2H), 1.20 - 1.01 (m, 2H), 0.93 - 0.74 (m, 2H) ppm. | $[M + H]^+$ 316.2 0.965 min. |
| pH | **31** | 150 | 52 (52%) | $^1$H NMR (400 MHz, Methanol-$d_4$) δ 7.68 - 7.59 (m, 3H), 7.51 - 7.45 (m, 2H), 7.43 - 7.37 (m, 1H), 4.09 (t, *J* = 6.2 Hz, 1H), 4.03 - 3.89 (m, 4H), 3.30 - 3.19 (m, 4H), 2.65 - 2.48 (m, 2H), 2.24 - 2.08 (m, 2H) ppm. | $[M + H]^+$ 352.2 0.986 min |
| 4-Py | **32** | 150 | 60 (60%) | $^1$H NMR (400 MHz, Methanol-$d_4$) δ 8.66 (d, *J* = 4.6 Hz, 2H), 7.66 (s, 1H), 7.57 (d, *J* = 4.6 Hz, 2H), 4.09 (t, *J* = 6.2 Hz, 1H), 4.02 - 3.91 (m, 4H), 3.32 - 3.18 (m, 4H), 2.68 - 2.49 (m, 2H), 2.26 - 2.08 (m, 2H) ppm. | $[M + H]^+$ 353.2 0.880 min |

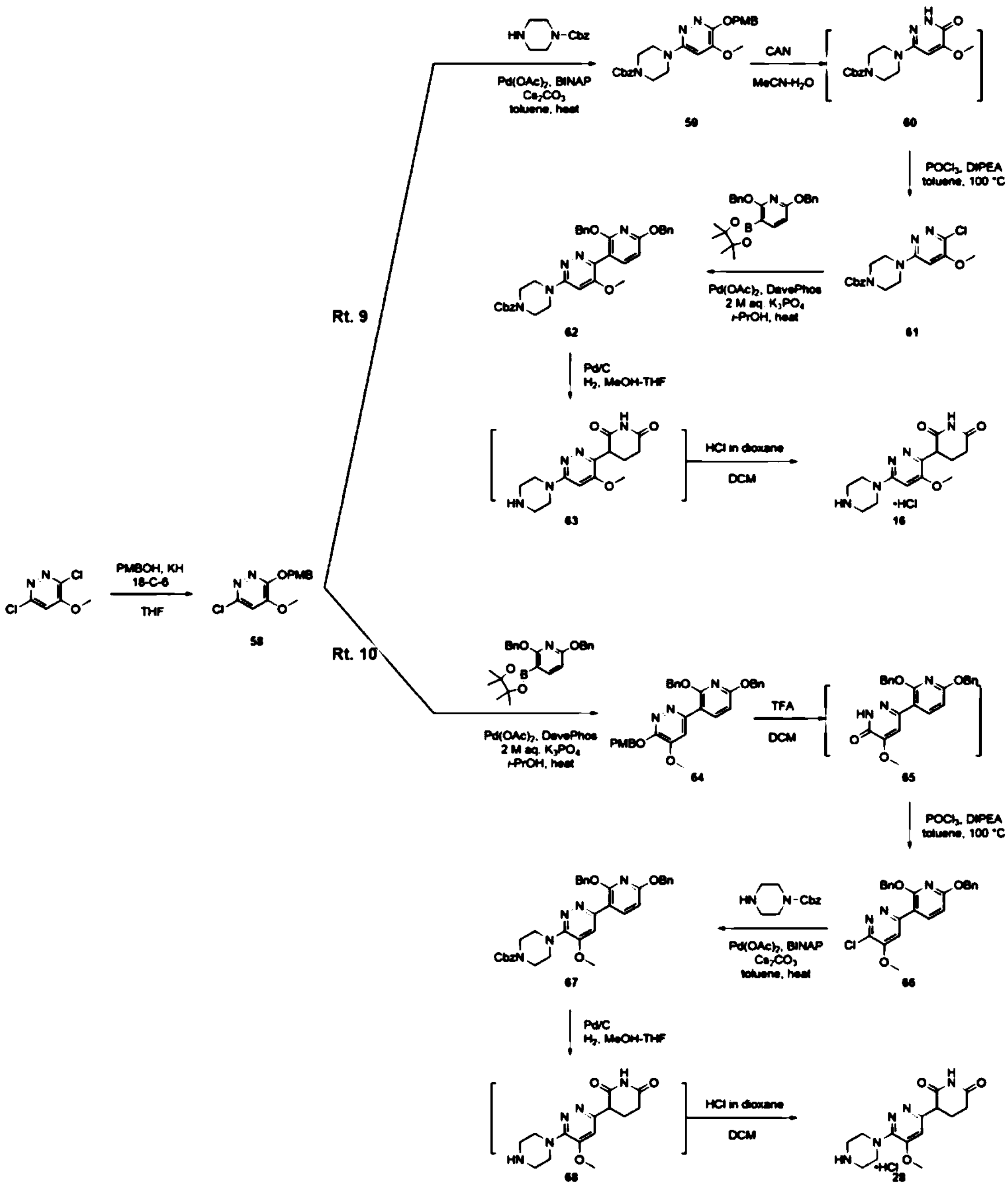


Synthetic route 6 Synthetic route of compounds 16 and 28

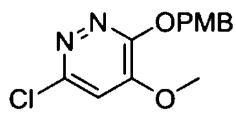

58

KH (2.24 g, 30 wt%, 16.76 mmol, 2.0 equiv.) was added to a round-bottom flask, and then anhydrous n-hexane (5 mL) was added. The above materials were stirred and subjected to standing. Then, the supernatant was discarded. The above process repeated twice. The residual solvent was removed in vacuum by using an oil pump. The reaction was purged and backfilled with argon. Dry THF (tetrahydrofuran) (20 mL) was then injected, and then the above reaction

system was placed in a 0°C ice-water bath. A solution of PMBOH (p-methoxybenzyl alcohol) (1.50 g, 10.89 mmol, 1.3 equiv.) in THF (tetrahydrofuran) (5 mL) was dropwise added under stirring, followed by adding 18-C-6 (111 mg, 0.42 mmol, 0.05 equiv.). The reaction system continued to be stirred for 2 h at room temperature. A solution of 3,6-dichloro-4-methoxypyridazine (1.50 g, 8.38 mmol, 1.0 equiv.) in dry THF (20 mL) was dropwise added to the above reaction solution at 0°C. After the addition was completed, the reaction system was stirred for 6 h at room temperature. No remaining materials were detected by TLC. The reaction system was poured onto crushed ice, and then a small amount of water (20 mL) was added. The water phase was extracted with DCM (1,2-dichloroethane) (3×80 mL). The organic phases were combined, washed with sodium chloride solution and dried. The dried organic phase was subjected to suction filtration and underwent rotary evaporation under reduced pressure to remove the solvent. The resulting solid was purified by chromatography to obtain 1.08 g of compound 58, with a yield of 46%.

MS (ESI) m/z 281.0. $^1$H NMR (400MHz, Chloroform-d) δ 7.37(d, J=8.2Hz, 2H), 7.17 - 6.77 (m, 3H), 5.32 (s, 2H), 3.98 (s, 3H), 3.78 (s,3H) ppm.

[Route 6 Rt. 9]:

Step 1, 1.07 g of compound 59 was synthesized by using compound 58 (1.00 g, 3.56 mmol) as an equivalent reference material according to the method of Step 1 in the synthesis route 4 - Rt. 7, with a yield of 65%. MS (ESI) m/z 465.2.

Step 2: compound 59 (1.00 g, 2.15 mmol, 1.0 equiv.) was dissolved in MeCN (20 mL), and then $H_2O$ (5 mL) was added. Subsequently, 1.0 equiv. of CAN (ceric ammonium nitrate) (totally 9.13 g, 17.22 mmol, 8.0 equiv.) was added to the reaction solution under stirring every 1 h. No remaining materials were detected by TLC. The reaction was quenched with a saturated sodium thiosulfate solution. The water phase was extracted with DCM-MeOH (10:1, v/v) (3×80 mL). The organic phases were combined, washed with a saturated sodium chloride solution (1×80 mL) and dried over anhydrous sodium sulfate. The dried organic phase was subjected to suction filtration and underwent rotary evaporation under reduced pressure to remove the solvent to obtain 511 mg of crude product compound 60. The obtained crude product was directly used for the next step without further purification. MS (ESI) m/z 345.1.

Step 3: 258 mg of compound 61 was synthesized by using crude product 60 (511 mg, about 1.48 mmol) as an equivalent reference material according to the method in Step 3 of the synthetic route 4-Rt.7, with a yield of 33% in two steps. MS (ESI) m/z 363.1.

Step 4: 312 mg of compound 62 was synthesized by using compound 61 (300 mg, 0.83 mmol) as an equivalent reference material according to the method of Step 4 in synthetic route 4-Rt.7, with a yield of 61%. MS(ESI) m/z 618.3.

Steps 5 and 6: 52 mg of target compound 16 was synthesized by using compound 62 (300 mg, 0.83 mmol) as an equivalent reference material according to the methods of the Steps 5 and 6 in the synthetic route 4-Rt.7, with a yield of 38%.

UPLC-MS (ESI) m/z 306.2, $t_R$ 0.911min. $^1$H NMR (400MHz, Methanol-$d_4$) δ 6.54 (s, 1H), 4.14 (t, J=6.4Hz, 1H), 3.86 (s, 3H), 3.65-3.60 (m, 4H), 2.97-2.89 (m, 4H), 2.62-2.54 (m, 2H), 2.27-2.08 (m, 2H) ppm.

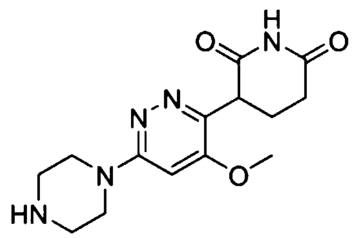

•HCl

**16**

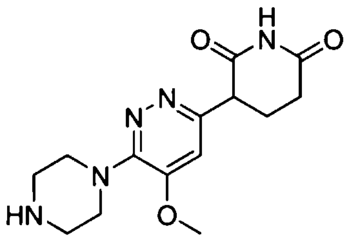

•HCl

**28**

[Synthetic route 6 Rt. 10]

Step 1: 1.03 g of compound 64 was synthesized by using compound 58 (1.00 g, 3.56 mmol) as an equivalent reference material according to the method of the Step 4 in the synthetic route 4-Rt.7, with a yield of 54%. MS (ESI) m/z 536.2.

Step 2: the compound 64 (1.00 g, 1.87 mmol, 1.0 equiv.) was dissolved in anhydrous DCM (1,2-dichloroethane) (10 mL), and TFA (trifluoroacetic acid) (10 mL) was slowly added to the above solution under stirring at 0°C. The reaction system continued to be stirred for 2-3 h at room temperature. No remaining materials were detected by TLC. The reaction system was subjected to rotary evaporation under reduced pressure to remove all volatile components to obtain 780 mg of residue as a crude product 65. The obtained crude product was directly used for the next step reaction. MS (ESI) m/z 416.2.

Step 3: 381 mg of compound 66 was synthesized by using crude product 65 (780 mg, about 1.87mmol) as an equivalent reference material according to the method in Step 3 of the synthetic route 4 - Rt. 7, with a yield of 47% in two steps. MS (ESI) m/z 434.1.

Step 4: 378 mg of compound 67 was synthesized by using compound 66 (300 mg, 0.69 mmol) as an equivalent reference material according to the method of the Step 1 in the synthetic route 4 - Rt. 7, with a yield of 74%. MS (ESI) m/z 618.3.

Steps 5 and 6: 62mg of target compound 16 was synthesized by using compound 67 (300 mg, 0.83 mmol) as an equivalent reference material according to the methods of the Steps 5 and 6 in the synthetic route 4 - Rt. 7, with a yield of 45%. UPLC-MS (ESI) m/z 306.2, $t_R$ 0.921 min. $^1$H NMR (400 MHz, Methanol-$d_4$) $\delta$ 6.99 (s, 1H), 4.04-3.86 (m, 8H), 3.29-3.20 (m, 4H), 2.65-2.49 (m, 2H), 2.22-2.11 (m, 2H) ppm.

$Boc_2O$ (5.32 g, 24.40 mmol, 4.0 equiv.) and DMAP (4-dimethylaminopyridine) (74 mg, 0.61 mmol, 0.1 equiv.) were added to a solution of 3,6-dichloropyridazin-4-amine (1.0 g, 6.10 mmol, 1.0 equiv.) in anhydrous THF (tetrahydrofuran) (30 mL). The reaction mixture was stirred for 18 h under reflux heating. No remaining materials were detected by TLC. After being cooled to room temperature, the reaction system was subjected to rotary evaporation under reduced pressure to remove the solvent. The resulting solid was purified by silica gel flash column chromatography to obtain 1.91 g of compound CCP-NDB, with a yield of 86%. MS (ESI) m/z 364.2.

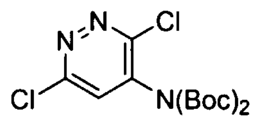

**CCP-NDB**

1.40 g of compound CbzNN-CP-NDB was synthesized by using compound CCP-NDB (1.50 g, 4.12 mmol) as an equivalent reference material according to the Step 1 of Rt. 11 in the synthetic route 7 and based on the method of the Step 1 in the synthetic route 4 - Rt. 7, with a yield of 62%. MS (ESI) m/z 548.2.

Step 2: compound CbzNN-CP-NDB (1.2 g, 2.19 mmol, 1.0 equiv.) was dissolved in dry DCM (1,2-dichloroethane) (10 mL), and then a HCl dioxane solution (5 mL) was added to the above solution under stirring at 0°C. The reaction system continued to be stirred for 2-4 h at room temperature. No remaining materials were detected by TLC. The reaction system was subjected to rotary evaporation under reduced pressure to remove all volatile components. The resulting residue was dispersed in water-EtOAc, and sodium bicarbonate was added to adjust the pH to 8. The water phase was extracted with EtOAc (3×40 mL). The organic phases were combined, washed with a saturated sodium chloride solution and dried. The dried organic phase was subjected to suction filtration and underwent rotary evaporation under reduced pressure to remove the solvent. The resulting solid was purified by chromatography to obtain 678 mg of compound CbzNN-CP-N, with a yield of 89%. MS (ESI) m/z 348.1.

Step 3: 582 mg of compound CbzNN-P(Py)-N was synthesized by using compound CbzNN-CP-N (600 mg, 1.72

mmol) as an equivalent reference material according to the method in the Step 4 of the synthetic route 4-Rt.7, with a yield of 56%. MS (ESI) m/z 536.2.

Step 4: 81 mg of target product 17 was synthesized by using compound CbzNN-P(Py)-N (500 mg, 0.83 mmol) obtained in Step 3 as equivalent reference materials according to the operations of the Steps C and D in the synthetic route 2, with a yield of 30%.

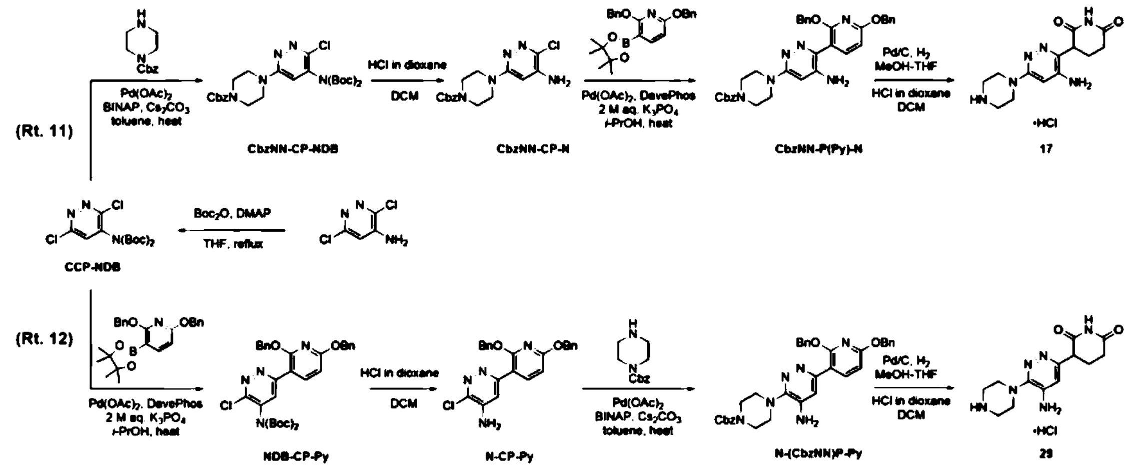


Synthetic route 7 Synthetic route of compounds 17 and 29

17: UPLC-MS(ESI) m/z 291.2, $t_R$ 0.758 min. $^1$H NMR (400 MHz, Methanol-$d_4$) $\delta$ 6.44 (s, 1H), 4.13 (t, J=6.6 Hz, 1H), 3.65-3.57 (m, 4H), 2.92 (m, 4H), 2.62-2.53 (m, 2H), 2.22-2.07 (m, 2H) ppm.

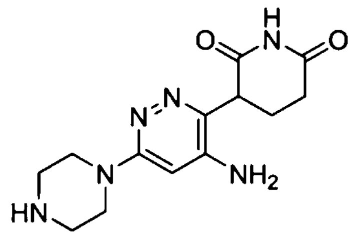

•HCl

**17**

1.86 g of compound NDB-CP-Py was synthesized by using compound CCP-NDB (1.5 g, 4.12 mmol) as an equivalent reference material according to the method of the Step 1 of Rt. 12 in the synthetic route 7 and based on the method of Step 4 in the synthetic route 4 - Rt. 7, with a yield of 73%. MS (ESI) m/z 619.2.

Step 2: 913 mg of compound N-CP-Py was synthesized by using compound NDB-CP-Py (1.5 g, 2.42 mmol) as an equivalent reference material according to the Step 2 of Rt. 11 in the synthetic route 7, with a yield of 90%. MS (ESI) m/z 419.1.

Step 3: 610 mg of compound N-(CbzNN)P-Py was synthesized by using compound N-CP-Py (800 mg, 1.91 mmol) as an equivalent reference material according to the method of the Step 1 in the synthetic route 4 - Rt. 7, with a yield of 53%. MS (ESI) m/z 603.2.

Step 4: 75 mg of target product 29 was obtained by using the compound N-(CbzNN)P-Py (600 mg, 1.00 mmol) obtained in Step 3 as an equivalent reference material according to the operations of the Steps C and D in the synthetic route 2, with a yield of 23%. UPLC-MS (ESI) m/z 291.2, $t_R$ 0.788 min. $^1$H NMR (400MHz, Methanol-$d_4$) $\delta$ 6.84 (s, 1H), 4.03-3.87 (m, 5H), 3.30-3.15 (m, 4H), 2.70-2.46 (m, 2H), 2.24-2.04 (m, 2H) ppm.

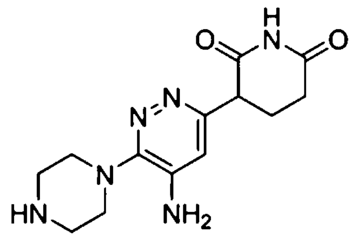

•HCl
**29**

138 mg of compound BocNN-GP-NH was obtained in five steps by using compound CMP-NC (2.00 g, 6.22 mmol) as a starting material according to the synthetic route of Rt. 13 in synthetic route 8 and based on Step 1, Step 2, Step 3 and Step 4 of the synthetic route 4 - Rt. 7 as well as the procedure of the Step C in the synthetic route 2. MS (ESI) m/z 419.2.

Step 6: the compound BocNN-GP-NH (100 mg, 0.24 mmol, 1.0 equiv.) was dissolved in DCE (5 mL), and then a 37 wt% aqueous solution of HCHO (28 μL, 0.36 mmol, 1.5 equiv.) was added. The above mixture was stirred for 30 min at room temperature. Subsequently, the system was cooled to 0°C, and then $NaBH(OAc)_3$ (253 mg, 1.20 mmol, 5.0 equiv.) was added to the reaction system. The reaction mixture was warmed to room temperature and continued to be stirred for 12 h. No remaining materials were detected by TLC. The reaction was quenched with a saturated sodium bicarbonate solution. The water phase was extracted with DCM-MeOH (5:1, v/v) (3×5 mL). The organic phases were combined and dried. The dried organic phase was subjected to suction filtration and underwent rotary evaporation under reduced pressure to remove the solvent to obtain a BocNN-GP-NM crude product. The obtained crude product was directly used for the next step without further purification. MS (ESI) m/z 433.2.

Step 7, 37 mg of target product 18 was obtained by using the crude product obtained in Step 6 as an equivalent reference material according to the method of the Step D in the synthetic route 2, with a yield of 38%.

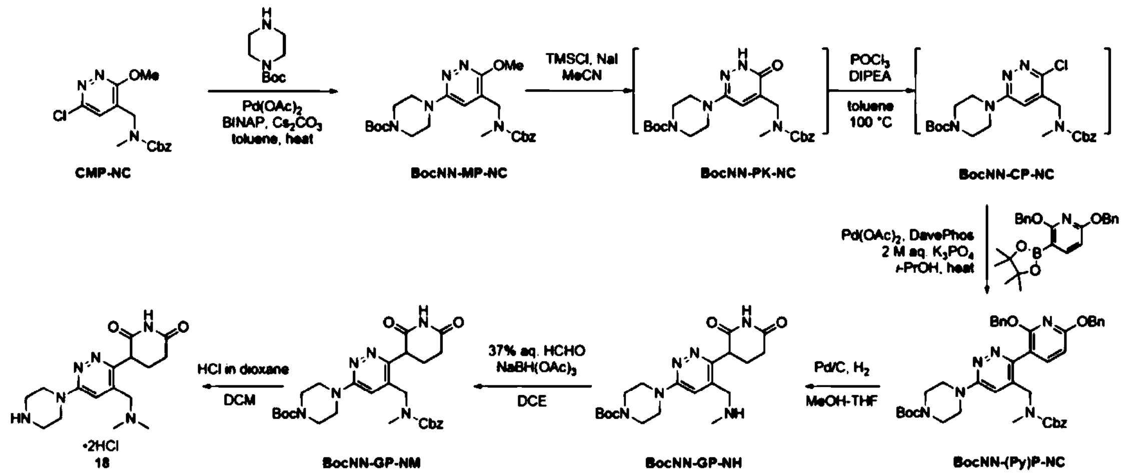


**(Rt. 13)**

Synthetic route 8 Synthetic route of compound 18

18: UPLC-MS(ESI) m/z $[M+2H]^{2+}$ 167.1, $t_R$ 0.781 min. $^1H$ NMR (400 MHz, Methanol-$d_4$) δ 6.74 (s, 1H), 4.12 (t, J=6.4 Hz, 1H), 3.84 (s, 2H), 3.64-3.58 (m, 4H), 2.96-2.88 (m, 4H), 2.62-2.57 (m, 2H), 2.41 (s, 6H), 2.26-2.09 (m, 2H) ppm.

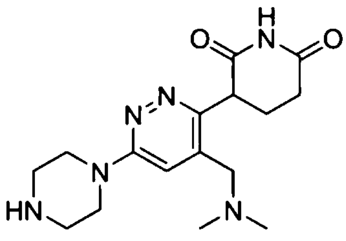

•2HCl

**18**

22 mg of compound 30 was finally synthesized by using compound CMP-NC (2.00 g, 6.22 mmol) as a starting material according to the method of the Step 1 of Rt. 14 in the synthetic route 9 and based on the method of the Step 4 in the synthetic route 4 - Rt. 7, the methods of the Steps 2 and 3 of Rt. 14 in the synthetic route 9 and the methods of the Steps 2 and 3 in the synthetic route 4 - Rt. 7, the method of the Step 4 in Rt. 14 in the synthetic route 9, the method of the Step 1 in the synthetic route 4 - Rt. 7, the methods of the Steps 5-7 of Rt.14 in the synthetic route 9 and the methods of the Steps 5-7 in the synthetic route 8 - Rt. 13.

30: UPLC-MS (ESI) m/z $[M+2H]^{2+}$ 167.1, $t_R$ 0.741 min. $^1H$ NMR (400 MHz, Methanol-$d_4$) $\delta$ 7.68-7.59 (m, 3H), 7.51-7.45 (m, 2H), 7.43-7.37 (m, 1H), 4.09 (t, J=6.2 Hz, 1H), 4.03-3.89 (m, 4H), 3.30-3.19 (m, 4H), 2.65-2.48 (m, 2H), 2.24-2.08 (m, 2H) ppm.

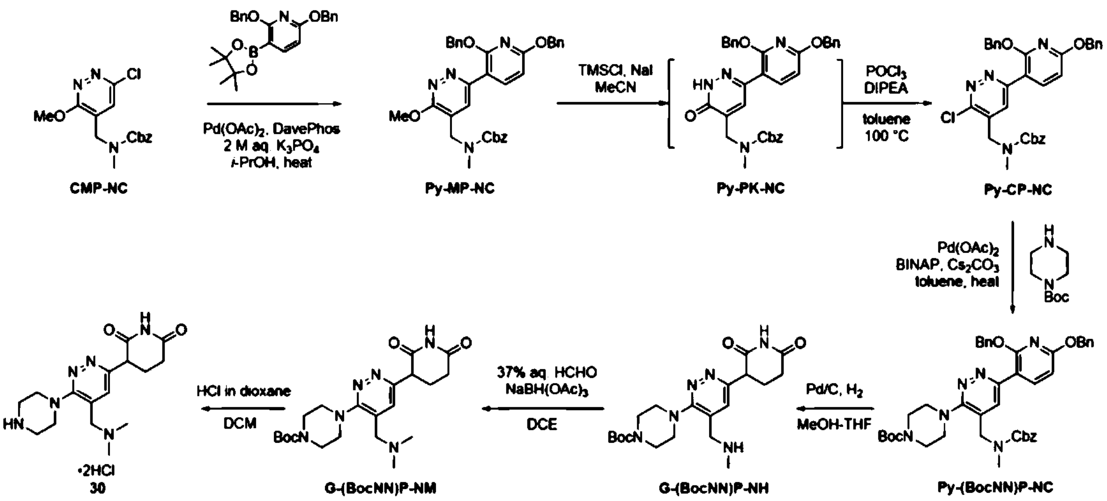


**(Rt. 14)**

Synthetic route 9 Synthetic route of compound 30

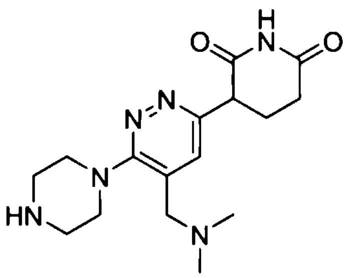

•2HCl

**30**

Compounds 25, 26, and 27 were synthesized by respectively using 3,6-dichloro-4-fluoropyridazine, 3,6-dichloropyridazine-4-carbonitrile, and 3,6-dichloro-4-(trifluoromethyl)pyridazine as an equivalent starting material according to the synthetic route method of the synthetic route 2 - Rt. 5.

25: UPLC-MS (ESI) m/z 294.1, $t_R$ 0.915 min. $^1H$ NMR (400 MHz, Methanol-$d_4$) δ 7.11 (d, J = 7.9 Hz, 1H), 4.07 (t, J = 6.1 Hz, 1H), 3.98-3.88 (m, 4H), 3.29-3.18 (m, 4H), 2.63-2.49 (m, 2H), 2.23-2.09 (m, 2H) ppm.

26: UPLC-MS(ESI) m/z 301.1, $t_R$ 0.932 min. $^1H$ NMR (400 MHz, Methanol-$d_4$) δ 7.67 (s, 1H), 4.06 (t, J=6.2 Hz, 0H), 4.01-3.90 (m, 4H), 3.28-3.19 (m, 4H), 2.65-2.48 (m, 2H), 2.24-2.07 (m, 2H) ppm.

27: UPLC-MS(ESI) m/z 344.1, $t_R$ 0.942min. $^1H$ NMR (400 MHz, Methanol-$d_4$) δ 7.62 (s, 1H), 4.05 (t, J=6.2 Hz, 1H), 4.00-3.89 (m, 4H), 3.32-3.20 (m, 4H), 2.67-2.48 (m, 2H), 2.24-2.04 (m, 2H) ppm.

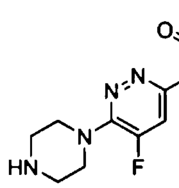
•HCl
**25**

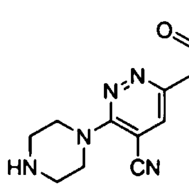
•HCl
**26**

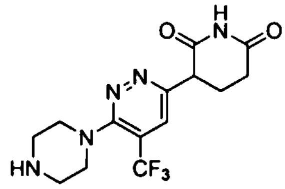
•HCl
**27**

Compounds 33, 34, 35, 36 and 37 were synthesized by respectively using 3,6-dichloro-4,5-dimethylpyridazine, 1,4-dichloro-6,7-dihydro-5H-cyclopenta[d]pyridazine, 1,4-dichloro-5,6,7,8-tetrahydrophthalazine, 1,4-dichlorophthalazine, and 5,8-dichloropyrazino[2,3-d]pyridazine as an equivalent starting material according to the synthetic route method of the Synthetic Route 2 - R t. 5.

33: UPLC-MS(ESI) m/z 304.2, $t_R$ 1.103 min. $^1H$ NMR (400 MHz, Methanol-$d_4$) δ 4.05 (t, J=6.0 Hz, 1H), 3.93-3.79 (m, 4H), 3.32-3.18 (m, 4H), 2.69-2.52 (m, 2H), 2.43 (s, 3H), 2.24-2.10 (m, 5H) ppm.

34: UPLC-MS(ESI) m/z 316.2, $t_R$ 1.116 min. $^1H$ NMR (400 MHz, Methanol-$d_4$) δ 4.06 (t, J=6.4 Hz, 1H), 3.95-3.84 (m, 4H), 3.33-3.22 (m, 4H), 2.99-2.82 (m, 4H), 2.67-2.52 (m, 2H), 2.38-2.10 (m, 4H) ppm.

35: UPLC-MS (ESI) m/z 330.2, $t_R$ 1.120 min. $^1H$ NMR (400 MHz, Methanol-$d_4$) δ 4.06 (t, J = 6.4 Hz, 1H), 3.96-3.84 (m, 4H), 3.34-3.21 (m, 4H), 3.14-2.87 (m, 4H), 2.67-2.51 (m, 2H), 2.26-2.09 (m, 2H), 1.93-1.78 (m, 4H) ppm.

36: UPLC-MS (ESI) m/z 326.1, $t_R$ 1.019 min. $^1H$ NMR (400 MHz, Methanol-$d_4$) δ 8.31 (dd, J = 8.8, 1.3 Hz, 1H), 8.10 (dd, J = 9.1, 1.4 Hz, 1H), 7.86 (ddd, J = 9.1, 7.8, 1.3 Hz, 1H), 7.72 (ddd, J = 9.0, 7.9, 1.3 Hz, 1H), 4.05-3.95 (m, 4H), 3.79 (t, J = 6.2 Hz, 1H), 3.28-3.21 (m, 4H), 2.70-2.49 (m, 2H), 2.26-2.11 (m, 2H) ppm.

37: UPLC-MS (ESI) m/z 328.1, $t_R$ 1.120 min. $^1H$ NMR (400 MHz, Methanol-$d_4$) δ 8.73 (d, J=3.5 Hz, 1H), 8.72 (d, J=3.5 Hz, 1H), 4.03-3.91 (m, 4H), 3.84 (t, J=6.6 Hz, 1H), 3.29-3.17 (m, 4H), 2.69-2.50 (m, 2H), 2.38-2.17 (m, 2H) ppm.

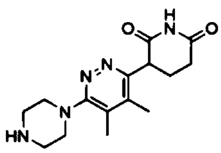
•HCl
**33**

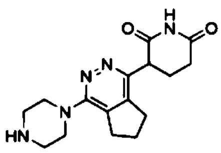
•HCl
**34**

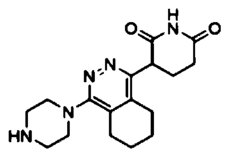
•HCl
**35**

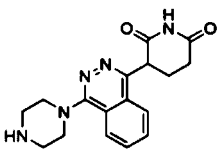
•HCl
**36**

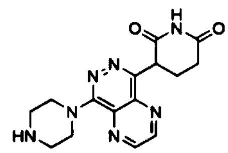
•HCl
**37**

According to the synthetic route of Rt. 15 in synthetic route 10,

Step 1: compound 56 (200 mg, 0.53 mmol, 1.0 equiv.) was dissolved in dry DCM (1,2-dichloroethane) (10 mL), and then $Et_3N$ (0.22 mL, 1.59 mmol, 3.0 equiv.), $Boc_2O$ (349 mg, 1.59 mmol, 3.0 equiv.) and DMAP (4-dimethylaminopyridine) (6 mg, 0.05 mmol, 0.1 equiv.) were then sequentially added. The reaction was stirred for 5 h at room temperature. No remaining materials were detected by TLC. The reaction was quenched with a saturated sodium bicarbonate solution. The water phase was extracted with DCM-MeOH (10:1, v/v) (3×10 mL). The organic phases were combined and dried. The dried organic phase was subjected to suction filtration and underwent rotary evaporation under reduced pressure to remove the solvent. The resulting solid was purified by flash silica gel column chromatography to obtain 192 mg of compound 69, with a yield of 76%. MS (ESI) m/z 476.2.

Step 2: the compound 69 (85 mg, 0.18 mmol, 1.0 equiv.) was dissolved in dry THF (tetrahydrofuran) (4 mL). The solution was cooled to -78°C. LiHMDS (0.27 mL, 1.0 M in THF, 0.27 mmol, 1.5 equiv.) was dropwise added to the above solution. After the addition was completed, the reaction system continued to be stirred for 1 h at the same temperature.(A) At the same temperature, a solution of NFSI (91 mg, 0.29 mmol, 1.6 equiv.) in dry THF (2.0 mL) was dropwise added to the reaction solution, and then the reaction was continued to be stirred for 2-5 h at the same temperature. No residual raw material was detected by LC-MS. The reaction was quenched with a saturated ammonium chloride solution. After the reaction was warmed to room temperature, the water phase was extracted with DCM-MeOH (10:1, v/v) (3×10 mL), and the organic phases were combined and dried with anhydrous sodium sulfate. The dried organic phase was subjected to

suction filtration and underwent rotary evaporation under reduced pressure to remove the solvent. The resulting solid was purified by chromatography to obtain 61 mg of compound 70, with a yield of 69%. MS (ESI) m/z 494.2.(B) Alternatively, at the same temperature, AcOD (17 μL, 0.29 mmol, 1.6 equiv.) was dropwise added to the reaction solution, and then the reaction continued to be stirred for 2-5 h at the same temperature. No remaining materials were detected by LC-MS. After the reaction was warmed to room temperature, the reaction system was diluted with water. The water phase was extracted with DCM-MeOH (10:1, v/v) (3×10 mL). The organic phases were combined and dried over anhydrous sodium sulfate. The dried organic phase was subjected to suction filtration and underwent rotary evaporation under reduced pressure to remove the solvent. The resulting solid was purified by chromatography to obtain 75 mg of compound 71, with a yield of 88%. MS (ESI) m/z 477.2.

Step 3: 24 mg of target product 38 and 24 mg of target product 39 were obtained by using the compound 70 (50 mg, 0.10 mmol) or 71 (60 mg, 0.13 mmol) obtained in Step 2 as an equivalent reference material according to the method of the Step D in the synthetic route 2, with the yields of 72% and 59%, respectively.

38: UPLC-MS (ESI) m/z 294.1, $t_R$ 0.965 min. $^1$H NMR (400 MHz, Methanol-$d_4$) δ 7.45 (d, J = 7.2 Hz, 1H), 6.99 (d, J = 7.3 Hz, 1H), 4.01-3.82 (m, 5H), 3.34-3.17 (m, 4H), 2.74-2.49 (m, 2H), 2.44-2.08 (m, 2H) ppm.

39: UPLC-MS (ESI) m/z 277.2, $t_R$ 0.977 min. $^1$H NMR (400 MHz, Methanol-$d_4$) δ 7.28 (d, J=8.6 Hz, 1H), 6.93 (d, J=8.6 Hz, 1H), 3.96-3.83 (m, 4H), 3.41-3.15 (m, 4H), 2.78-2.47 (m, 2H), 2.28-2.06 (m, 2H) ppm.

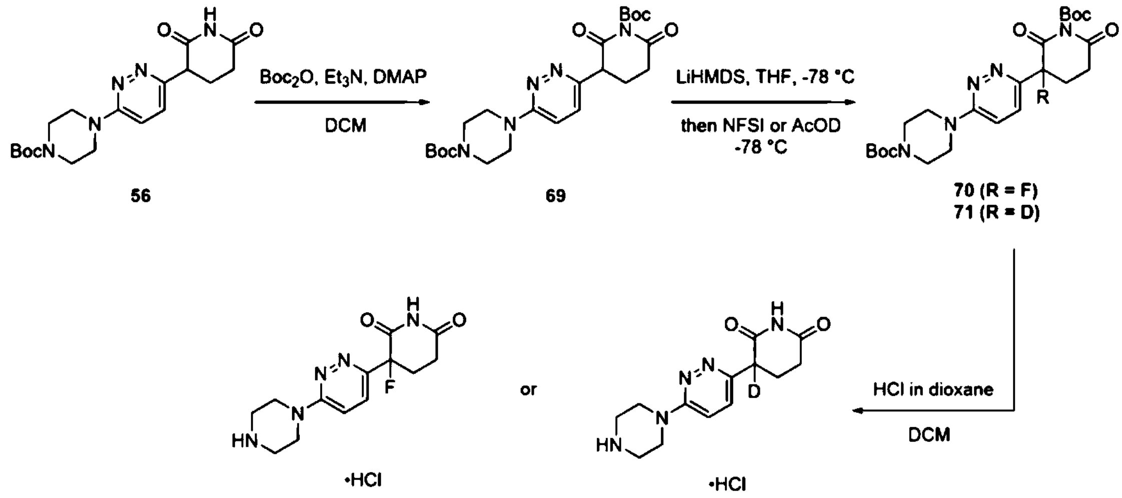


56

69

70 (R = F)
71 (R = D)

•HCl
38

or

•HCl
39

**(Rt. 15)**

Synthetic route 10 Synthetic route of compounds 38 and 39

According to the synthetic route 11 in Rt.16, Step 1: $Boc_2O$ (2.92 g, 13.38 mmol, 3.0 equiv.) and DMAP (4-dimethylaminopyridine) (163 mg, 1.34 mmol, 0.3 equiv.) were sequentially added to a solution of uracil (500 mg, 4.46 mmol, 1.0 equiv.) in DME (20 mL) under stirring, and then the reaction system was heated overnight under reflux. No remaining materials were detected by TLC. After being cooled to room temperature, the reaction system was subjected to rotary evaporation under reduced pressure to remove all volatile components to obtain crude product N1Boc-N3Boc-PM. The obtained crude product was directly used for the next step without further purification. MS (ESI) m/z 257.1 ([M+H-t-Bu]$^+$).

Step 2: the crude product obtained in the previous step was dissolved in DCM-MeOH (9:1, v/v, 10 mL), and then silica gel powders (300 mg, 300-400 mesh, 60wt%) were added. The reaction mixture was stirred at 60°C. The reaction progress was monitored by TLC. The reaction system was subjected to suction filtration and underwent rotary evaporation under reduced pressure to remove the solvent. The resulting solid was purified by chromatography to obtain 786 mg of the compound N1H-N3Boc-PM, with a yield of 83%. MS (ESI) m/z 213.1.

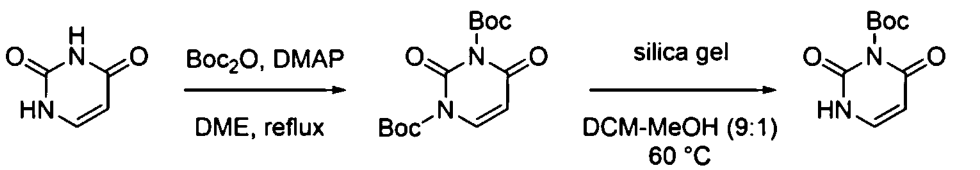


N1Boc-N3Boc-PM

N1H-N3Boc-PM

**(Rt. 16)**

Synthetic route 11 Synthetic route of compound N1H-N3Boc-PM

According to the synthetic route in synthetic route 12, Step 1: N1H-N3Boc-PM (100 mg, 0.47 mmol, 1.0 equiv.), chiral prolinamide cat-1 (31 mg, 0.09 mmol, 0.3 equiv.) and 3-chloro-6-iodopyridazine (170 mg, 0.71 mmol, 1.5 equiv.) were added to an argon-filled reaction tube, and then the above reaction was purged and backfilled with argon. Then, deoxygenated ultrapure water (2.5 mL), CuI (9 mg, 0.05 mmol, 0.1 equiv.) and $K_2CO_3$ (130 mg, 0.94 mmol, 2.0 equiv.) were added, and then the above reaction was purged and backfilled with argon. The reaction system was stirred for 24 h under an argon atmosphere at 100°C. No remaining materials were detected by TLC. After the reaction system was cooled to room temperature, the water phase was extracted with EtOAc (3×5 mL). The organic phases were combined and dried over anhydrous sodium sulfate. The dried organic phase was subjected to suction filtration and underwent rotary evaporation under reduced pressure to remove the solvent. The resulting solid was purified by chromatography to obtain 89mg of compound 72, with a yield of 58%. MS (ESI) m/z 325.1.

Step 2: 64 mg of compound 73 was obtained by using compound 72 (70 mg, 0.22 mmol) as an equivalent reference material according to the Step 1 of the synthetic route 4 - Rt. 7, with a yield of 63%. MS (ESI) m/z 419.2 ([M+H-t-Bu]$^+$).

Step 3 and Step 4: 24 mg of target product 40 was obtained by using the compound 73 obtained in Step 2 (50 mg, 1.00 mmol) as an equivalent reference material according to the operations of the Steps C and D in the synthetic route 2, with a yield of 72%. UPLC-MS(ESI)m/z 277.1, $t_R$ 0.768 min. $^1$H NMR (400MHz, Methanol-$d_4$) δ 7.52 (d, J=7.9Hz, 1H), 7.09 (d, J=7.9Hz, 1H), 4.12-3.82 (m, 6H), 3.47-3.14 (m, 4H), 2.74 (dd, J=7.0, 4.2Hz, 2H) ppm.

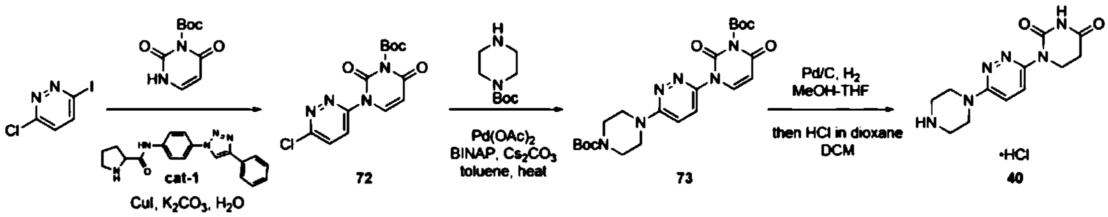


Synthetic route 12 Synthetic route of compound 40

According to the synthetic route in synthetic route 13, Step 1, 3,4,6-trichloropyridazine (1.50 g, 8.18 mmol, 1.0 equiv.) was dissolved in dry DMF (40 mL), and then $K_2CO_3$ (2.26 g, 16.36 mmol, 2.0 equiv.) was added to the solution at 0°C. Subsequently, a solution of tert-butylpiperazine-1-carboxylate (1.98 g, 10.63 mmol, 1.3 equiv.) in dry DMF (20 mL) was slowly dropwise added to the above reaction mixture at the same temperature. After being automatically warmed to room temperature, the reaction system continued to be stirred for 3 h. No remaining materials were detected by TLC. The reaction system was diluted with water (100 mL). The water phase was extracted with EtOAc (4×100 mL). The organic phases were combined and dried over anhydrous sodium sulfate. The dried organic phase was subjected to suction filtration and underwent rotary evaporation under reduced pressure to remove the solvent. The resulting solid was purified by chromatography to obtain 2.45 g of compound 74, with a yield of 90%. MS (ESI) m/z 333.1.

Step 2, 1.08 g of compound 75 was synthesized by using compound 74 (1.00 g, 3.00 mmol) as an equivalent reference material according to the Step B in the synthetic route 2, with a yield of 61%. MS (ESI) m/z 588.2.

Step 3, the compound 75 (450 mg, 0.76 mmol, 1.0 equiv.) was dissolved in anhydrous MeOH (8 mL). (A) MeONa (62 mg, 1.15 mmol, 1.5 equiv.) was added to the solution at 0°C, then the reaction system was stirred for 10 min at room temperature and then stirred overnight under reflux. No remaining materials were detected by TLC. The reaction system was poured into ice water. The water phase was extracted with EtOAc (4×20 mL). The organic phases were combined and dried over anhydrous sodium sulfate. The dried organic phase was subjected to suction filtration and underwent rotary evaporation under reduced pressure to remove the solvent. The resulting solid was purified by silica gel flash column chromatography to obtain 344 mg of compound 76, with a yield of 77%. MS(ESI) m/z 584.3; (B) a methanol solution of dimethylamine (0.58 mL, 2.0 M in MeOH, 1.15 mmol, 1.5 equiv.) was added to the above solution at 0°C by using a sealed reaction tube. The reaction system was stirred for 10 min at room temperature, then the tube was sealed and the system was heated for 4 h under stirring at 100°C. The reaction system was cooled to room temperature and depressurized, and no remaining materials were detected by TLC. Then, the reaction solution was diluted with water. The water phase was extracted with EtOAc (4 × 20 mL). The organic phases were combined and dried over anhydrous sodium sulfate, and then dried organic phase was subjected to suction filtration and rotary evaporation under reduced pressure to remove the solvent. The resulting solid was purified by chromatography to obtain 269 mg of compound 77, with a 59% yield. MS (ESI): m/z 597.3.

Steps 4 and 5, according to the operations of the Steps C and D in the synthetic route 2, 51 mg of target product 41 was obtained by using the compound 76 (250 mg, 0.43 mmol) obtained in Step 2 as an equivalent reference material, with a

yield of 35%; or 31 mg of target product 42 was obtained by using compound 77 (200 mg, 0.34 mmol) as an equivalent reference material to obtain, with a yield of 26%.

41: UPLC-MS (ESI) m/z 306.2, $t_R$ 0.682 min. $^1$H NMR (400 MHz, Methanol-$d_4$) δ 7.02 (d, J=0.6 Hz, 1H), 4.02-3.94 (m, 4H), 3.49-3.38 (m, 9H), 2.62-2.51 (m, 2H), 2.16 (dtd, J=9.7, 6.2, 2.1 Hz, 2H) ppm.

42: UPLC-MS (ESI) m/z 319.2, $t_R$ 0.595 min. $^1$H NMR (400 MHz, Methanol-$d_4$) δ 6.79 (d, J=0.6 Hz, 1H), 3.98 (td, J=6.2, 0.7 Hz, 1H), 3.80-3.71 (m, 2H), 3.54-3.48 (m, 2H), 3.46-3.36 (m, 4H), 3.15 (s, 6H), 2.63-2.52 (m, 2H), 2.16 (dtd, J=9.7, 6.2, 2.1 Hz, 2H) ppm.

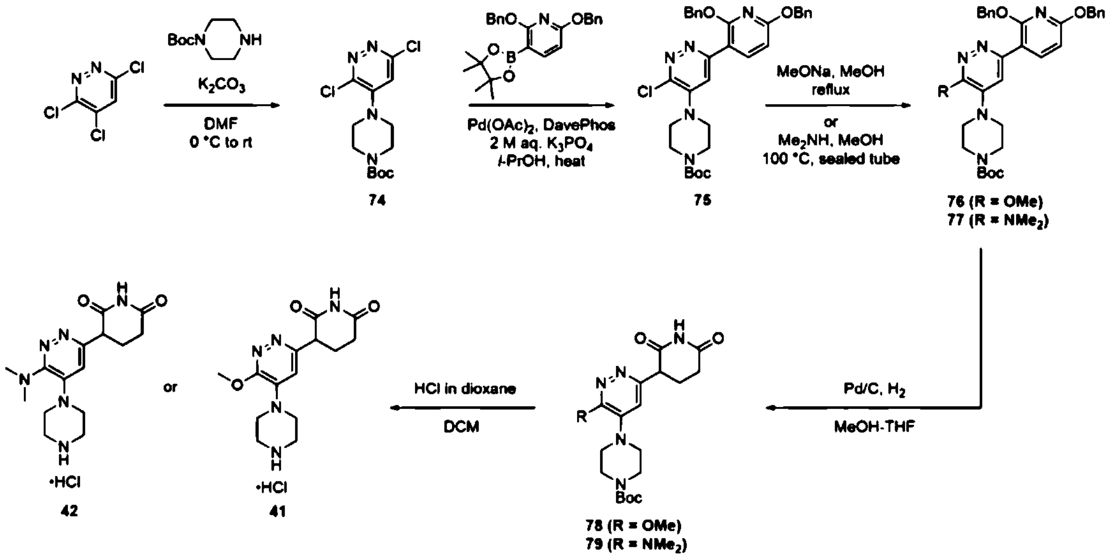


Synthetic route 13 Synthetic route of compounds 41 and 42

TMSCHN2 (trimethylsilyldiazomethane) (0.35 mL, 2.0 M in hexanes, 0.71 mmol, 3.0 equiv.) was slowly added to a solution of compound 56 (100 mg, 0.27 mmol, 1.0 equiv.) in dry THF (tetrahydrofuran) (3 mL). The reaction system was stirred for 12 h at room temperature. No remaining materials were detected by TLC. The reaction was quenched with water. The water phase was extracted with DCM-MeOH (10:1, v/v) (3×5 mL). The organic phases were combined and dried over anhydrous sodium sulfate. The dried organic phase was subjected to suction filtration and underwent rotary evaporation under reduced pressure to remove the solvent. The resulting solid was purified by silica gel flash column chromatography to obtain 78 mg of compound 82, with a yield of 75%. MS (ESI) m/z 334.1 ([M+H-t-Bu]$^+$).

33 mg of target product 43 was obtained by using the compound 82 (50 mg, 0.13 mmol) obtained in the previous step as an equivalent reference material according to the operation of the Step D in the synthetic route 2, with a yield of 80%.

UPLC-MS(ESI)m/z 290.2, $t_R$ 1.021 min. $^1$H NMR (400MHz, Methanol-$d_4$) δ 7.47 (dd, J=8.6, 0.6Hz, 1H), 6.95 (d, J=8.3Hz, 1H), 4.03 (td, J=6.5, 0.7Hz, 1H), 3.94-3.81 (m, 4H), 3.31-3.18 (m, 4H), 3.12 (s, 3H), 2.68-2.51 (m, 2H), 2.26-2.09 (m, 2H) ppm.

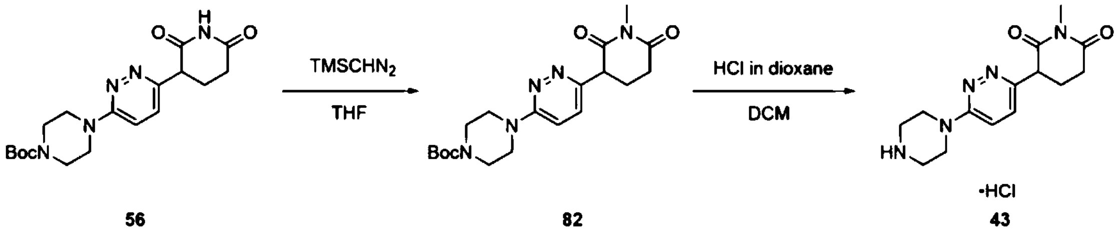


Synthetic route 14 Synthetic route of compound 43

Example 3 Preparation of compound PRTB-01 to compound PRTB-16

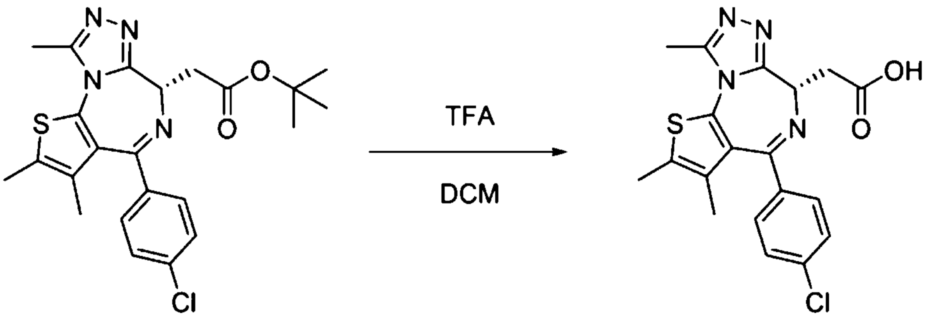


JQ1 JQ1 acid

Synthesis route 15 Synthesis of compound JQ1 acid

At 0°C, TFA (trifluoroacetic acid) (8 mL) was slowly dropwise added to a solution of JQ1 (1.00 g, 2.19 mmol, 1.0 equiv.) in dry DCM (1,2-dichloroethane) (20 mL). The reaction system was stirred for 6 h at room temperature. No remaining materials were detected by TLC. The reaction solution was concentrated under reduced pressure to obtain a residue. The obtained residue was dissolved in EtOAc, washed with saturated brine and dried with anhydrous sodium sulfate. The dried residue was subjected to suction filtration and underwent rotary evaporation under reduced pressure to remove the solvent. The resulting solid was purified by silica gel flash column chromatography to obtain 805 mg of compound JQ1 acid, with a yield of 92%. MS (ESI) m/z 399.1 ([M+H]$^+$).

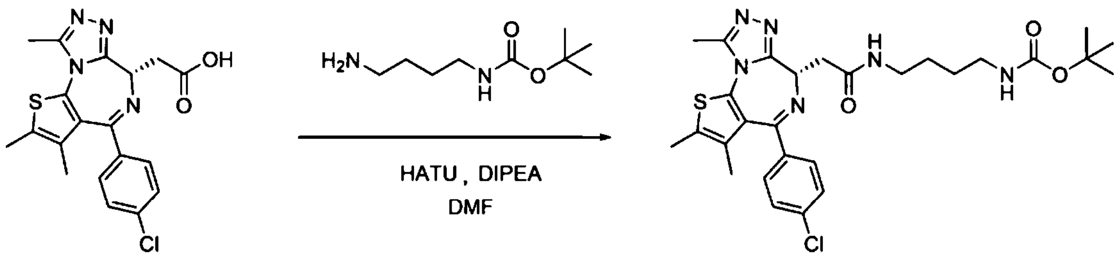


JQ1 acid JQ1-LB1

Synthetic route 16 Synthesis of compound JQ1-LB1

The compound JQ1 acid (60 mg, 0.15 mmol, 1.0 equiv.), tert-butyl(4-aminobutyl)carbamate (30 mg, 0.16 mmol, 1.05 equiv.), HATU (85 mg, 0.22 mmol, 1.5 equiv.) and DIPEA (diisopropylethylamine) (78 μL, 0.45 mmol, 3.0 equiv.) were dissolved in anhydrous DMF (2 mL). The reaction system was stirred at room temperature overnight under argon protection. No remaining materials were detected by TLC. The reaction was quenched with water. The water phase was extracted with EtOAc. The organic phases were combined, washed with saturated brine and dried over anhydrous sodium sulfate. The dried organic phase was subjected to suction filtration and underwent rotary evaporation under reduced pressure to remove the solvent. The resulting solid was purified by silica gel flash column chromatography to obtain 72 mg of compound JQ1-LB1, with a yield of 84%. MS(ESI) m/z 472.0 ([M+H-Boc]$^+$).

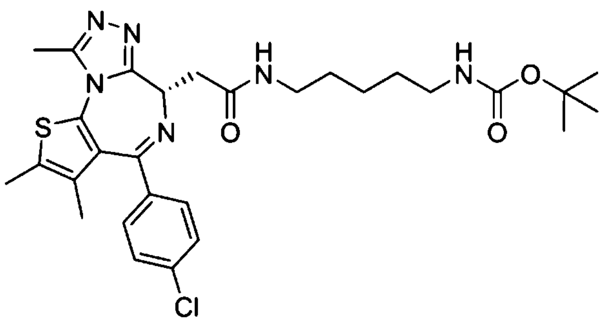

JQ1-LB2LB13

68 mg of compound JQ1-LB2LB13 was obtained by replacing tert-butyl(4-aminobutyl)carbamate with tert-butyl(5-aminopentyl)carbamate according to the synthetic method of compound JQ1-LB1, with a yield of 78%. MS(ESI) m/z 486.0 ([M+H-Boc]$^+$).

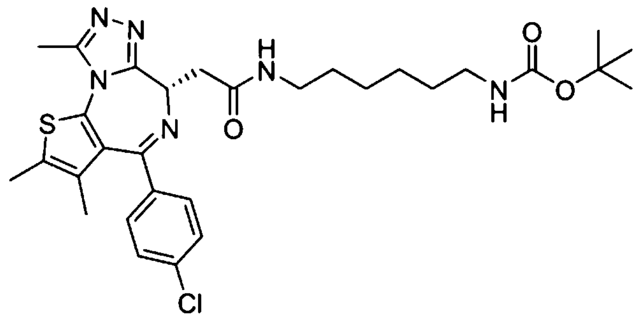

**JQ1-LB3**

65 mg of compound JQ1-LB3 was obtained by replacing tert-butyl(4-aminobutyl)carbamate with tert-butyl(6-aminohexyl)carbamate according to the synthesis method of compound JQ1-LB1, with a yield of 73%. MS(ESI) m/z 500.1 ([M+H-Boc]$^+$).

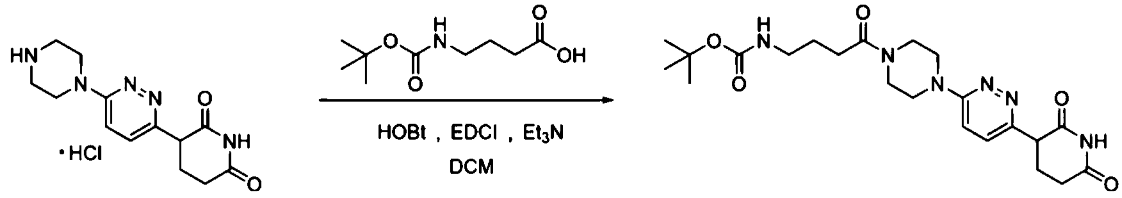


**7** **7-LB4**

Synthetic route 17 Compound 7-LB4

4-((tert-butoxycarbonyl)amino)butanoic acid (91 mg, 0.45 mmol, 2.0 equiv.), EDCI (129 mg, 0.67 mmol, 3.0 equiv.) and HOBt (61 mg, 0.45 mmol, 2.0 equiv.) were dissolved in dry DCM (1,2-dichloroethane) (3 mL). The obtained solution was stirred for 1 h at room temperature. A solution of compound 7 (70 mg, 0.22 mmol, 1.0 equiv.) in anhydrous DCM (1,2-dichloroethane) (2 mL) and TEA (187 μL, 1.35 mmol, 6.0 equiv.) were added to the above reaction solution. The reaction system was stirred for 3 h at room temperature. No remaining materials were detected by TLC. The reaction was quenched with a saturated sodium bicarbonate solution at 0°C. The water phase was extracted with a DCM-MeOH (5:1) mixed solvent. The organic phases were combined and dried over anhydrous sodium sulfate. The dried organic phase was subjected to suction filtration and underwent rotary evaporation under reduced pressure to remove the solvent. The resulting solid was purified by silica gel flash column chromatography to obtain 68 mg of compound 7-LB4, with a yield of 66%. MS (ESI) m/z 361.4 ([M+H-Boc]$^+$).

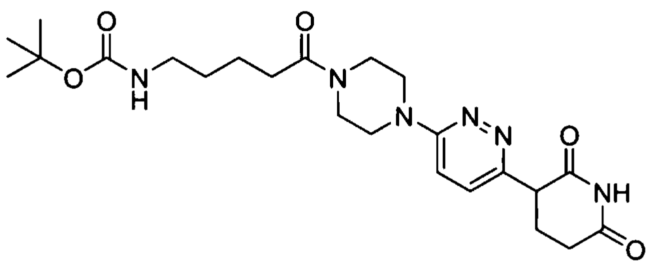

**7-LB5**

77 mg of compound 7-LB5 was obtained by replacing 4-((tert-butoxycarbonyl)amino)butanoic acid with 5-((tert-butoxycarbonyl)amino)pentanoic acid according to the synthesis method of compound 7-LB4, with a yield of 72%. MS(ESI) m/z 375.4 ([M+H-Boc]$^+$).

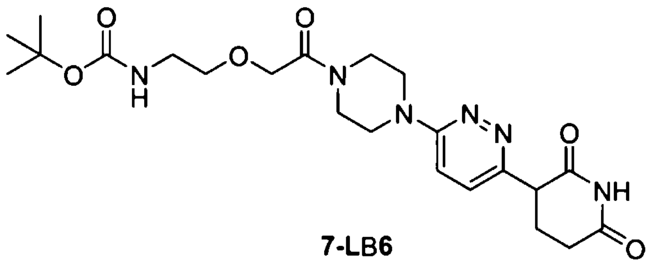

**7-LB6**

70 mg of compound 7-LB6 was obtained by replacing 4-((tert-butoxycarbonyl)amino) butanoic acid with 2-(2-((tert-butoxycarbonyl)amino)ethoxy) acetic acid according to the synthesis method of compound 7-LB4, with a yield of 65%. MS (ESI) m/z 377.4 ([M+H-Boc]+).

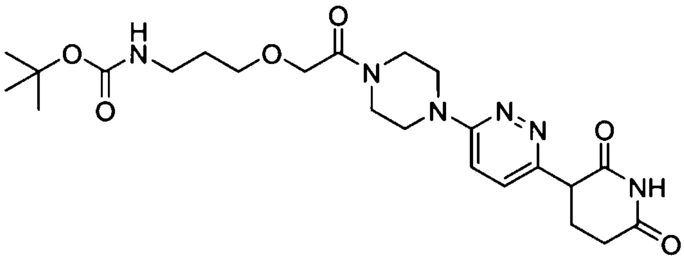

**7-LB7**

67 mg of compound 7-LB7 was obtained by replacing 4-((tert-butoxycarbonyl)amino)butanoic acid with 2-(2-((tert-butoxycarbonyl)amino)ethoxy)acetic acid according to the synthesis method of compound 7-LB4, with a yield of 61%. MS (ESI) m/z 391.4 ([M+H-Boc]+).

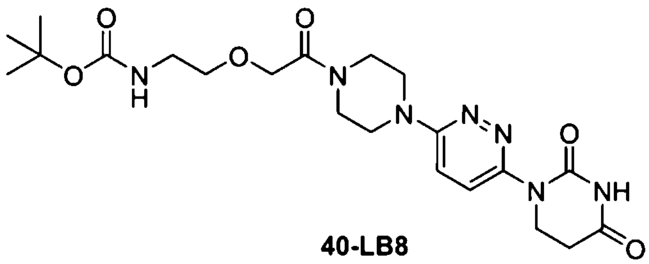

**40-LB8**

75 mg of compound 40-LB8 was obtained by replacing compound 7 with compound 40 according to the synthesis method of compound 7-LB6, with a yield of 70%. MS (ESI) m/z 377.4 ([M+H-Boc]+).

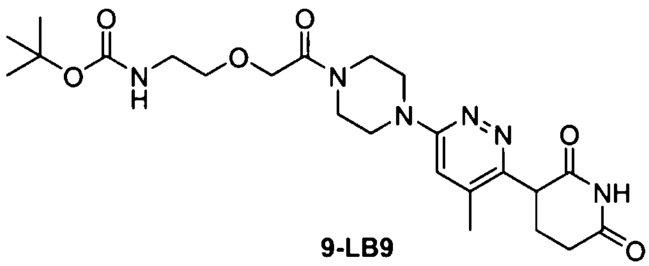

**9-LB9**

76 mg of compound 9-LB9 was obtained by replacing compound 7 with compound 9 according to the synthesis method of compound 7-LB6, with a yield of 69%. MS (ESI) m/z 391.4 ([M+H-Boc]+).

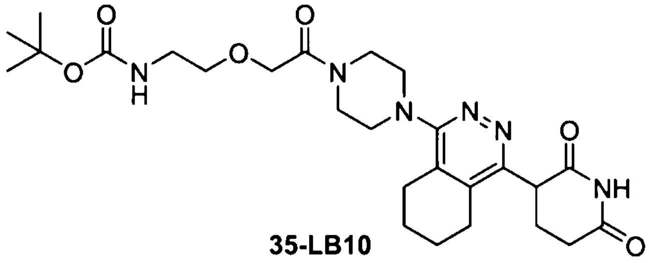

35-LB10

74 mg of compound 35-LB10 was obtained by replacing compound 7 with compound 35 according to the synthesis method of compound 7-LB6, with a yield of 62%. MS (ESI) m/z 431.5 ([M+H-Boc]+).

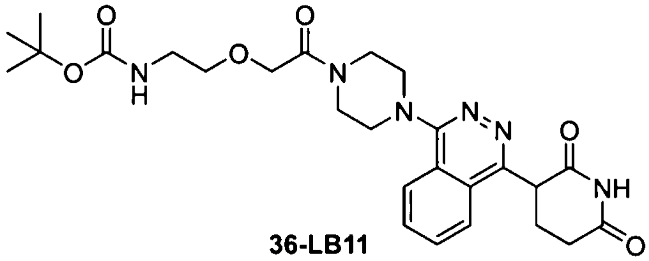

36-LB11

84 mg of compound 36-LB11 was obtained by replacing compound 7 with compound 36 according to the synthesis method of compound 7-LB6, with a yield of 71%. MS (ESI) m/z 427.5 ([M+H-Boc]$^+$).

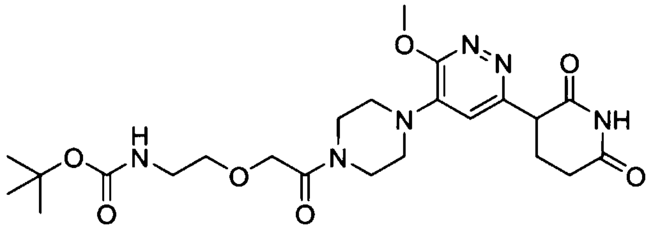

41-LB16

74 mg of compound 41-LB16 was obtained by replacing compound 7 with compound 41 according to the synthesis method of compound 7-LB6, with a yield of 65%. MS (ESI) m/z 407.4 ([M+H-Boc]$^+$).

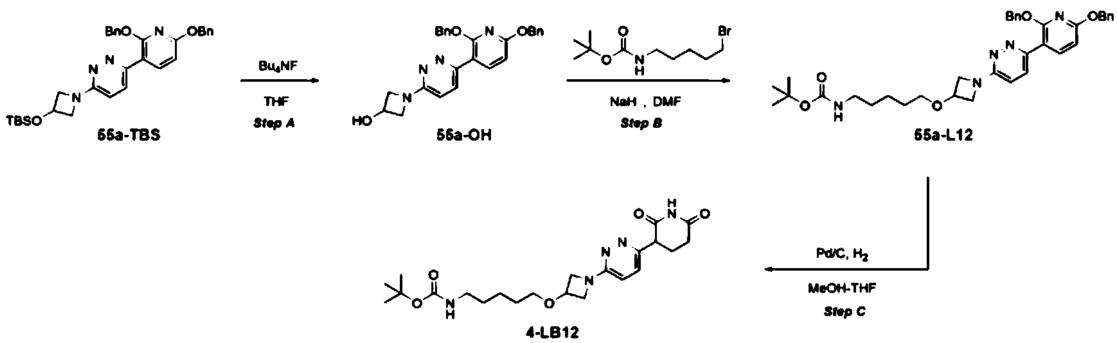


Synthetic route 18 Synthesis of compounds 55a-OH, 55a-LB12 and 4-LB12

[Step A] 1.21 g of compound 55a-TBS was obtained by replacing 3-(benzyloxy)azetidine with 3-((tert-butyldimethylsilyl)oxy)azetidine according to the synthesis method of compound 55a. The compound 55a-TBS (800 mg, 1.44 mmol, 1.0 equiv.) was dissolved in anhydrous THF (10 mL), and then a solution of TBAF (tetrabutylammonium fluoride) in THF (tetrahydrofuran) (2.16 mL, 2.16 mmol, 1.0 M in THF, 1.5 equiv.) was dropwise added to the solution. The reaction mixed solution was stirred for 4 h at room temperature. No remaining materials were detected by TLC. The reaction was quenched with a saturated sodium bicarbonate solution. The water phase was extracted with EtOAc. The organic phases

were combined and dried over anhydrous sodium sulfate. The dried organic phase was subjected to suction filtration and underwent rotary evaporation under reduced pressure to remove the solvent. The resulting solid was purified by chromatography to obtain 594 mg of compound 55a-OH, with a yield of 93%. MS (ESI) m/z 441.5 ([M+H]+).

[Step B] At 0°C, NaH (54 mg, 1.36 mmol, 60 wt%, 1.5 equiv.) was added to a solution of compound 55a-OH (400 mg, 0.91 mmol, 1.0 equiv.) in anhydrous DMF (5 mL). The reaction mixture was stirred for 1 h at room temperature. The reaction mixed solution was placed in an ice-water bath, and then a solution of tert-butyl(5-bromopentyl)carbamate (266 mg, 1.00 mmol, 1.1 equiv.) in anhydrous DMF (2 mL) was dropwise added to the reaction mixed solution. The reaction system continued to be stirred for 5 h at room temperature. No remaining materials were detected by TLC. The reaction was quenched with a saturated ammonium chloride solution. The water phase was extracted with EtOAc. The organic phases were combined and dried over anhydrous sodium sulfate. The dried organic phase was subjected to suction filtration and underwent rotary evaporation under reduced pressure to remove the solvent. The resulting solid was purified by silica gel flash column chromatography to obtain 330 mg of compound 55a-LB12, with a yield of 58%. MS (ESI) m/z 526.7 ([M+H-Boc]+).

[Step C] The compound 55a-LB12 (300 mg, 0.48 mmol, 1.0 equiv.) was dissolved in a mixed solution of MeOH-THF (4:1, 5 mL), and Pd/C (60 mg, 10 wt% on Carbon) was added to the above solution. The reaction system was stirred overnight at room temperature under a hydrogen atmosphere (balloon). No remaining materials were detected by TLC. The reaction system was subjected to suction filtration and rotary evaporation under reduced pressure to remove the solvent. The resulting solid was purified by chromatography to obtain 173 mg of compound 4-LB12, with a yield of 81%. MS (ESI) m/z 348.4 ([M+H-Boc]+).

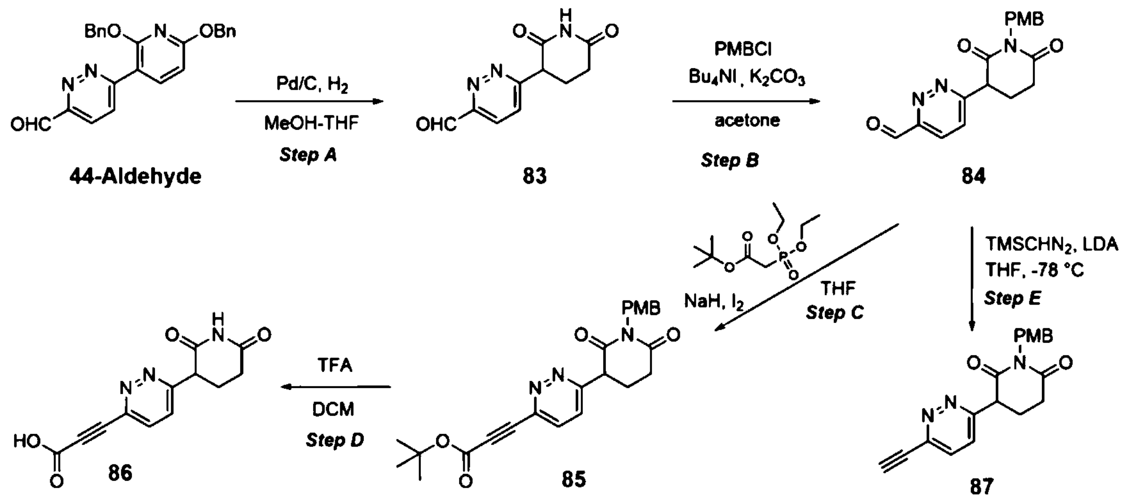


44-Aldehyde, 83, 84, 85, 86, 87

Synthetic route 19 Synthesis of compounds 83, 84, 85, 86 and 87

[Step A] 1.42 g of compound 44-Aldehyde was obtained by replacing (6-chloropyridazin-3-yl)ethan-1-one with 6-chloropyridazine-3-carbaldehyde according to the synthesis method of compound 44. The compound 44-Aldehyde (1.00 g, 2.52 mmol, 1.0 equiv.) was dissolved in a mixed solvent of MeOH-THF (5:2, 14 mL), and then Pd/C (100 mg, 10 wt% on Carbon) was added to the above solution. The reaction system was stirred overnight at room temperature under a hydrogen atmosphere (balloon). No remaining materials were detected by TLC. The reaction system was subjected to suction filtration and rotary evaporation under reduced pressure to remove the solvent. The resulting solid was purified by chromatography to obtain 427 mg of compound 83, with a yield of 77%. MS (ESI) m/z 220.2 ([M+H]+).

[Step B] 530 mg of compound 84 was obtained by using compound 83 as an equivalent reference material (400 mg, 1.82 mmol, 1.0 equiv.) according to the synthesis method of compound 46, with a yield of 86%. MS (ESI) m/z 340.4 ([M+H]+).

[Step C] At 0°C, a solution of tert-butyl 2-(diethoxyphosphoryl)acetate (82 mg, 0.32 mmol, 1.1 equiv.) in anhydrous THF (1 mL) was dropwise added to a suspension of NaH (14 mg, 0.35 mmol, 60 wt%, 1.2 equiv.) in anhydrous THF (1 mL). The reaction solution continued to be stirred for 30 min at the same temperature. At 0°C, $I_2$ (89 mg, 0.35 mmol, 1.2 equiv.) was added to the above mixed solution. The reaction solution continued to be stirred for 3 h at the same temperature. At the same temperature, a solution of compound 84 (100 mg, 0.29 mmol, 1.0 equiv.) in anhydrous THF (1 mL) was dropwise added. After the addition was completed, the reaction system continued to be stirred for 15 min at the same temperature, followed by supplementing NaH (14 mg, 0.35 mmol, 60 wt%, 1.2 equiv.). The reaction system was automatically warmed to room temperature and continued to be stirred for 1 h. No remaining materials were detected by TLC. The reaction was quenched with water. The resulting water phase was extracted with a DCM-MeOH (5:1) mixed

solvent. The organic phases were combined and dried with anhydrous sodium sulfate. The dried organic phase was subjected to suction filtration and underwent rotary evaporation under reduced pressure to remove the solvent. The resulting solid was purified by silica gel flash column chromatography to obtain 97mg of compound 85, with a yield of 76%. MS (ESI) m/z 380.4 ([M+H-t-Bu]$^+$).

[Step D] At 0°C, TFA (trifluoroacetic acid) (2 mL) was dropwise added to a solution of compound 85 (90 mg, 0.21 mmol, 1.0 equiv.) in anhydrous DCM (1,2-dichloroethane) (3 mL). The reaction system was automatically warmed to room temperature and continued to stirred for 4 h. No remaining materials were detected by TLC. The reaction system was subjected to rotary evaporation under reduced pressure to remove all volatile components to obtain 46 mg of crude product 86. The obtained crude product was used directly for the next step without purification.

[Step E] At -78°C, TMSCHN2 (trimethylsilyl)diazomethane (1.90 M in hexanes, 0.18 ml, 0.35 mmol, 1.2 equiv.) was added to a solution of LDA (lithium diisopropylamide) in THF (tetrahydrofuran) (1 mL) (about 1.20 mmol, about 1.20 equiv.). The reaction solution was stirred for 30 min at the same temperature. At -78°C, a solution of compound 84 (100 mg, 0.29 mmol, 1.0 equiv.) in anhydrous THF (1 mL) was dropwise added to the above solution. The reaction system continued to be stirred for 1 h at -78°C and then heated to reflux for 2 h. No remaining materials were detected by TLC. After the reaction system was cooled to room temperature, the reaction was quenched with water. The water phase was extracted with a mixed solvent of DCM (1,2-dichloroethane)-MeOH (5:1). The organic phases were combined and dried over anhydrous sodium sulfate. The dried organic phase was subjected to suction filtration and rotary evaporation under reduced pressure to remove the solvent. The resulting solid was purified by flash silica gel column chromatography to obtain 61 mg of compound 87, with a yield of 63%. MS (ESI) m/z 336.4 ([M+H]$^+$).

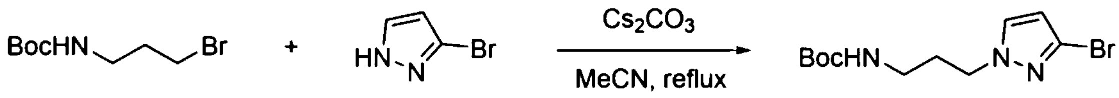


88

Synthetic route 20 Synthesis of compound 88

Compounds 3-bromo-1H-pyrazole (100 mg, 0.68 mmol, 1.0 equiv.), tert-butyl(3-bromopropyl)carbamate (243 mg, 1.02 mmol, 1.5 equiv.) and $Cs_2CO_3$ (443 mg, 1.36 mmol, 2.0 equiv.) were suspended in dry MeCN (10 mL). The reaction mixture was stirred overnight under reflux. No remaining materials were detected by TLC. The reaction system was diluted with MTBE (methyl tert-butyl ether), subjected to suction filtration and rotary evaporation under reduced pressure to remove the solvent. The resulting solid was purified by silica gel flash column chromatography to obtain 104 mg of compound 88, with a yield of 50%. MS (ESI) m/z 205.1 ([M+H-Boc]$^+$).

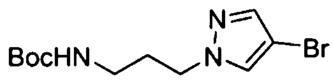

89

187 mg of compound 89 was obtained by replacing compound 3-bromo-1H-pyrazole with 4-bromo-1H-pyrazole according to the synthesis method of compound 88, with a yield of 90%. MS (ESI) m/z 205.1 ([M+H-Boc]$^+$).

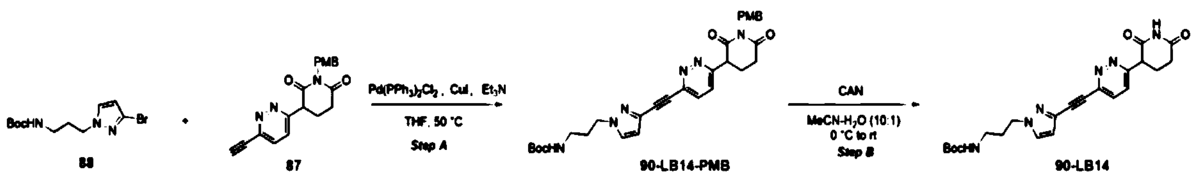


Synthetic route 20 Synthesis of compounds 90-LB14-PMB and 90-LB14

[StepA] Compound 87 (80 mg, 0.24 mmol, 1.0 equiv.), compound 88 (87 mg, 0.29 mmol, 1.2 equiv.), CuI (9 mg, 0.05 mmol, 0.2 equiv.), $Et_3N$ (66 μL, 0.48 mmol, 2.0 equiv.) and $Pd(PPh_3)_2Cl_2$ (17 mg, 0.02 mmol, 0.1 equiv.) were sequentially added to dry THF (tetrahydrofuran) (2 mL). The reaction mixture was purged and backfilled with argon for three times and then stirred for 3 h at 50°C under an argon atmosphere. No remaining materials were detected by TLC. After being cooled to room temperature, the reaction solution was diluted with MTBE (methyl tert-butyl ether), subjected to

suction filtration and rotary evaporation under reduced pressure to remove the solvent. The resulting solid was purified by chromatography to obtain 86 mg of compound 90-LB14-PMB, with a yield of 64%. MS (ESI) m/z 459.5 ([M+H-Boc]$^+$).

[Step B] CAN (ceric ammonium nitrate) (766 mg, 1.40 mmol, 10.0 equiv.) was added in batch to a solution of compound 90-LB14-PMB (80 mg, 0.14 mmol, 1.0 equiv.) in MeCN-$H_2O$ (10 mL, 10:1, v/v) at 0°C under an argon atmosphere. The reaction mixed solution was stirred for 15 min at 0°C and then stirred for 2 h at room temperature. No remaining materials were detected by TLC, and then saturated aqueous sodium bicarbonate solution was added. The water phase was extracted with a mixed solvent of DCM (1,2-dichloroethane)-MeOH (5:1, v/v). The organic phases were combined and dried with anhydrous sodium sulfate. The dried organic phase was subjected to suction filtration and underwent rotary evaporation under reduced pressure to remove the solvent. The resulting solid was purified by using a TLC preparative plate to obtain 45 mg of compound 90-LB14, with a yield of 72%. MS (ESI) m/z 339.2 ([M+H-Boc]$^+$).

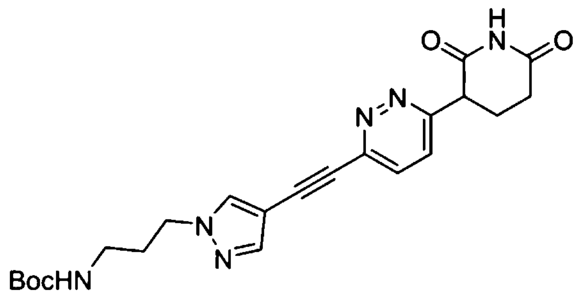

**91-LB15**

42 mg of compound 91-LB15 was obtained according to the synthetic method of compound 90-LB14, with a yield of 68%. MS (ESI) m/z 339.2 ([M+H-Boc]$^+$).

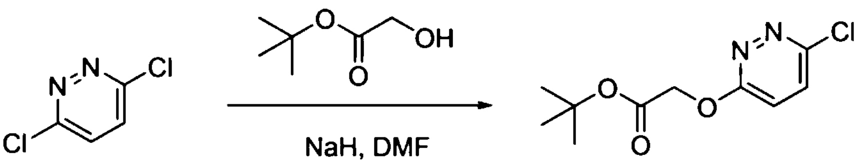


**92**

Synthetic route 21 Synthesis of compound 92

At 0°C, NaH (644 mg, 16.11 mmol, 60 wt%, 1.2 equiv.) was added to dry DMF (2 mL), and then tert-butyl 2-hydroxyacetate (1.77 g, 13.43 mmol, 1.0 equiv.) was added to the above suspension. The reaction system was stirred for 30 min at the same temperature and then 3,6-dichloropyridazine (2.00 g, 13.43 mmol, 1.0 equiv.) was added in one portion. The resulting mixture was automatically warmed to room temperature under an argon atmosphere and continued to be stirred overnight. No remaining materials were detected by TLC. The reaction was carefully quenched with ice water. The water phase was extracted with EtOAc. The organic phases were combined and washed with saturated brine, subjected to suction filtration and concentrated under reduced pressure. The resulting solid was purified by column chromatography to obtain 2.06 g of compound 92, with a yield of 63%. MS (ESI) m/z 189.6 ([M+H-t-Bu]$^+$).

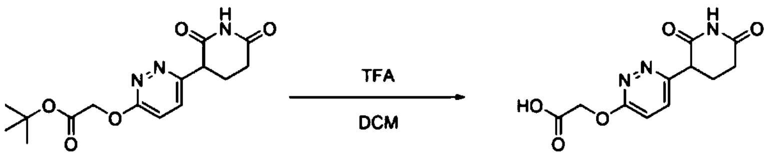


**93** **94**

Synthetic route 22 Synthesis of compounds 93 and 94

The compound 93 was synthesized by using compound 92 according to the synthesis method of compound 8. The compound 93 (1.00 g, 3.11 mmol, 1.0 equiv.) was dissolved in dry DCM (1,2-dichloroethane) (20 mL), and then TFA (trifluoroacetic acid) (5 mL) was slowly added to the above solution at 0°C. The reaction system was automatically warmed to room temperature under an argon atmosphere and continued to be stirred for 4 h. No remaining materials were detected by TLC. The reaction system was subjected to rotary evaporation under reduced pressure to remove the solvent to obtain

842 mg of crude product compound 94. The obtained crude product was directly used for the next step without further purification. MS (ESI) m/z 266.2 ([M+H]+).

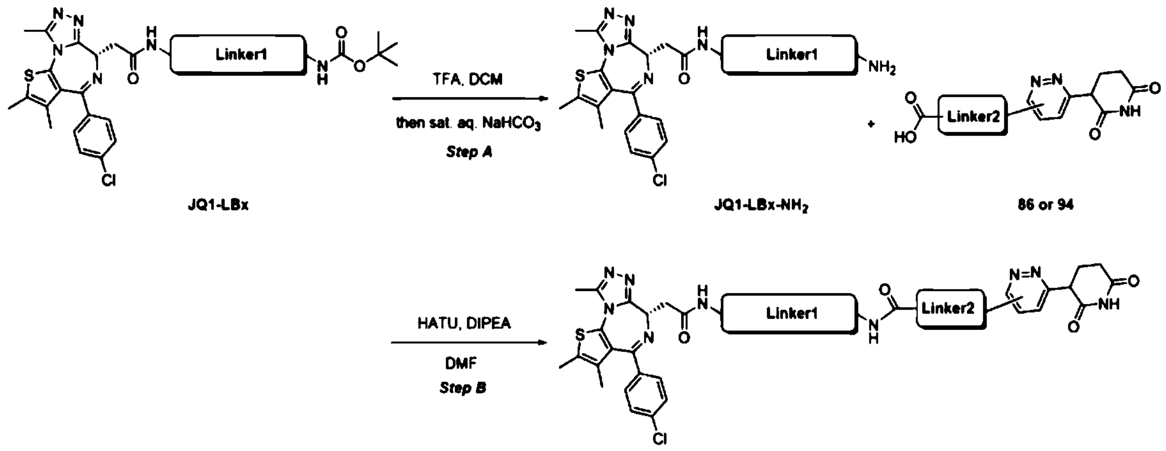


JQ1-LBx
JQ1-LBx-NH2
86 or 94
PRTB-01 ~ PRTB-03, PRTB-13

Synthetic route 23 Synthesis of protein degraders PRTB-01, PRTB-02, PRTB-03 and PRTB-13

The protein degraders PRTB-01, PRTB-02, PRTB-03 and PRTB-13 were synthesized according to the synthetic strategy in the above figure.

[Step A] JQ1-LB1 or JQ1-LB2LB13 or JQ1-LB3 was dissolved in dry DCM (1,2-dichloroethane) (1,2-dichloroethane) (0.2M), and then TFA (trifluoroacetic acid) (1/3 volume of DCM) was slowly added to the solution at 0°C. The reaction system was automatically warmed to room temperature under an argon atmosphere and continued to be stirred for 4-6 h. No remaining materials were detected by LC-MS. The reaction system was subjected to rotary evaporation under reduced pressure to remove the solvent. The residue was dissolved in methanol, and then a saturated sodium bicarbonate solution was added to adjust the pH to 8. Subsequently, the reaction mixture was extracted with a DCM-MeOH (5:1, v/v) mixed solvent. The organic phases were combined and dried over anhydrous sodium sulfate. The dried organic phase was filtered and subjected to rotary evaporation under reduced pressure to remove the solvent. The residue continued to be subjected to rotary evaporation to dryness under reduced pressure for 30 min at 45°C and 0.6 mbar to obtain crude product JQ1-LBx-$NH_2$. The obtained crude product was directly used for the next step.

[Step B] JQ1-LB1 (1.0 equiv.) and 94 (1.0 equiv.), or JQ1-LB2LB13 (1.0 equiv.) and 94 (1.0 equiv.), or JQ1-LB3 (1.0 equiv.) and 94 (1.0 equiv.), or JQ1-LB2LB13 (1.0 equiv.) and 86 (1.0 equiv.) were dissolved in dry DMF (0.2 M), and then HATU (2-(7-azabenzotriazol-1-yl)-N,N,N',N'-tetramethyluronium hexafluorophosphate) (1.5 equiv.) and DIPEA (diisopropylethylamine) (3.0 equiv.) were sequentially added. The reaction system was stirred overnight at room temperature under argon protection. When the complete consumption of one of the materials was detected by LC-MS, the reaction was quenched with water. The water phase was extracted with a mixed solvent of DCM-MeOH (5:1, v/v). The organic phases were combined and dried with anhydrous sodium sulfate. The dried organic phase was subjected to suction filtration and underwent rotary evaporation under reduced pressure to remove the solvent. The resulting solid was purified by preparative HPLC to obtain PRTB-01, PRTB-02, PRTB-03 and PRTB-13, respectively.

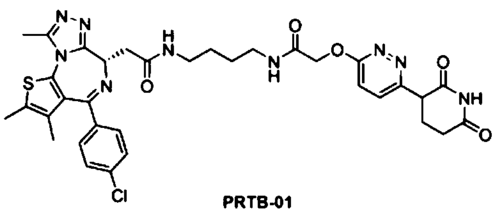

PRTB-01

The protein degrader PRTB-01 was synthesized and purified according to the above general synthetic strategy to obtain 12 mg of target product. $^1$H NMR (400MHz, DMSO-$d_6$) δ 11.07 (s, 1H), 7.74-7.62 (m, 4H), 7.62-7.55 (m, 1H), 7.55-7.48 (m, 2H), 7.09 (d, J=8.1Hz, 1H), 5.78 (t, J= 9.3Hz, 1H), 4.69 (d, J=0.6Hz, 2H), 3.73-3.65 (m, 1H), 3.24-2.98 (m, 6H), 2.63 (s, 3H), 2.59-2.48 (m, 2H), 2.21-2.04 (m, 2H), 1.45 (p, J=2.8Hz, 4H) ppm. UPLC-MS(ESI)m/z 718.2, $t_R$ 0.842min, 96%purity.

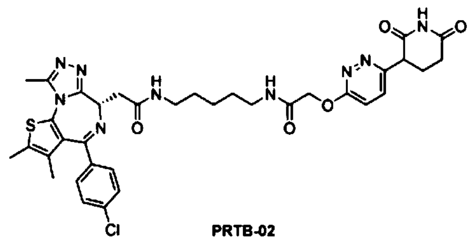

PRTB-02

The protein degrader PRTB-02 was synthesized and purified according to the above general synthetic strategy to obtain 7 mg of target product. $^1$H NMR (400MHz, DMSO-$d_6$) δ11.07 (s, 1H), 7.71 (t, J=4.8Hz, 1H), 7.71-7.62 (m, 3H), 7.62-7.55 (m, 1H), 7.55-7.48 (m, 2H), 7.09 (d, J=8.1Hz, 1H), 5.78 (t, J=9.3Hz, 1H), 4.69 (d, J=0.6Hz, 2H), 3.73-3.65 (m, 1H), 3.22-2.98 (m, 7H), 2.63 (s, 3H), 2.59-2.48 (m, 2H), 2.23-2.04 (m, 2H), 1.53-1.41 (m, 4H), 1.39-1.27(m, 2H) ppm. UPLC-MS(ESI)m/z 732.2, $t_R$ 0.855min, 98% purity.

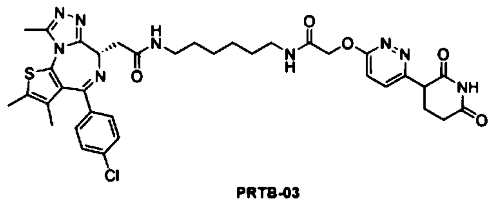

PRTB-03

The protein degrader PRTB-03 was synthesized and purified according to the above general synthetic strategy to obtain 12 mg of target product. $^1$H NMR (400MHz, DMSO-$d_6$) δ 11.07 (s, 1H), 7.70 (t, J=4.7Hz, 1H), 7.71-7.62 (m, 3H), 7.62-7.55 (m, 1H), 7.55-7.48 (m, 2H), 7.09 (d, J=8.1Hz, 1H), 5.78 (t, J=9.3Hz, 1H), 4.69 (d, J=0.7Hz, 2H), 3.73-3.65 (m, 1H), 3.23-3.10 (m, 4H), 3.13-3.04 (m, 1H), 3.08-2.98 (m, 1H), 2.63 (s, 3H), 2.59-2.48 (m, 2H), 2.23-2.04 (m, 2H), 1.58-1.45 (m, 4H), 1.40-1.27 (m, 4H) ppm. UPLC-MS(ESI)m/z 746.3, $t_R$ 0.851min, 96% purity.

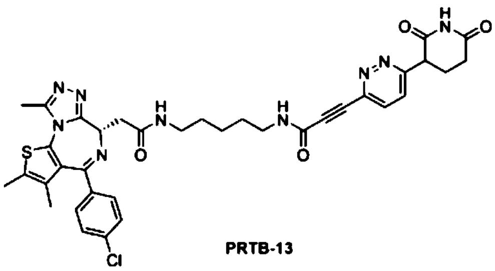

PRTB-13

The protein degrader PRTB-13 was synthesized and purified according to the above general synthetic strategy to obtain 15 mg of target product. $^1$H NMR (400MHz, DMSO-$d_6$) δ 11.07 (s, 1H), 7.97 (t, J=4.6Hz, 1H), 7.74-7.62 (m, 5H), 7.55-7.48 (m, 2H), 5.78 (t, J=9.3Hz, 1H), 3.69 (t, J=6.2Hz, 1H), 3.25-2.98 (m, 6H), 2.63 (s, 3H), 2.59-2.48 (m, 2H), 2.22-2.04 (m, 2H), 1.60-1.41 (m, 4H), 1.38-1.26 (m, 2H) ppm. UPLC-MS(ESI)m/z 726.2, $t_R$ 0.780min, 98% purity.

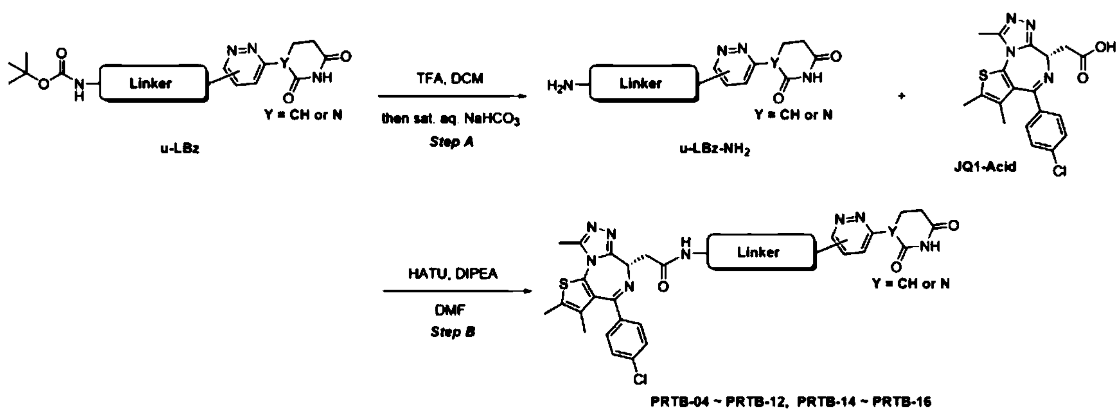


u-LBz
u-LBz-NH2
JQ1-Acid
PRTB-04 ~ PRTB-12, PRTB-14 ~ PRTB-16

Synthetic route 24 Synthesis of protein degraders PRTB-04 to PRTB-12 and PRTB-14 to PRTB-16

The protein degraders PRTB-04 to PRTB-12 and PRTB-14 to PRTB-16 were synthesized according to the synthetic strategy in the FIG. as described above.

[Step A] 7-LB4 or 7-LB5 or 7-LB6 or 7-LB7 or 40-LB8 or 9-LB9 or 35-LB10 or 36-LB11 or 4-LB12 or 90-LB14 or 91-LB15 or 41-LB16 was dissolved in dry DCM (1,2-dichloroethane) (0.2M), and then TFA (trifluoroacetic acid) (1/2 volume of DCM) was slowly added to the above solution at 0°C. The reaction system was automatically warmed to room temperature under an argon atmosphere and continued to be stirred for 4 h. No remaining materials were detected by LC-MS. The reaction system was subjected to rotary evaporation under reduced pressure to remove the solvent. The residue was dissolved in methanol, and then a saturated sodium bicarbonate solution was added to adjust the pH to 8. Subsequently, the reaction mixture was extracted with a DCM-MeOH (5:1, v/v) mixed solvent. The organic phases were combined and dried over anhydrous sodium sulfate. The dried organic phase was filtered and subjected to rotary evaporation under reduced pressure to remove the solvent. The reaction mixture continued to be subjected to rotary evaporation to dryness at 45°C and 0.6 mbar for 30 min to obtain a crude product u-LBz-$NH_2$. The obtained crude product was used directly for the next step without further purification.

[Step B] JQ1-Acid (1.0 equiv.) and one of a plurality of crude products u-LBz-$NH_2$ obtained in the previous step (1.0 equiv.) were dissolved in dry DMF (0.2 M), and then HATU (1.5 equiv.) and DIPEA (diisopropylethylamine) (3.0 equiv.) were sequentially added. The reaction system was stirred at room temperature overnight under argon protection. When the complete consumption of one of the materials was detected by LC-MS, the reaction was quenched with water. The water phase was extracted with a mixed solvent of DCM-MeOH (5:1, v/v). The organic phases were combined and dried with anhydrous sodium sulfate. The dried organic phase was subjected to suction filtration and underwent rotary evaporation under reduced pressure to remove the solvent. The resulting solid was purified by preparative HPLC to obtain PRTB-04, PRTB-05, PRTB-06, PRTB-07, PRTB-08, PRTB-09, PRTB-10, PRTB-11, PRTB-12, PRTB-14, PRTB-15 and PRTB-16, respectively.

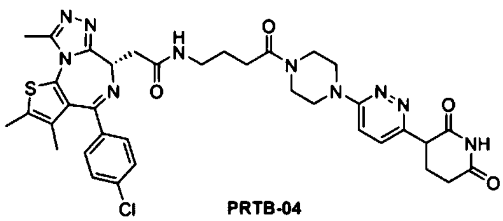

PRTB-04

The protein degrader PRTB-04 was synthesized and purified according to the above general synthetic strategy to obtain 18 mg of target product. $^1$H NMR (400 MHz, DMSO-$d_6$) δ 11.07 (s, 1H), 7.78 (d, J = 9.5 Hz, 1H), 7.78 (s, 1H), 7.69-7.62 (m, 2H), 7.55-7.46 (m, 2H), 7.46 (d, J = 0.7 Hz, 1H), 6.92 (d, J = 8.3 Hz, 1H), 5.78 (t, J = 9.3 Hz, 1H), 3.72-3.52 (m, 11H), 3.13 (td, J = 6.0, 4.7 Hz, 2H), 3.07 (d, J = 5.9 Hz, 1H), 3.08-2.98 (m, 1H), 2.63 (s, 3H), 2.59-2.48 (m, 2H), 2.29 (d, J = 0.9 Hz, 1H), 2.21-2.04 (m, 2H), 1.76 (tt, J = 7.7, 5.9 Hz, 2H) ppm. UPLC-MS (ESI) m/z 743.3, $t_R$ 0.671 min, 98% purity.

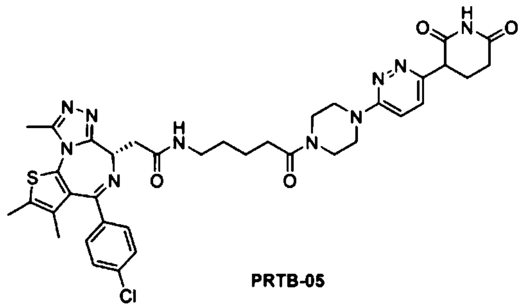

PRTB-05

The protein degrader PRTB-05 was synthesized and purified according to the above general synthetic strategy to obtain 10 mg of target product. $^1$H NMR (400 MHz, DMSO-$d_6$) δ 11.07 (s, 1H), 7.71-7.62 (m, 2H), 7.55-7.44 (m, 2H), 6.92 (d, J = 8.3 Hz, 1H), 5.78 (t, J = 9.3 Hz, 1H), 3.72-3.52 (m, 7H), 3.20-3.12 (m, 1H), 3.16-3.06 (m, 1H), 3.09-2.98 (m, 1H), 2.63 (s, 2H), 2.58-2.49 (m, 1H), 2.28-2.21 (m, 1H), 2.25-2.04 (m, 2H), 1.60-1.46 (m, 3H) ppm. UPLC-MS (ESI) m/z 757.3, $t_R$ 0.685 min, 98% purity.

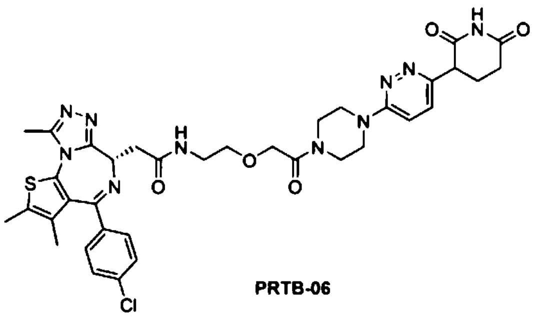

PRTB-06

The protein degrader PRTB-06 was synthesized and purified according to the above general synthetic strategy to obtain 7 mg of target product. $^1$H NMR (400 MHz, DMSO-$d_6$) δ 11.07 (s, 1H), 7.76 (t, J=4.8 Hz, 1H), 7.69-7.62 (m, 2H), 7.55-7.48 (m, 2H), 7.51-7.44 (m, 1H), 6.92 (d, J=8.3 Hz, 1H), 5.78 (t, J=9.3 Hz, 1H), 4.13 (s, 2H), 3.72-3.58 (m, 9H), 3.62-3.55 (m, 2H), 3.40-3.24 (m, 2H), 3.14-2.98 (m, 2H), 2.63 (s, 3H), 2.59-2.48 (m, 2H), 2.21-2.04 (m, 2H) ppm. UPLC-MS (ESI) m/z 759.3, $t_R$ 0.601 min, 98% purity.

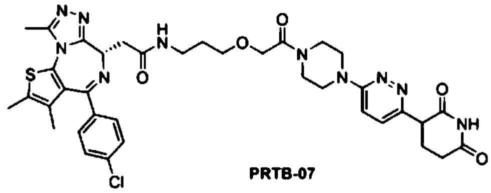

PRTB-07

The protein degrader PRTB-07 was synthesized and purified according to the above general synthetic strategy to obtain 12 mg of target product. $^1$HNMR (400MHz, DMSO-$d_6$) δ 11.07 (s, 1H), 7.69 (t, J=4.6Hz, 1H), 7.69-7.62 (m, 2H), 7.55-7.44 (m, 3H), 6.92 (d, J=8.3Hz, 1H), 5.78 (t, J=9.3Hz, 1H), 4.14 (s, 2H), 3.72-3.51 (m, 11H), 3.19 (td, J=6.1, 4.6Hz, 2H), 3.14-2.98 (m, 2H), 2.59-2.48 (m, 2H), 2.22-2.04 (m, 2H), 1.80 (pd, J=6.0, 2.5Hz, 2H) ppm. UPLC-MS (ESI) m/z 773.3, $t_R$ 0.618min, 98% purity.

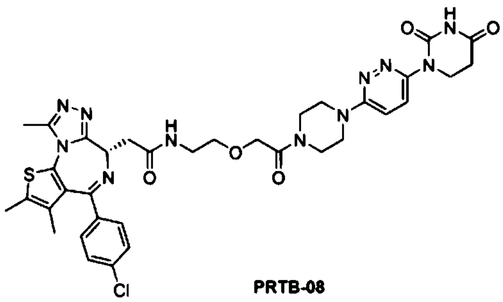

PRTB-08

The protein degrader PRTB-08 was synthesized and purified according to the above general synthetic strategy to obtain 12 mg of target product. $^1$HNMR (400MHz, DMSO-$d_6$) δ 10.64 (s, 1H), 7.76 (t, J=4.8Hz, 1H), 7.69-7.62 (m, 2H), 7.55-7.48 (m, 2H), 7.06 (d, J=7.9Hz, 1H), 7.01 (d, J=7.9Hz, 1H), 5.78 (t, J=9.3Hz, 1H), 4.13 (s, 2H), 3.95 (ddd, J=16.5, 7.0, 4.2Hz, 2H), 3.71-3.55 (m, 11H), 3.39-3.25 (m, 2H), 3.14-2.98 (m, 2H), 2.71 (ddd, J=7.3, 4.2, 3.3Hz, 2H), 2.31 (d, J=1.5Hz, 6H) ppm. UPLC-MS (ESI)m/z 760.2, $t_R$ 0.511min, 99% purity.

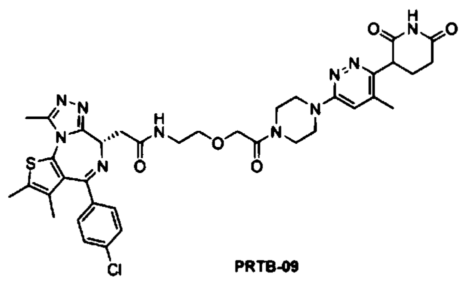

PRTB-09

The protein degrader PRTB-09 was synthesized and purified according to the above general synthetic strategy to

obtain 15 mg of target product. $^1$H NMR (400 MHz, DMSO-$d_6$) δ 11.13 (s, 1H), 7.76 (t, J = 4.8 Hz, 1H), 7.69 - 7.62 (m, 2H), 7.55 - 7.48 (m, 2H), 6.67 (d, J = 0.9 Hz, 1H), 5.78 (t, J = 9.3 Hz, 1H), 4.15 - 4.07 (m, 3H), 3.72 (dd, J = 6.7, 3.8 Hz, 2H), 3.69 - 3.63 (m, 4H), 3.60 (ddd, J = 11.9, 6.7, 3.8 Hz, 4H), 3.39 - 3.25 (m, 2H), 3.14 - 2.98 (m, 2H), 2.64 - 2.48 (m, 2H), 2.40 (d, J = 0.7 Hz, 3H), 2.31 (d, J = 1.5 Hz, 6H), 2.22 - 2.05 (m, 2H) ppm. UPLC-MS(ESI) m/z 773.3, $t_R$ 0.624 min, 98% purity.

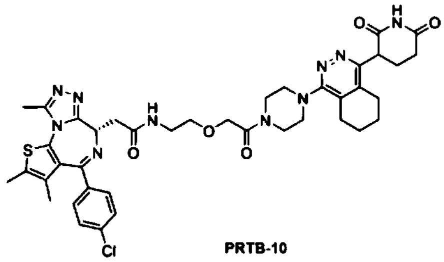

PRTB-10

The protein degrader PRTB-10 was synthesized and purified according to the above general synthetic strategy to obtain 8 mg of target product. $^1$H NMR (400 MHz, DMSO-$d_6$) δ 11.12 (s, 1H), 7.76 (t, J = 4.8 Hz, 1H), 7.69 - 7.62 (m, 2H), 7.55 - 7.48 (m, 2H), 5.78 (t, J = 9.3 Hz, 1H), 4.13 (s, 2H), 4.06 (t, J = 6.4 Hz, 1H), 3.75 - 3.54 (m, 11H), 3.39 - 3.25 (m, 2H), 3.14 - 2.96 (m, 4H), 2.95 - 2.85 (m, 2H), 2.64 - 2.48 (m, 2H), 2.31 (d, J = 1.5 Hz, 6H), 2.25 - 2.07 (m, 2H), 1.90 - 1.78 (m, 4H) ppm. UPLC-MS (ESI) m/z 813.3, $t_R$ 0.701 min, 96% purity.

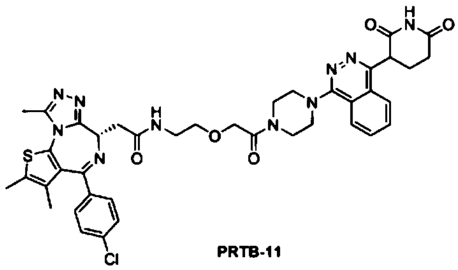

PRTB-11

The protein degrader PRTB-11 was synthesized and purified according to the above general synthetic strategy to obtain 5 mg of target product. $^1$H NMR (400 MHz, DMSO-$d_6$) δ 11.17 (s, 1H), 8.33 (dd, J = 8.8, 1.3 Hz, 1H), 8.13 (dd, J = 8.6, 1.3 Hz, 1H), 7.83 (ddd, J = 9.1, 7.8, 1.3 Hz, 1H), 7.76 (t, J = 4.8 Hz, 1H), 7.71 (ddd, J = 9.0, 7.7, 1.3 Hz, 1H), 7.69 - 7.62 (m, 2H), 7.55 - 7.48 (m, 2H), 5.78 (t, J = 9.3 Hz, 1H), 4.13 (s, 2H), 3.83 - 3.70 (m, 5H), 3.67 (t, J = 4.3 Hz, 2H), 3.63 - 3.54 (m, 4H), 3.40 - 3.24 (m, 2H), 3.14 - 2.98 (m, 2H), 2.63 (s, 3H), 2.62 - 2.48 (m, 2H), 2.31 (s, 3H), 2.25 - 2.08 (m, 2H) ppm. UPLC-MS(ESI) m/z 809.3, $t_R$ 0.695 min, 96% purity.

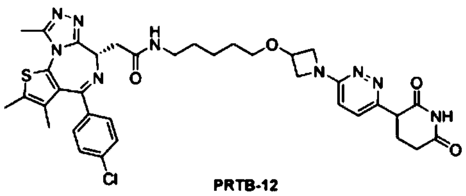

PRTB-12

The protein degrader PRTB-12 was synthesized and purified according to the above general synthetic strategy to obtain 11 mg of target product. $^1$H NMR (400 MHz, DMSO-$d_6$) δ 11.07 (s, 1H), 7.71 - 7.62 (m, 3H), 7.55 - 7.48 (m, 2H), 7.51 - 7.44 (m, 1H), 6.94 (d, J = 8.6 Hz, 1H), 5.78 (t, J = 9.3 Hz, 1H), 4.21 (p, J = 3.7 Hz, 1H), 3.93 (dd, J = 12.3, 3.7 Hz, 2H), 3.79 (dd, J = 12.5, 3.8 Hz, 2H), 3.68 (td, J = 6.2, 0.7 Hz, 1H), 3.48 (t, J = 6.2 Hz, 2H), 3.13 (qd, J = 5.4, 1.6 Hz, 2H), 3.12 - 3.04 (m, 1H), 3.08 - 2.98 (m, 1H), 2.63 (s, 3H), 2.59 - 2.48 (m, 2H), 2.22 - 2.04 (m, 2H), 1.62 - 1.48 (m, 2H), 1.53 - 1.33 (m, 4H) ppm. UPLC-MS (ESI) m/z 730.3, $t_R$ 0.885 min, 97% purity.

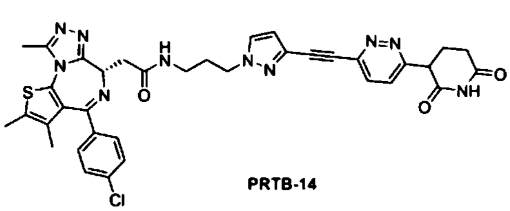

PRTB-14

The protein degrader PRTB-14 was synthesized and purified according to the above general synthetic strategy to obtain 16 mg of target product. $^1$H NMR (400 MHz, DMSO-$d_6$) δ 111.07 (s, 1H), 7.75 (t, J = 3.8 Hz, 1H), 7.72 - 7.62 (m, 4H), 7.56 (d, J = 3.3 Hz, 1H), 7.54 - 7.48 (m, 2H), 6.37 (d, J = 3.1 Hz, 1H), 5.78 (t, J = 9.3 Hz, 1H), 4.10 (td, J = 5.3, 0.7 Hz, 2H), 3.72 - 3.65 (m, 1H), 3.20 - 2.98 (m, 4H), 2.63 (s, 3H), 2.59 - 2.48 (m, 2H), 2.22 - 2.04 (m, 2H), 1.96 (p, J = 5.5 Hz, 2H) ppm. UPLC-MS(ESI) m/z 721.2, $t_R$ 1.157 min, 97% purity.

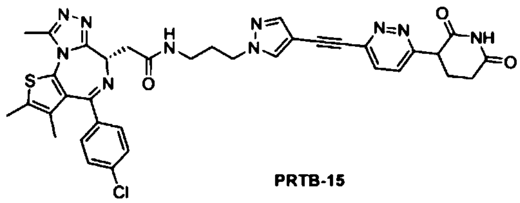

PRTB-15

The protein degrader PRTB-15 was synthesized and purified according to the above general synthetic strategy to obtain 18 mg of target product. $^1$H NMR (400 MHz, DMSO-$d_6$) δ 11.07 (s, 1H), 7.75 (t, J = 3.8 Hz, 1H), 7.73 - 7.65 (m, 2H), 7.69 - 7.61 (m, 4H), 7.55 - 7.48 (m, 2H), 5.78 (t, J = 9.3 Hz, 1H), 4.10 (td, J = 5.4, 1.2 Hz, 2H), 3.72 - 3.65 (m, 1H), 3.16 (td, J = 5.7, 3.8 Hz, 2H), 3.15 - 2.98 (m, 2H), 2.59 - 2.48 (m, 2H), 2.22 - 2.04 (m, 2H), 1.96 (p, J = 5.5 Hz, 2H) ppm. UPLC-MS (ESI) m/z 721.2, $t_R$ 1.133 min, 97% purity.

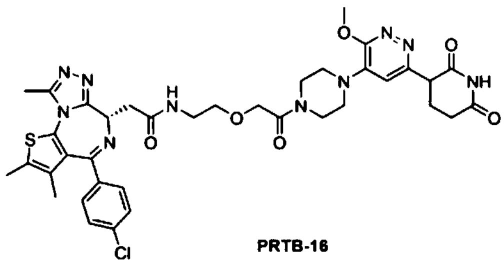

PRTB-16

The protein degrader PRTB-16 was synthesized and purified according to the above general synthetic strategy to obtain 12 mg of target product. $^1$H NMR (400 MHz, DMSO-$d_6$) δ 11.08 (s, 1H), 7.76 (t, J = 4.8 Hz, 1H), 7.69 - 7.62 (m, 2H), 7.55 - 7.48 (m, 2H), 7.02 (d, J = 0.6 Hz, 1H), 5.78 (t, J = 9.3 Hz, 1H), 4.13 (s, 2H), 4.03 - 3.95 (m, 1H), 3.98 (s, 3H), 3.70 - 3.55 (m, 6H), 3.40 - 3.18 (m, 7H), 3.14 - 2.98 (m, 2H), 2.63 (s, 3H), 2.59 - 2.49 (m, 2H), 2.31 (s, 3H), 2.22 - 2.05 (m, 2H) ppm. UPLC-MS(ESI) m/z 789.3, $t_R$ 0.709 min, 96% purity.

Example 4 Preparation of compound PRTA-01 to compound PRTA-06

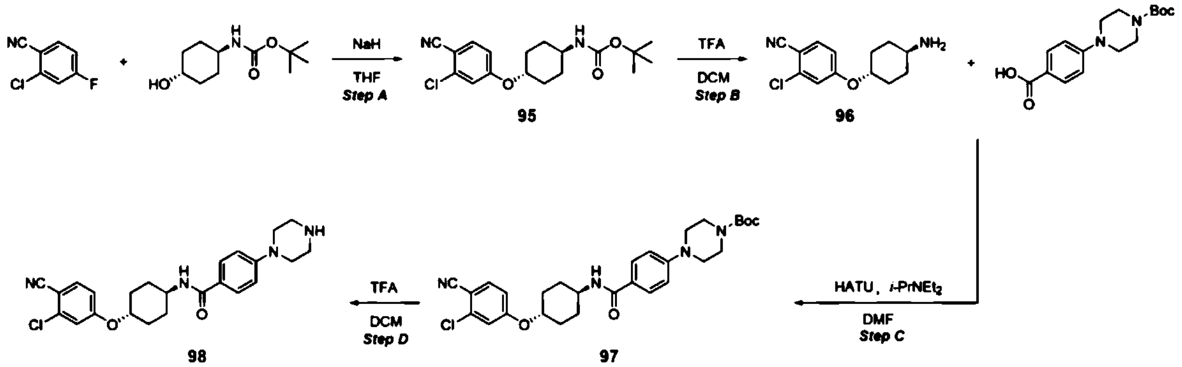


Synthetic route 25 Synthesis of compounds 95, 96 and 97

[Step A] Tert-butyl ((1r,4r)-4-hydroxycyclohexyl)carbamate (5.00 g, 23.22 mmol, 1.0 equiv.) was dissolved in dry THF (100 mL), and then NaH (2.09 g, 52.25 mmol, 60 wt%, 2.25 equiv.) was added in batch to the above reaction solution at 0°C. The reaction system was warmed to room temperature automatically under argon atmosphere and continued to be stirred for 40 min. At 0°C, 2-chloro-4-fluorobenzonitrile (5.42 g, 34.84 mmol, 1.5 equiv.) was added to the above reaction

solution. The reaction system was automatically warmed to room temperature and continued to be stirred for 5 h. No remaining materials were detected by TLC. The reaction mixture was poured into the ice-water mixture. The water phase was extracted with EtOAc. The organic phases were combined, washed with saturated brine and dried over anhydrous sodium sulfate. The dried organic phase was filtered and subjected to rotary evaporation under reduced pressure to remove the solvent. The resulting solid was purified by chromatography to obtain 6.96 g of compound 95, with a yield of 85%. MS (ESI) m/z 251.1 ([M+H-Boc]$^+$).

[Step B] At 0°C, TFA (trifluoroacetic acid) (1.85 mL, 28.48 mmol, 4.0 equiv.) was slowly added to a solution of compound 95 (2.50 g, 7.12 mmol, 1.0 equiv.) in dry DCM (1,2-dichloroethane) (30 mL). The reaction system was stirred for 3 h at 0°C. No remaining materials were detected by TLC. The reaction solution was diluted with DCM (1,2-dichloroethane) and then subjected to rotary evaporation under reduced pressure. Then, the residue was dissolved in methanol and then diluted with water. A saturated sodium bicarbonate solution was added to adjust the pH to 8. The water phase was extracted with EtOAc. The organic phases were combined, dried over anhydrous sodium sulfate, filtered and subjected to rotary evaporation under reduced pressure to remove the solvent, and then the residue continued to be subjected to rotary evaporation to dryness under reduced pressure at 45 °C and 0.6 mbar for 30 min to obtain 1.33 g of crude product 96. The obtained crude product was used directly for the next step without further purification. MS (ESI) m/z 251.1 ([M+H]$^+$).

[Step C] Compound 4-(4-(tert-butoxycarbonyl)piperazin-1-yl)benzoic acid (4.70 g, 15.33 mmol, 1.05 equiv.), the crude product 96 (3.66 g, about 14.60 mmol, 1.00 equiv.), HATU (8.46 g, 21.90 mmol, 1.5 equiv.) and DIPEA (diisopropylethylamine) (7.59 mL, 43.80 mmol, 3.0 equiv.) were dissolved in anhydrous DMF (150 mL). The reaction system was stirred overnight at room temperature under argon protection. No remaining materials were detected by TLC. The reaction was quenched with water. The water phase was extracted with EtOAc. The organic phases were combined, washed with saturated brine and dried over anhydrous sodium sulfate. The dried organic phase was subjected to suction filtration and underwent rotary evaporation under reduced pressure to remove the solvent. The resulting solid was purified by chromatography to obtain 6.42 g of compound 97, with a yield of 82%. MS (ESI) m/z 439.2 ([M+H-Boc]$^+$).

[Step D] At 0°C, TFA (trifluoroacetic acid) (0.98 mL, 14.84 mmol, 4.0 equiv.) was slowly added to a solution of compound 97 (2.00 g, 3.71 mmol, 1.0 equiv.) in dry DCM (1,2-dichloroethane) (50 mL). The reaction system was stirred for 3 h at 0°C. No remaining materials were detected by TLC. The reaction solution was diluted with DCM (1,2-dichloroethane). The diluted reaction solution was subjected to rotary evaporation under reduced pressure to remove the solvent. The residue was dissolved in methanol and diluted with water, and then a saturated sodium bicarbonate solution was added to adjust the pH to 8. The water phase was extracted with EtOAc. The organic phases were combined and dried over anhydrous sodium sulfate. The dried organic phase was filtered and subjected to rotary evaporation under reduced pressure to remove the solvent. Then, the reaction system continued to be subjected to rotary evaporation to dryness for 30 min at 45°C and 0.6 mbar to obtain 1.68 g of crude product 98. The obtained crude product was used directly for the next step without further purification. $^1$H NMR (400 MHz, DMSO-$d_6$) δ 7.83 (d, J = 8.8 Hz, 1H), 7.71 (d, J = 8.9 Hz, 1H), 7.68 - 7.61 (m, 2H), 7.16 (d, J = 2.2 Hz, 1H), 7.09 - 7.02 (m, 2H), 6.95 (dd, J = 8.8, 2.2 Hz, 1H), 3.93 (tt, J = 6.2, 3.6 Hz, 1H), 3.75 - 3.64 (m, 1H), 3.35 - 3.21 (m, 4H), 3.10 - 2.95 (m, 4H), 2.39 (p, J = 3.2 Hz, 1H), 2.02 - 1.88 (m, 2H), 1.87 - 1.63 (m, 6H) ppm. MS(ESI) m/z 439.2 ([M+H]$^+$).

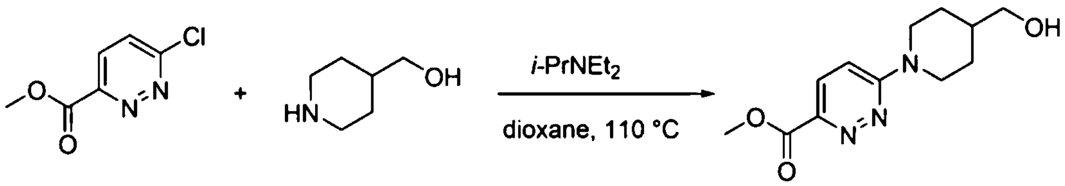


99

Synthetic route 26 Synthesis of compound 99

At 0°C and under an argon atmosphere, ceric ammonium nitrate (CAN) (766 mg, 1.40 mmol, 10.0 equiv.) was added in batch to a solution of compound 90-LB14-PMB (80 mg, 0.14 mmol, 1.0 equiv.) in MeCN-$H_2O$ (10 mL, 10:1, v/v). The reaction mixed solution was stirred for 15 min at 0°C and then stirred for 2 h at room temperature. No remaining materials were detected by TLC. A saturated aqueous sodium bicarbonate solution was added. The water phase was extracted with a DCM-MeOH (5:1, v/v) mixed solvent. The organic phases were combined and dried over anhydrous sodium sulfate. The dried organic phase was subjected to suction filtration and underwent rotary evaporation under reduced pressure to remove the solvent. The resulting solid as purified by a TLC preparative plate to obtain 45 mg of compound 90-LB14, with a yield of 72%. MS (ESI) m/z 339.2 ([M+H-Boc]$^+$).

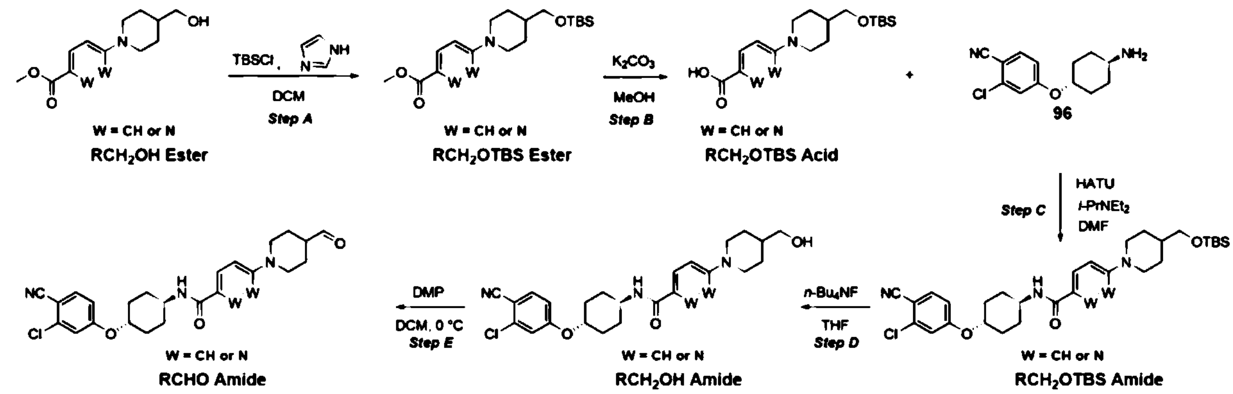


Synthetic Route 27 Synthesis of Compound RCH$_2$OTBS Acid and RCH$_2$OTBS Amide

[Step A] A material 99 represented by RCH$_2$OH ester (1.0 equiv.) or methyl 4-(4-(hydroxymethyl)piperidin-1-yl) benzoate was dissolved in DCM (1,2-dichloroethane) (0.2M). At 0°C, imidazole (2.0 equiv.) was added to the above solution. The resulting solution was stirred for 15 min at the same temperature, and then TBSCl (tert-butyldimethylchlorosilane) (1.2 equiv.) was added in batch. The resulting reaction mixture was automatically warmed to room temperature and continued to be stirred for 2 h. No remaining materials were detected by TLC. The reaction was quenched with a saturated aqueous sodium bicarbonate solution. The water phase was extracted with DCM (1,2-dichloroethane). The organic phases were combined, washed with saturated brine and dried with anhydrous sodium sulfate. The dried organic phase was filtered and subjected to rotary evaporation under reduced pressure to remove the solvent. The resulting solid was purified by silica gel flash column chromatography to obtain a compound represented by RCH$_2$OTBS Ester.

[Step B] The compound represented by RCH$_2$OTBS Ester (1.0 equiv.) was dissolved in anhydrous MeOH (0.2 M). At 0°C, Finely ground $K_2CO_3$ (3.0 equiv.) was added in batch to the above solution. The resulting reaction mixture was automatically warmed to room temperature and continued to be stirred for 4 h. No remaining materials were detected by TLC. The reaction mixture was diluted with water, and then a 3N aqueous HCl solution was used to adjust the pH of the diluted reaction mixture to 5-7. The water phase was extracted with EtOAc. The organic phases were combined and dried over anhydrous sodium sulfate. The dried organic phase was filtered and subjected to rotary evaporation under reduced pressure to remove the solvent, and then continued to be subjected to rotary evaporation to dryness for 30 min under reduced pressure at 45°C and 0.6 mbar to obtain a series of crude products represented by RCH$_2$OTBS Acid. The crude products were used directly for the next step without further purification.

[Step C] The compound represented by RCH$_2$OTBS Acid (1.0 equiv.), the compound 96 (1.05 equiv.), HATU (1.5 equiv.) and DIPEA (diisopropylethylamine) (3.0 equiv.) were dissolved in anhydrous DMF (0.2 M). The reaction system was stirred at room temperature overnight under argon protection. When the complete consumption of one of the materials was detected by TLC, the reaction was quenched with water. The water phase was extracted with EtOAc. The organic phases were combined and dried with anhydrous sodium sulfate. The dried organic phase was subjected to suction filtration and underwent rotary evaporation under reduced pressure to remove the solvent. The resulting solid was purified by silica gel flash column chromatography to obtain a compound represented by RCH$_2$OTBS Amide.

[Step D] The compound represented by RCH$_2$OTBS Amide (1.0 equiv.) was dissolved in anhydrous THF (0.2 M), and then TBAF (tetrabutylammonium fluoride) (1.0 M in THF, 1.5 equiv.) was added to the above solution. The reaction system was stirred for 3 h at room temperature under argon protection. When the complete consumption of the materials was detected by TLC, the reaction system was diluted with water. The water phase was extracted with EtOAc. The organic phases were combined and dried with anhydrous sodium sulfate. The dried organic phase was subjected to suction filtration and rotary evaporation under reduced pressure to remove the solvent. The resulting solid was purified by silica gel flash column chromatography to obtain the compound represented by RCH$_2$OH Amide.

[Step E] The compound represented by RCH$_2$OH Amide (1.0 equiv.) was dissolved in anhydrous DCM (1,2-dichloroethane) (0.1 M), and then DMP (2.0 equiv.) was added in batch to the above solution at 0°C. The reaction system was stirred for 1 h at 0°C under argon protection. When the complete consumption of the materials was detected by TLC, the reaction system was diluted with water. The reaction was quenched with a diluted sodium thiosulfate solution and a saturated sodium bicarbonate solution. The water phase was extracted with DCM (1,2-dichloroethane). The organic phases were combined and dried with anhydrous sodium sulfate. The dried organic phase was subjected to suction filtration and underwent rotary evaporation under reduced pressure to remove the solvent. The resulting solid was purified by flash silica gel column chromatography to obtain the compound represented by RCHO Amide.

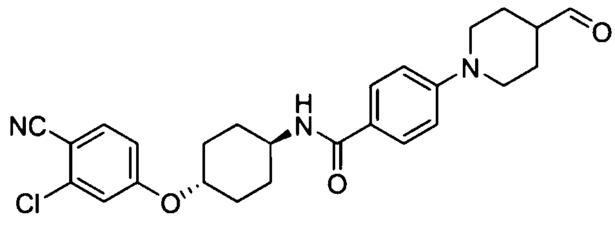

**100**

According to the above general synthetic strategy, 86 mg of target product compound 100 was synthesized and purified. $^1$H NMR (400 MHz, DMSO-$d_6$) δ 9.54 (d, J = 7.5 Hz, 1H), 7.83 (d, J = 8.8 Hz, 1H), 7.71 (d, J = 8.9 Hz, 1H), 7.68 - 7.61 (m, 2H), 7.16 (d, J = 2.2 Hz, 1H), 7.09 - 7.02 (m, 2H), 6.95 (dd, J = 8.8, 2.2 Hz, 1H), 3.93 (tt, J = 6.2, 3.6 Hz, 1H), 3.75 - 3.64 (m, 1H), 3.45 (ddd, J = 12.5, 8.4, 5.8 Hz, 2H), 3.34 (ddd, J = 12.5, 8.3, 5.8 Hz, 2H), 2.47 (dp, J = 7.7, 5.5 Hz, 1H), 2.02 - 1.63 (m, 12H) ppm. MS (ESI) m/z 466.2 ([M+H]$^+$).

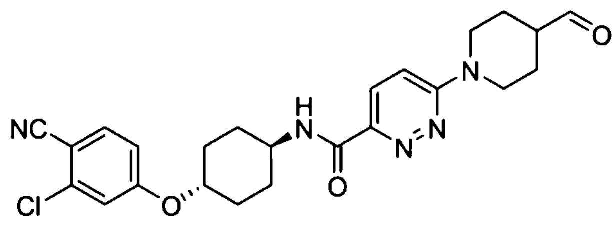

**101**

According to the above general synthetic strategy, 217 mg of target product compound 101 was synthesized and purified. $^1$H NMR (400 MHz, DMSO-$d_6$) δ 9.54 (d, J = 7.5 Hz, 1H), 7.93 - 7.82 (m, 2H), 7.71 (d, J = 8.9 Hz, 1H), 7.22 - 7.14 (m, 2H), 6.95 (dd, J = 8.8, 2.2 Hz, 1H), 3.98 - 3.86 (m, 3H), 3.83 - 3.72 (m, 1H), 3.60 (ddd, J = 12.3, 8.8, 6.1 Hz, 2H), 2.47 (dp, J = 7.7, 5.3 Hz, 1H), 2.02 - 1.73 (m, 9H), 1.77 - 1.62 (m, 5H) ppm. MS (ESI) m/z 468.2 ([M+H]$^+$).

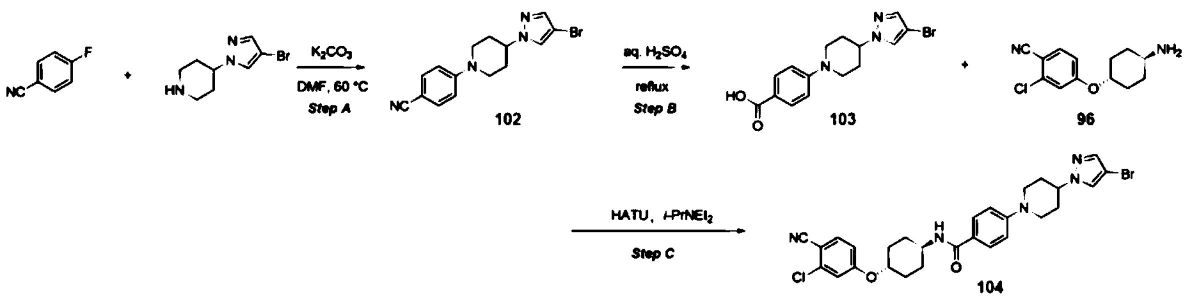


Synthetic route 28 Synthesis of compounds 102, 103 and 104

[Step A] 4-fluorobenzonitrile (1.58 g, 13.04 mmol, 1.5 equiv.), 4-(4-bromo-1H-pyrazol-1-yl)piperidine (2.00 g, 8.69 mmol, 1.0 equiv.) and finely ground $K_2CO_3$ (2.40 g, 17.38 mmol, 2.0 equiv.) were added to dry DMF (100 mL). The resulting reaction mixture was stirred at 60°C under argon atmosphere overnight. No remaining materials were detected by TLC. The reaction system was diluted with water. The water phase was extracted with DCM (1,2-dichloroethane). The organic phases were combined, washed with saturated brine and dried over anhydrous sodium sulfate. The dried organic phase was filtered and subjected to rotary evaporation under reduced pressure to remove the solvent. The resulting solid was purified by column chromatography to obtain 2.36 g of compound 102, with a yield of 82%. MS(ESI) m/z 331.1 ([M+H]$^+$).

[Step B] The compound 102 (2.20 g, 6.64 mmol, 1.0 equiv.) was added to a 50% sulfuric acid aqueous solution (1 M). The reaction system was heated to reflux for 1 h under an argon atmosphere. No remaining materials were detected by TLC. The reaction system was cooled, and then slowly poured into a saturated aqueous sodium bicarbonate-ice mixture. A 4N HCl aqueous solution was used to carefully adjust the pH to 4-6. The water phase was extracted with EtOAc. The organic phases were combined and dried over anhydrous sodium sulfate. The dried organic phase was filtered and subjected to rotary evaporation under reduced pressure to remove the solvent and then continued to be subjected to rotary

evaporation under reduced pressure for 30 min to dryness at 45°C and 0.6 mbar to obtain 2.21 g of crude product compound 103. MS (ESI) m/z 348.0 ([M-H]-).

[Step C] The compound 103 (500 mg, ~1.43 mmol, 1.0 equiv.), the compound 96 (376 mg, ~1.50 mmol, 1.05 equiv.), HATU (830 mg, 2.14 mmol, 1.5 equiv.) and DIPEA (diisopropylethylamine) (0.75 mL, 4.29 mmol, 3.0 equiv.) were dissolved in anhydrous DMF (20 mL). The reaction system was stirred at room temperature overnight under argon protection. No remaining materials were detected by TLC. The reaction was quenched with water. The water phase was extracted with EtOAc. The organic phases were combined, washed with saturated brine and dried over anhydrous sodium sulfate. The dried organic phase was subjected to suction filtration and underwent rotary evaporation under reduced pressure to remove the solvent. The resulting solid was purified by chromatography to obtain 718 mg of compound 104, with a yield of 86%. MS (ESI) m/z 582.1 ([M+H]+).

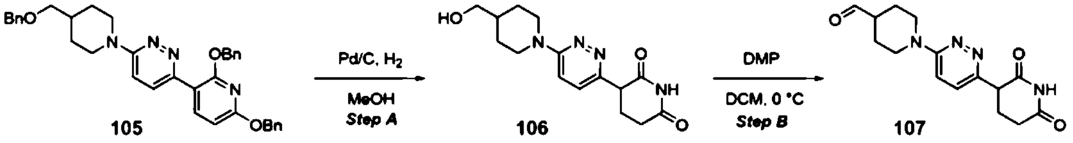

105 106 107

Synthetic route 29 Synthesis of compounds 105, 106 and 107

[Step A] 238 mg of compound 105 was synthesized by using a starting material 4-((benzyloxy)methyl)piperidine according to the synthetic method of compound 55b. The compound 105 (200 mg, 0.35 mmol, 1.0 equiv.) was dissolved in anhydrous MeOH (10 mL), and then Pd/C (30 mg, 10 wt% on Carbon) was added. Then, the reaction system was vigorously stirred for 3 h at room temperature under a hydrogen atmosphere (balloon). No remaining materials were detected by TLC. The reaction mixture was subjected to suction filtration and underwent rotary evaporation under reduced pressure to remove the solvent. The resulting solid was purified by chromatography to obtain 65 mg of compound 106, with a yield of 61%. MS (ESI) m/z 305.2 ([M+H]+).

[Step B] At 0°C, DMP (116 mg, 0.27 mmol, 1.3 equiv.) was added in batch to a solution of compound 106 (65 mg, 0.21 mmol, 1.0 equiv.) in anhydrous DCM (1,2-dichloroethane) (5 mL). The resulting reaction system was stirred for 1 h at 0°C under argon protection. When the complete consumption of the materials was detected by TLC, the reaction system was diluted with water. The reaction was quenched with a diluted sodium thiosulfate solution and a saturated sodium bicarbonate solution. The water phase was extracted with DCM (1,2-dichloroethane). The organic phases were combined and dried with anhydrous sodium sulfate. The dried organic phase was subjected to suction filtration and underwent rotary evaporation under reduced pressure to remove the solvent. The resulting solid was purified by chromatography to obtain 59 mg of compound 107, with a yield of 91%. $^1H$ NMR (400 MHz, DMSO-$d_6$) δ 11.07 (s, 1H), 9.54 (d, J = 7.5 Hz, 1H), 7.51 - 7.44 (m, 1H), 6.90 (d, J = 8.6 Hz, 1H), 3.92 (ddd, J = 12.3, 8.8, 6.0 Hz, 2H), 3.68 (td, J = 6.2, 0.7 Hz, 1H), 3.60 (ddd, J = 12.3, 8.8, 6.1 Hz, 2H), 2.59 - 2.48 (m, 2H), 2.47 (dt, J = 7.6, 5.5 Hz, 1H), 2.22 - 2.04 (m, 2H), 1.96 - 1.83 (m, 2H), 1.83 - 1.70 (m, 2H) ppm. MS (ESI) m/z 303.1 ([M+H]+).

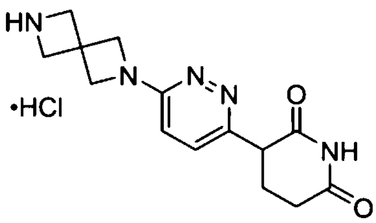

**108**

92 mg of compound 108 was synthesized by using a starting material tert-butyl 2,6-diazaspiro[3.3]heptane-2-carboxylate according to the synthetic method of compound 7. $^1H$ NMR (400 MHz, DMSO-$d_6$) δ 11.07 (s, 1H), 9.23 - 9.13 (m, 1H), 9.02 - 8.92 (m, 1H), 7.51 - 7.44 (m, 1H), 6.89 (d, J = 8.6 Hz, 1H), 3.92 (s, 4H), 3.68 (td, J = 6.2, 0.7 Hz, 1H), 3.23 (d, J = 6.8 Hz, 2H), 2.61 - 2.48 (m, 2H), 2.22 - 2.04 (m, 2H) ppm. MS (ESI) m/z 288.1 ([M+H]+).

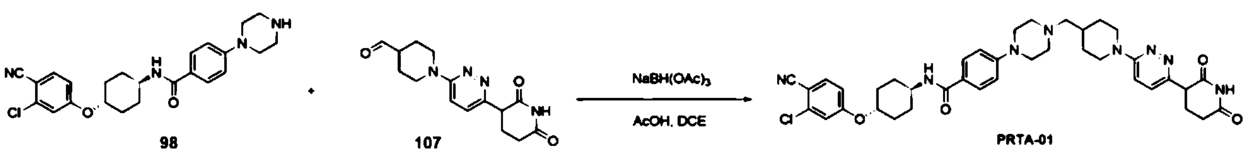

98 + 107 PRTA-01

Synthetic route 30 Synthesis of compound PRTA-01

The compound 107 (22 mg, 0.07 mmol, 1.0 equiv.) and the compound 98 (38 mg, 0.09 mmol, 1.2 equiv.) were dissolved in anhydrous DCE (1,2-dichloroethane) (3 mL) containing 10 v% AcOH (0.30 mL) at room temperature. After the mixture was stirred for 15 min, $NaBH(OAc)_3$ (18 mg, 0.09 mmol, 1.2 equiv.) was added. Then, the above mixture continued to be stirred for 3 h. When the complete consumption of the materials was detected by TLC, the reaction system was diluted with water. The reaction was quenched with a diluted sodium thiosulfate solution and a saturated sodium bicarbonate solution. The water phase was extracted with DCM (1,2-dichloroethane). The organic phases were combined and dried with anhydrous sodium sulfate. The dried organic phase was subjected to suction filtration and underwent rotary evaporation under reduced pressure to remove the solvent. The resulting solid was purified by preparative HPLC to obtain 36 mg of compound PRTA-01, with a yield of 68%. $^1H$ NMR (400 MHz, DMSO-$d_6$) δ 11.07 (s, 1H), 7.83 (d, J = 8.8 Hz, 1H), 7.71 (d, J = 8.9 Hz, 1H), 7.68 - 7.62 (m, 2H), 7.51 - 7.44 (m, 1H), 7.16 (d, J = 2.2 Hz, 1H), 7.09 - 7.02 (m, 2H), 6.98 - 6.86 (m, 2H), 4.02 - 3.89 (m, 3H), 3.82 (ddd, J = 12.4, 8.1, 6.9 Hz, 2H), 3.75 - 3.65 (m, 2H), 3.21 (ddd, J = 7.5, 5.7, 3.0 Hz, 5H), 2.65 - 2.45 (m, 9H), 2.22 - 2.04 (m, 2H), 2.02 - 1.63 (m, 16H) ppm. UPLC-MS(ESI) m/z 725.3, $t_R$ 0.831 min, 97% purity.

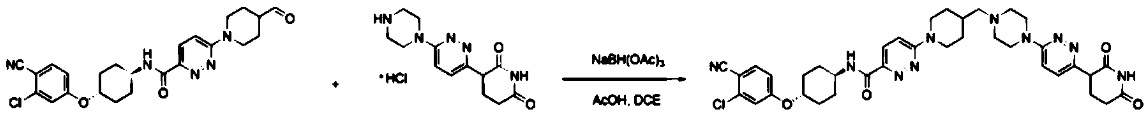


101 7 PRTA-02

Synthetic route 31 Synthesis of Compound PRTA-02

The compound 7 (35 mg, 0.11 mmol, 1.0 equiv.) and the compound 101 (63 mg, 0.13 mmol, 1.2 equiv.) were dissolved in anhydrous DCE (3 mL) containing 10 v% AcOH (0.30 mL) at room temperature. After the mixture was stirred for 15 min, $NaBH(OAc)_3$ (31 mg, 0.15 mmol, 1.3 equiv.) was added. Then, the above mixture continued to be stirred for 2 h. When the complete consumption of the materials was detected by TLC, the reaction system was diluted with water. The reaction was quenched with a diluted sodium thiosulfate solution and a saturated sodium bicarbonate solution. The water phase was extracted with DCM (1,2-dichloroethane). The organic phases were combined and dried with anhydrous sodium sulfate. The dried organic phase was subjected to suction filtration and underwent rotary evaporation under reduced pressure to remove the solvent. The resulting solid was purified by preparative HPLC to obtain 51 mg of compound PRTA-02, with a yield of 62%. $^1H$ NMR (400 MHz, DMSO-$d_6$) δ 11.07 (s, 1H), 7.93 - 7.82 (m, 2H), 7.71 (d, J = 8.8 Hz, 1H), 7.47 (dd, J = 8.5, 0.6 Hz, 1H), 7.23 - 7.14 (m, 2H), 6.98 - 6.88 (m, 2H), 4.02 - 3.92 (m, 2H), 3.96 - 3.89 (m, 1H), 3.88 - 3.77 (m, 2H), 3.81 - 3.72 (m, 1H), 3.68 (td, J = 6.2, 0.6 Hz, 1H), 3.65 - 3.51 (m, 4H), 2.73 - 2.64 (m, 2H), 2.67 - 2.61 (m, 2H), 2.64 - 2.57 (m, 1H), 2.53 (ddd, J = 8.2, 7.4, 0.9 Hz, 2H), 2.49 (dd, J = 11.5, 4.9 Hz, 1H), 2.22 - 2.04 (m, 2H), 2.02 - 1.62 (m, 13H) ppm. UPLC-MS(ESI) m/z 727.3, $t_R$ 0.685 min, 98% purity.

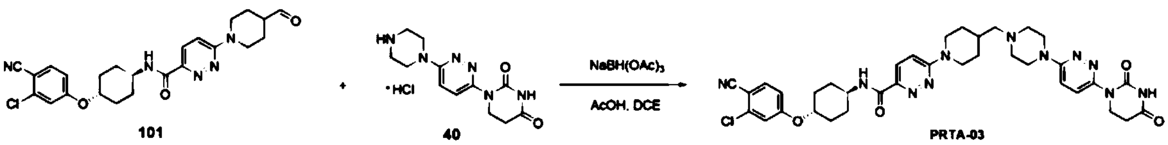


101 40 PRTA-03

Synthetic route 32 Synthesis of compound PRTA-03

44 mg of target product protein degrader PRTA-03 was obtained and purified by using compounds 101 and 40 according to the synthetic method of PRTA-02 as described above. $^1H$ NMR (400 MHz, DMSO-$d_6$) δ 10.64 (s, 1H), 7.93 - 7.82 (m, 2H), 7.71 (d, J = 8.8 Hz, 1H), 7.23 - 7.14 (m, 2H), 7.06 (d, J = 8.0 Hz, 1H), 7.00 (d, J = 8.0 Hz, 1H), 6.95 (dd, J = 8.8, 2.2 Hz, 1H), 4.02 - 3.95 (m, 1H), 3.99 - 3.89 (m, 4H), 3.84 (d, J = 7.8 Hz, 1H), 3.84 - 3.78 (m, 1H), 3.82 - 3.72 (m, 1H), 3.66 - 3.51 (m, 4H), 2.75 - 2.57 (m, 7H), 2.49 (dd, J = 11.4, 4.9 Hz, 1H), 2.02 - 1.62 (m, 13H) ppm. UPLC-MS (ESI) m/z 728.3, $t_R$ 0.602 min, 97% purity.

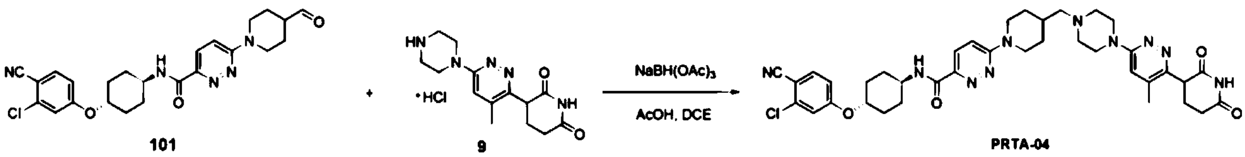


101 9 PRTA-04

Synthetic route 33 Synthesis of compound PRTA-04

53 mg of target product protein degrader PRTA-04 was obtained and purified by using compounds 101 and 9 according to the synthesis method of PRTA-02 as described above. $^1$H NMR (400 MHz, DMSO-$d_6$) δ 11.13 (s, 1H), 7.93 - 7.82 (m, 2H), 7.71 (d, J = 8.8 Hz, 1H), 7.23 - 7.14 (m, 2H), 6.95 (dd, J = 8.8, 2.3 Hz, 1H), 6.67 (d, J = 0.6 Hz, 1H), 4.11 (t, J = 6.1 Hz, 1H), 4.02 - 3.92 (m, 2H), 3.96 - 3.89 (m, 1H), 3.88 - 3.77 (m, 2H), 3.81 - 3.71 (m, 1H), 3.67 - 3.57 (m, 2H), 3.61 - 3.52 (m, 2H), 2.73 - 2.45 (m, 8H), 2.23 - 2.04 (m, 2H), 2.02 - 1.62 (m, 14H) ppm. UPLC-MS (ESI) m/z 741.3, $t_R$ 0.698 min, 96% purity.

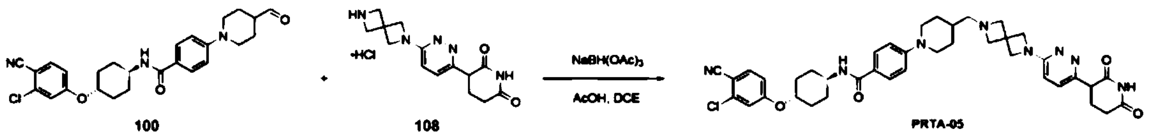

100 + 108 → PRTA-05

Synthetic route 34 Synthesis of compound PRTA-05

34 mg of target product protein degrader PRTA-05 was obtained and purified by using compounds 101 and 108 according to the synthesis method of PRTA-01 as described above. $^1$H NMR (400 MHz, DMSO-$d_6$) δ 11.07 (s, 1H), 7.83 (d, J = 8.8 Hz, 1H), 7.71 (d, J = 8.9 Hz, 1H), 7.68 - 7.61 (m, 2H), 7.51 - 7.44 (m, 1H), 7.16 (d, J = 2.2 Hz, 1H), 7.09 - 7.02 (m, 2H), 6.95 (dd, J = 8.8, 2.2 Hz, 1H), 6.89 (d, J = 8.6 Hz, 1H), 3.98 - 3.90 (m, 1H), 3.93 (s, 2H), 3.87 (s, 2H), 3.75 - 3.64 (m, 2H), 3.42 (ddd, J = 12.3, 8.3, 6.1 Hz, 2H), 3.09 (ddd, J = 12.3, 8.2, 6.2 Hz, 2H), 2.60 (dd, J = 11.2, 3.7 Hz, 1H), 2.59 - 2.48 (m, 3H), 2.22 - 2.04 (m, 2H), 2.02 - 1.88 (m, 2H), 1.88 - 1.63 (m, 11H) ppm. UPLC-MS(ESI) m/z 737.3, $t_R$ 0.672 min, 98% purity.

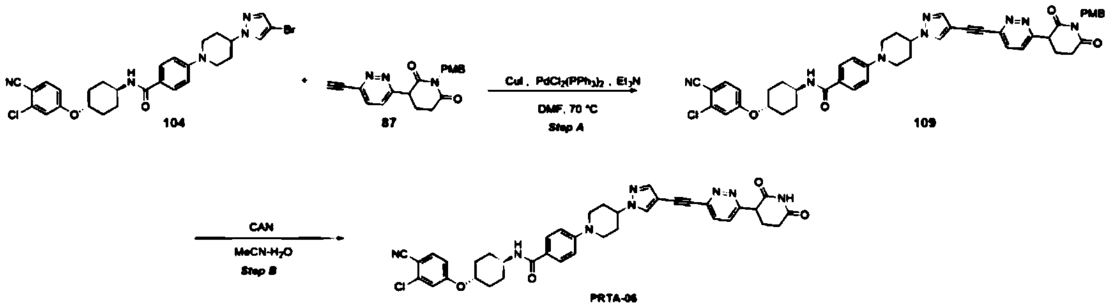

104 + 87 → 109 → PRTA-06

Synthetic route 35 Synthesis of compound PRTA-06

[Step A] $Pd(PPh_3)_2Cl_2$ (8 mg, 0.01 mmol, 0.1 equiv.) and CuI (2 mg, 0.13 mmol, 0.12 equiv.) were placed in a round-bottom flask, and then the round-bottom flask was purged and backfilled with argon for three times. Subsequently, dry DMF (1.0 mL), the compound 104 (63 mg, 0.11 mmol, 1.0 equiv.), the compound 87 (54 mg, 0.16 mmol, 1.5 equiv.) and $Et_3N$ (1.0 mL) were sequentially added. The round-bottom flask was purged and backfilled with argon again. Then, the reaction system was stirred overnight at 70°C under an argon atmosphere. No remaining materials were detected by TLC. The reaction system was diluted with water. The resulting water phase was extracted with DCM (1,2-dichloroethane). The organic phases were combined and dried with anhydrous sodium sulfate. The dried organic phase was subjected to suction filtration and underwent rotary evaporation under reduced pressure to remove the solvent. The resulting solid was purified by using a TLC preparative plate to obtain 62 mg of compound 109, with a yield of 68%. MS (ESI) m/z 837.3 ([M+H-Boc]$^+$).

[Step B] CAN (ceric ammonium nitrate) (192 mg, 0.35 mmol, 5.0 equiv.) was added in batch to a solution of compound 109 (62 mg, 0.07 mmol, 1.0 equiv.) in MeCN-$H_2O$ (5 mL, 10:1, v/v) under an argon atmosphere at 0°C. The reaction mixed solution was stirred for 10 min at 0°C and then stirred for 1 h at room temperature. No remaining materials were detected by TLC. The reaction system was washed with a saturated aqueous sodium bicarbonate solution. The water phase was extracted with a DCM-MeOH (5:1, v/v) mixed solvent. The organic phases were combined and dried over anhydrous sodium sulfate. The dried organic phase was subjected to suction filtration and underwent rotary evaporation under reduced pressure to remove the solvent. Then, 21 mg of compound PRTA-06 was obtained by preparative HPLC, with a yield of 40%. $^1$H NMR (400 MHz, DMSO-$d_6$) δ 11.07 (s, 1H), 7.87 (d, J = 1.8 Hz, 1H), 7.83 (d, J = 8.8 Hz, 1H), 7.74 - 7.65 (m, 3H), 7.68 - 7.61 (m, 3H), 7.16 (d, J = 2.2 Hz, 1H), 7.09 - 7.02 (m, 2H), 6.95 (dd, J = 8.8, 2.2 Hz, 1H), 4.47 (pd, J = 3.1, 0.7 Hz, 1H), 3.93 (tt, J = 6.2, 3.6 Hz, 1H), 3.75 - 3.56 (m, 6H), 2.59 - 2.48 (m, 2H), 2.22 - 1.63 (m, 15H) ppm. UPLC-MS (ESI) m/z 717.3, $t_R$ 0.713 min, 98% purity.

Example 5 Binding activity of compound to E3 ubiquitin ligase CRBN

The binding activity of the compound disclosed in the present patent to the E3 ubiquitin ligase CRBN was determined by competitive binding of the disclosed compound with XL665-labeled thalidomide to the CRBN protein, thereby preventing the occurrence of fluorescence resonance energy transfer (FRET). Accordingly, the binding affinity of the compound to the CRBN protein was determined.

CRBN binding activity test kit (CEREBLON BINDING KITS (PE, 64BDCRBNPEG)).

The assay was based on a homogeneous time-resolved fluorescence (HTRF) technology. When a donor and an acceptor were in close proximity, the donor transferred energy to the acceptor so that the acceptor was excited to emit an emission light 665 nm.

The donor used in the kit of the present disclosure was a europium-labeled GST antibody (GST Eu Cryptate Antibody), and the acceptor was thalidomide labeled with XL665 (Thalidomide-Red reagent). The donor bound to the CRBN protein comprising a GST label. The compound disclosed in the present patent competitively bound to the CRBN protein with thalidomide. The binding activity of the compound was determined according to the fluorescence value emitted by the acceptor at 665 nm. The stronger binding force of the compound indicated the weaker signal.

The donor and the acceptor were diluted 50-fold respectively by using a kit buffer (PROTAC binding buffer 1). The CRBN protein was diluted 45-fold. The 8 mM solution of the compound disclosed in the present patent was diluted 10-fold with 1X diluent from the kit, followed by 5-fold serial dilutions for a total of 7 consecutive dilutions.

5 μl of compound, 5 μl of diluted CRBN protein and 10 μl of donor-acceptor isovolumetric mixture were sequentially added to each well of a white 384-well plate (PE, Part number:6008280). The above system was incubated for 3 h at room temperature.

A ratio of acceptor to donor emission signals for each individual well was calculated. Ratio = signal value at 665 nm/signal value at 620 nm * $10^4$

$$\text{Coefficient of variation (CV)} = \text{standard deviation (SD)/average Ratio} \times 100$$

The $IC_{50}$ value was calculated based on the compound concentration, Ratio and the coefficient of variation by using GraphPad Prism.

Table 10 Binding activity of compounds 1-18 to E3 ubiquitin ligase CRBN

| ID | Structure | $IC_{50}$ (μM) | ID | Structure | $IC_{50}$ (μM) |
|---|---|---|---|---|---|
| 1 | 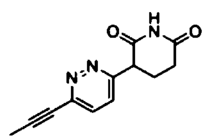 | 50 | 10 | 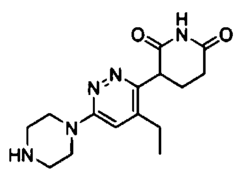 •HCl | >50<br>100 |
| 2 | 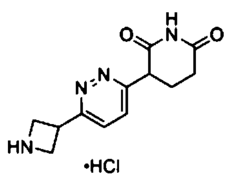 •HCl | 50 | 11 | 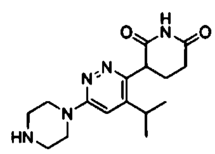 •HCl | >50<br>100 |
| 3 | 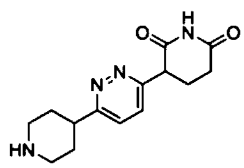 •HCl | 50 | 12 | 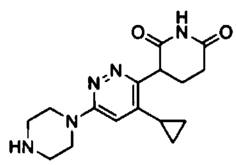 •HCl | >50<br>100 |
| 4 | 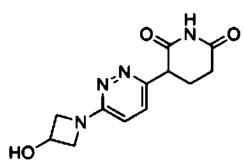 | 50 | 13 | 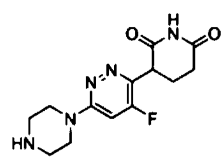 •HCl | 50 |

(continued)

| ID | Structure | $IC_{50}$ (μM) | ID | Structure | $IC_{50}$ (μM) |
|---|---|---|---|---|---|
| 5 | 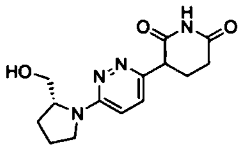 | 50 | 14 | 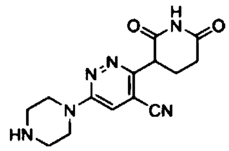 •HCl | 50 |
| 6 | 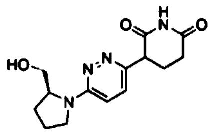 | 50 | 15 | 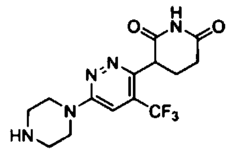 •HCl | 50 |
| 7 | 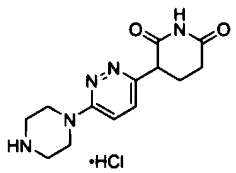 •HCl | 50 | 16 | 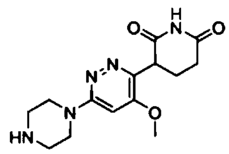 •HCl | 50 |
| 8 | 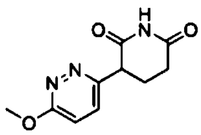 | 50 | 17 | 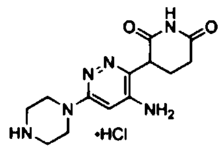 •HCl | 50 |
| 9 | 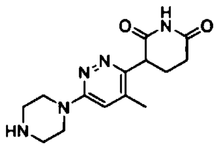 •HCl | 50 | 18 | 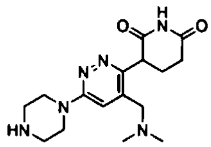 •2HCl | >50<br>100 |

Table 11 Binding activity of compounds 19-34 to E3 ubiquitin ligase CRBN

| ID | Structure | $IC_{50}$ (μM) | ID | Structure | $IC_{50}$ (μM) |
|---|---|---|---|---|---|
| 19 | 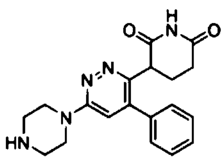 •HCl | >100 | 27 | 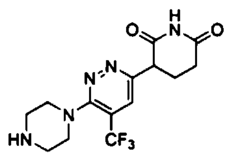 •HCl | 50 |
| 20 | 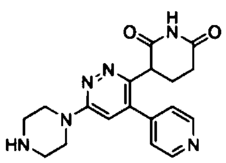 •HCl | >100 | 28 | 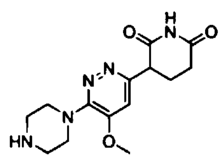 •HCl | 50 |
| 21 | 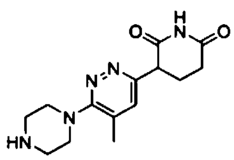 •HCl | 50 | 29 | 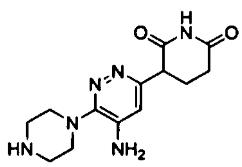 •HCl | 50 |

(continued)

| ID | Structure | $IC_{50}$ (μM) | ID | Structure | $IC_{50}$ (μM) |
|---|---|---|---|---|---|
| 22 | 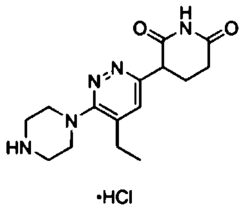 | >50 | 30 | 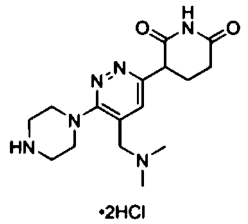 | >50<br>100 |
| 23 | 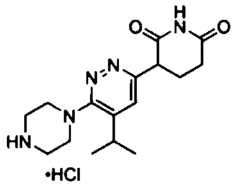 | 100 | 31 | 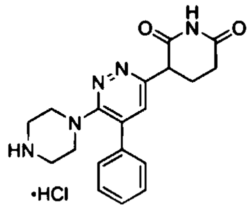 | >50<br>100 |
| 24 | 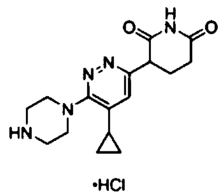 | >50 | 32 | 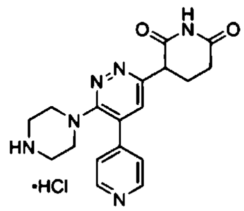 | >50<br>100 |
| 25 | 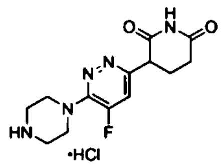 | 50 | 33 | 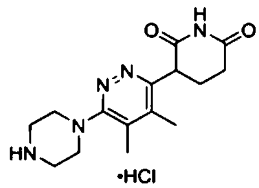 | 50 |
| 26 | 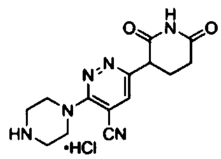 | 50 | 34 | 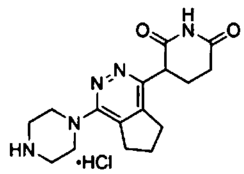 | >50<br>100 |

Table 12 Binding activity of compounds 35-43 to E3 ubiquitin ligase CRBN

| ID | Structure | $IC_{50}$ (μM) | ID | Structure | $IC_{50}$ (μM) |
|---|---|---|---|---|---|
| 35 | 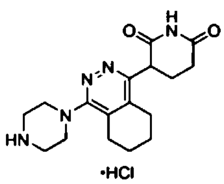 | >50<br>100 | 41 | 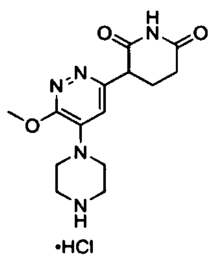 | 50 |

(continued)

| ID | Structure | $IC_{50}$ (μM) | ID | Structure | $IC_{50}$ (μM) |
|---|---|---|---|---|---|
| 36 | 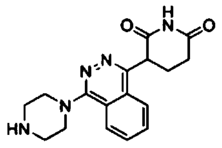 •HCl | >50<br>100 | 42 | 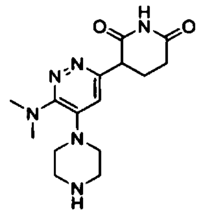 •HCl | 50 |
| 37 | 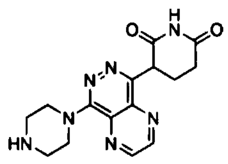 •HCl | >50<br>100 | 43 | 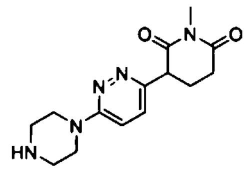 •HCl | >100 |
| 38 | 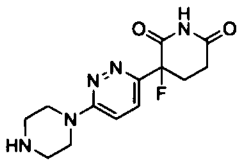 •HCl | 50 | | | |
| 39 | 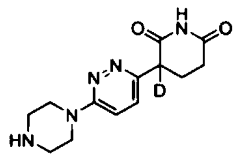 •HCl | 50 | | | |
| 40 | 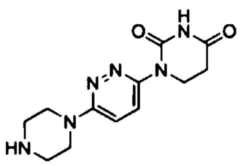 •HCl | 50 | | | |

Example 6 Evaluation of protein degrader compounds in reducing bromodomain protein (BRD) expression levels

The human chronic myelomonocytic leukemia MV-4-11 cells selected in this experiment were cultured in Iscove's Modified Dulbecco's Medium (IMDM) culture medium containing 10% fetal bovine serum (Gibco, USA) in an incubator under the conditions of at 37°C, 5% $CO_2$ and 95% humidity. When the growth density reached 80%, a culture medium containing the compound was selected, and the cells were inoculated into a 6-well plate with a cell density of $1\times10^6$/ml, so that the final concentrations of the compound were 0.1, 1, 10 and 20 μM. After 24 hours of treatment, the cells were collected, washed twice with PBS and lysed with an RIPA lysis buffer containing a protease inhibitor and a phosphatase inhibitor. The lysed mixed solution was centrifuged to obtain a total protein extract. The concentration of the protein in the extract was detected by using a BCA method.

Protein electrophoresis was performed via SDS-PAGE, followed by constant-current electrotransfer at 200 mA for 150 min to transfer proteins onto a polyvinylidene fluoride (PVDF) membrane. The PVDF membrane was placed in 5% skim milk and blocked for 1 h at room temperature. BRD4 (#13440, CST) (1:1000) and C-MYC (ab32072, 1:1000) were added for incubation overnight at 4°C. The membrane was washed with Tris buffered saline Tween (TBST) for 3 times, with 10 min each time. The mixture of the membrane and a secondary antibody were incubated for 60 min at room temperature. The membrane was washed with TBST for 3 times, with 10 min each time. The ECL light-emitting liquid was dropwise added for exposure. Each sample was simultaneously detected using GAPDH protein as an internal reference. The protein profile was analyzed for grayscale values using analysis software ImageJ. The gray scale correction value of each

sample was calculated by using the formula was used below: gray scale correction value = (target protein gray scale value/corresponding internal reference gray scale value) × $10^3$Then, the calculated gray scale correction value was compared with a control gray scale correction value to calculate a degradation rate. Then, nonlinear curve fitting was performed on a logarithmic concentration-inhibition rate using Prism to obtain the $DC_{50}$ and $D_{max}$ values of the compound.

Table 13 summarizes the evaluation of the reduction effects of protein degraders PRTB-01 to 16 on BRD protein expression levels. $DC_{50}$ represents the concentration of the protein degrader when it induces 50% of the BRD protein to be degraded, with the level classified as A ($DC_{50}$<1 μM), B ($DC_{50}$ 1 to 10 μM), or C ($DC_{50}$>10 μM). $D_{max}$ represents the proportion of the maximum degree of BRD protein degradation induced by the protein degrader to the total amount of BRD protein, with the level graded as A ($D_{max}$>85%), B ($D_{max}$85~50%), or C ($DC_{50}$<50%)

Table 13 Evaluation of protein degraders PRTB-01 to 16 in reducing BRD protein expression levels

| Compound | Structure | $DC_{50}$ | Dmax |
|---|---|---|---|
| **PRTB-01** | 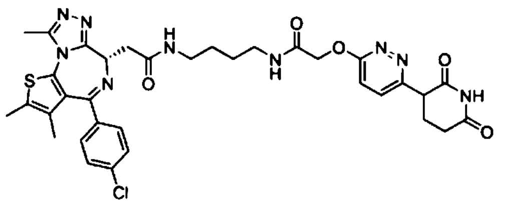 | B | C |
| **PRTB-02** | 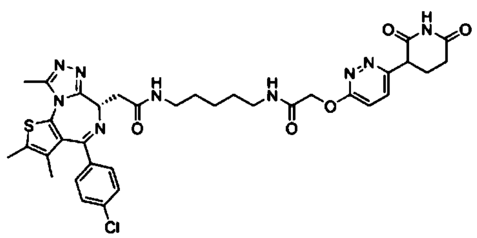 | A | A |
| **PRTB-03** | 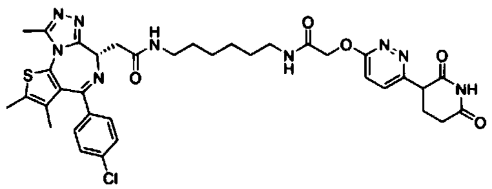 | B | C |
| **PRTB-04** | 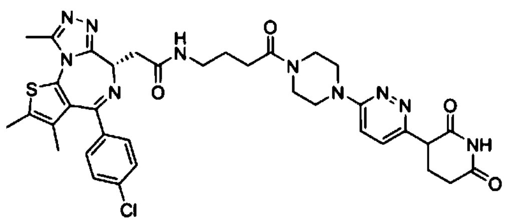 | B | C |
| **PRTB-05** | 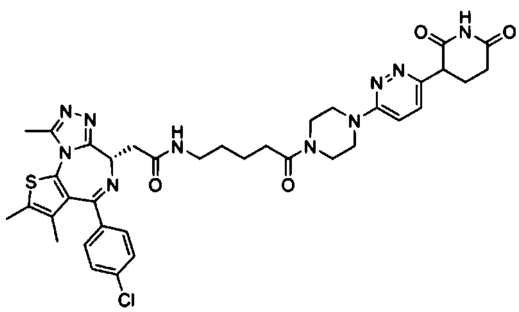 | B | C |

(continued)

| Compound | Structure | $DC_{50}$ | Dmax |
|---|---|---|---|
| PRTB-06 | 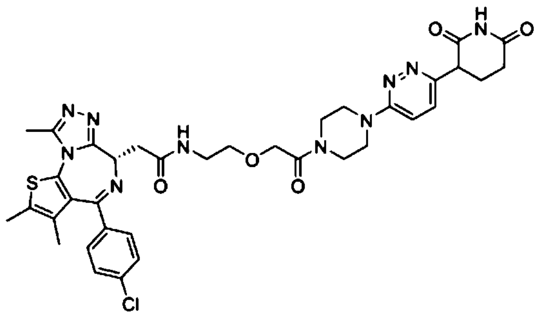 | B | B |
| PRTB-07 | 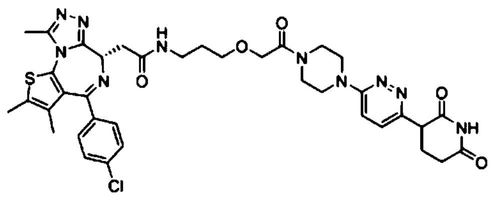 | C | C |
| PRTB-08 | 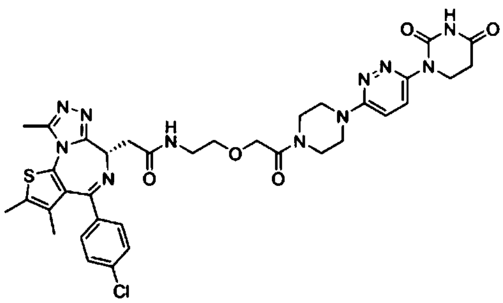 | B | B |
| PRTB-09 | 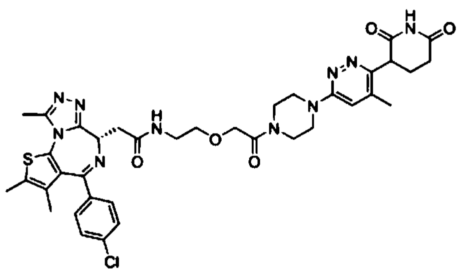 | B | B |
| PRTB-10 | 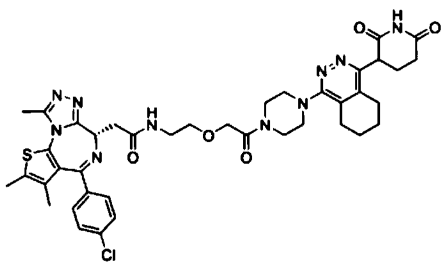 | C | C |
| PRTB-11 | 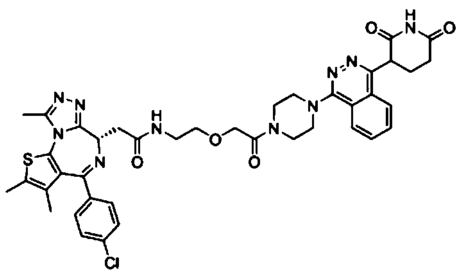 | C | C |

(continued)

| Compound | Structure | $DC_{50}$ | Dmax |
|---|---|---|---|
| PRTB-12 | 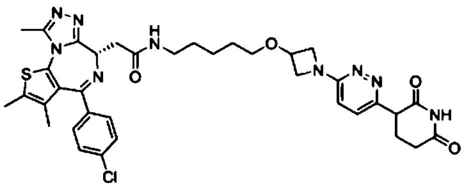 | A | A |
| PRTB-13 | 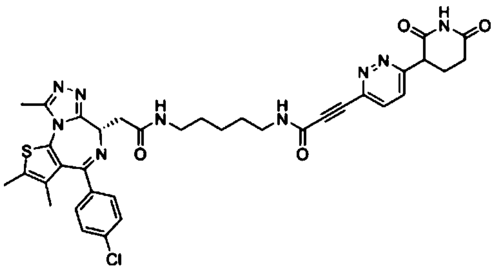 | A | A |
| PRTB-14 | 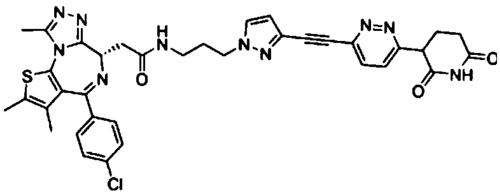 | A | B |
| PRTB-15 | 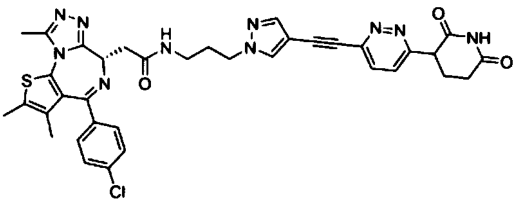 | A | A |
| PRTB-16 | 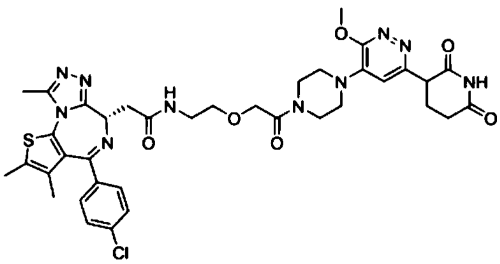 | C | C |

Example 7 Evaluation of protein degrader compounds in reducing androgen receptor (AR) expression levels

The human prostate cancer cell line LNCaP cells selected in this experiment were cultured in an RPMI-1640 culture medium containing 10% fetal bovine serum (Gibco, USA) in an incubator under the conditions of 37°C, 5% $CO_2$ and 95% humidity. When the growth density reached 80%, the cells were inoculated into a 6-well plate with $5\times10^5$ cells per well. After culturing for 24 h, the culture medium was replaced with a culture medium containing the compounds so that the final concentrations of the compounds were 0.01, 0.1, 1 and 10 μM. After 24 h of treatment, the supernatant was removed, the cells were washed twice with PBS, and a RIPA lysis buffer containing a protease inhibitor and a phosphatase inhibitor was added to the cells. After lysis and centrifugation, the total protein extract was obtained. The concentration of the protein in the extract was determined by using the BCA method.

Protein electrophoresis was performed by using SDS-PAGE, followed by electrotransfer at a constant current of 200 mA for 90 min to transfer proteins onto the PVDF membrane. The PVDF membrane was placed in 5% skim milk and blocked for 1 h at room temperature. An anti-Androgen Receptor antibody [EPR1535(2)] (ab133273)(1:10000) was added for incubation overnight at 4°C. The membrane was washed with TBST for 3 times, with 10 min each time. The mixture of the membrane and a secondary antibody were incubated for 60 min at room temperature. The membrane was washed

with TBST for 3 times, with 10 min each time. The ECL light-emitting liquid was dropwise added for exposure. Each sample was simultaneously detected using α-tubulin protein as an internal reference. The protein profile was analyzed for grayscale values using analysis software ImageJ. The gray scale correction value of each sample was calculated by using the formula: gray scale correction value = (gray scale value of the target protein/gray scale value of the corresponding internal reference) > $10^3$. Then, the calculated gray scale correction value was compared with a control gray scale correction value to calculate a degradation rate. Then, nonlinear curve fitting was performed on a logarithmic concentration-inhibition rate using Prism to obtain the $DC_{50}$ and $D_{max}$ values of the compound.

Table 14 summarizes the evaluation of protein degraders PRTA-01 to 06 in reducing AR protein expression levels. $DC_{50}$ represents the concentration of the protein degrader when it induces AR protein degradation to 50% of the initial level, and the level is classified as A ($DC_{50}$<1 μM), B ($DC_{50}$ 1~10 μM), or C ($DC_{50}$>10 μM). $D_{max}$ represents the proportion of the maximum degree of BRD protein degradation induced by the protein degrader to the total AR protein amount, with the level graded as A ($D_{max}$>85%), B ($D_{max}$85-50%), or C ($D_{max}$<50%).

Table 14 Evaluation of protein degraders PRTA-01 to 06 in reducing AR protein expression levels

| Compound | Structure | $DC_{50}$ | $D_{max}$ |
|---|---|---|---|
| **PRTA-01** | 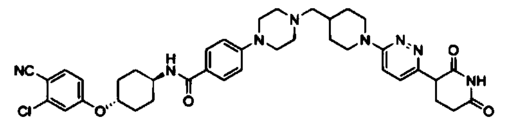 | A | B |
| **PRTA-02** | 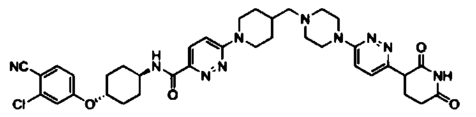 | A | A |
| **PRTA-03** | 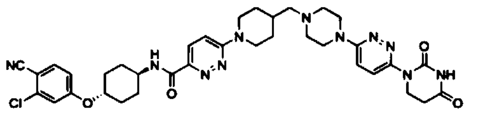 | A | A |
| **PRTA-04** | 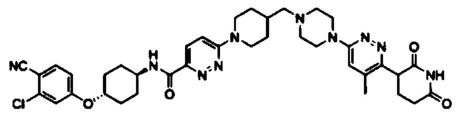 | B | B |
| **PRTA-05** | 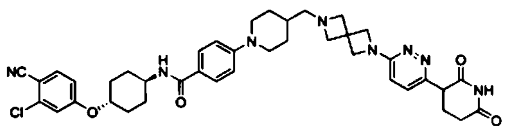 | A | B |
| **PRTA-06** | 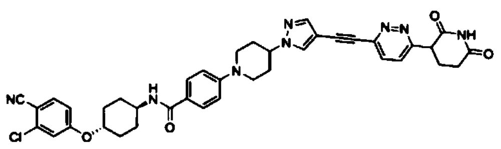 | A | A |

## Claims

**1.** A compound capable of binding to a cereblon (CRBN) protein, wherein the compound is represented by Formula (I):

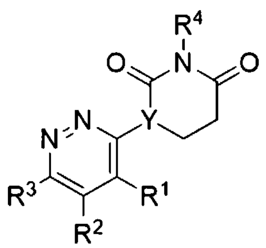

(I),

wherein $R^1$ and $R^2$ are one or more selected from a condensed ring fused to a pyridazine parent core, H, alkyl, OR, F, CN, $CF_3$, $NR_2$, Ph and 4-Py;
Y is one or more selected from CH, CD, CF and N;
$R^3$ is one or more selected from OR, $NR_2$, $CHR_2$ and C≡CR; and
$R^4$ is selected from H or alkyl.

**2.** The compound binding to the CRBN protein according to claim 1, wherein the condensed ring is selected from a fused ring, cyclopentane, cyclohexane, benzene and/or pyrazine.

**3.** The compound binding to the CRBN protein according to claim 1, wherein both $R^1$ and $R^2$ in the compound are selected from alkyl.

**4.** The compound binding to the CRBN protein according to claim 1, wherein $R^1$ and $R^2$ in the compound are not at the same time selected from H, alkyl, OR, F, CN, $CF_3$, $NR_2$, Ph or 4-Py.

**5.** A pharmaceutically acceptable salt of the CRBN protein-binding compound (I) according to claim 1, wherein the pharmaceutically acceptable salt refers to a non-toxic acid or base addition salt prepared from a parent compound.

**6.** A protein degrader, wherein the protein degrader is a target protein ligand (POI) + a Linker + a CRBN protein ligand; and the CRBN protein ligand is a compound (I), and the structural formula of the protein degrader is (II):

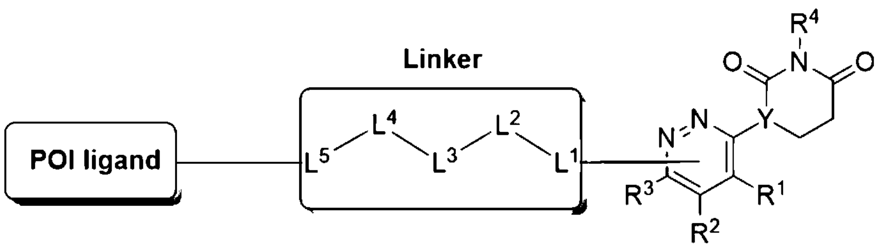

(II)

in the Formula II, the moiety $L^1$ of the Linker is attached to C to which $R^2$ is attached, wherein $R^2$ is $L^1$; $R^3$ is one or more selected from OR, $NR_2$, $CHR_2$, and C≡CR;
in the Formula II, $L^1$ in a Linker substructure is attached to C to which $R^3$ is attached, wherein $R^3$ is $L^1$, and $R^2$ is one or more selected from the condensed ring fused to the pyridazine parent core, H, alkyl, OR, F, CN, $CF_3$, $NR_2$, Ph and 4-Py;
in the Formula II, $R^1$ is one or more selected from the condensed ring fused to the pyridazine parent core, H, alkyl, OR, F, CN, $CF_3$, $NR_2$, Ph and 4-Py;
$R^4$ is selected from H or alkyl; and
Y is one or more selected from CH, CD, CF, $CCH_3$ and N.

**7.** The protein degrader according to claim 6, wherein the moieties $L^1$, $L^3$ and $L^5$ of the Linker are one or more selected from:

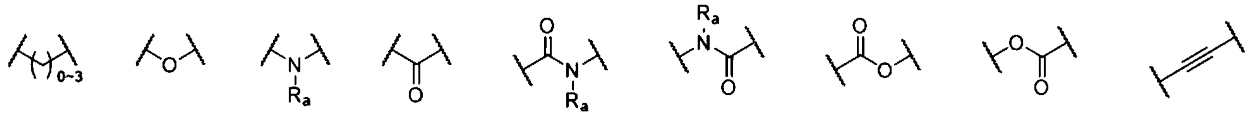

Ra = H, alkyl, $SO_2$(alyl), $SO_2$(aryl)
wherein $R_a$ is one or more selected from H, alkyl, $SO_2$(alyl) or $SO_2$(aryl).

**8.** The protein degrader according to claim 6, wherein the moieties $L^2$ and $L^4$ of the Linker are one or more selected from:

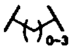

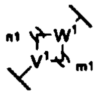

m1, n1 = 1, 2, 3;
m1+n1<=4
m1 = n1 = 2: W1, V1 = CH or N
m1 x n1 <= 3; W1, V1 = CH or N, but cannot be N at the same time

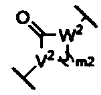

m2 = 1, 2, 3;
W2, V2 = CH or N

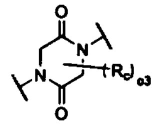

Rc = alkyl
o3 = 0 - 4

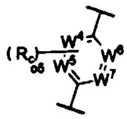

W4, W5, W6, W7 = C or CH or N, and N count <3
Rc = alkyl or halo or CN or $CF_3$, o5 = 0 - 4

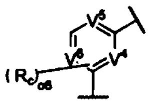

V4, V5, V6 = C or CH or N, and N count <3
Rc = alkyl or halo or CN or $CF_3$, o6 = 0 - 4

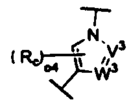 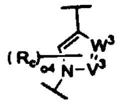

W3, V3 = C or CH or N

Rc = alkyl

o4 = 0 ~ 3

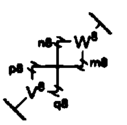

W8, V8 = CH or N
m8, n8, p8, q8 = 1, 2, 3
and m8 + n8 < 5
and p8 + q 8 < 5

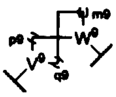

W9, V9 = CH or N
m9, p9, q9 = 1, 2, 3
and p9 + q9 < 5

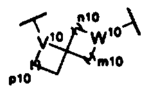

W10, V10 = CH or N
m10, n10, p10 = 1, 2, 3
and m10 + n10 < 5

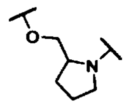 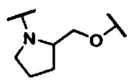

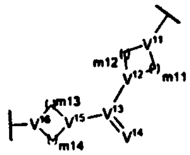

V11, V12, V15, V16 = CH or N
V13 = none, C, CH2, O, $NR_a$
V14 = O, none
Ra = H, alkyl, $SO_2$(alyl), $SO_2$(aryl)

m11, m12, m13, m14 = 1, 2, 3
and m11 + m12 < 5
and m13 + m14 < 5

unless m11 = m12 = 2, V11 and V12 cannot be N at the same time
unless m13 = m14 = 2, V15 and V16 cannot be N at the same time

when V13 = C and V14 = O, V12, V15 = CH, N

when V13 = CH2 and V14 = none, V12 and V15 cannot be N at the same time

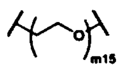 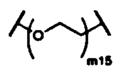

m15 = 1-20

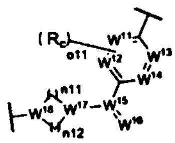 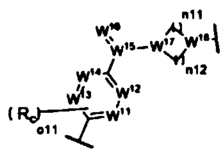

W11, W12, W13, W14 = C, CH, N
W17, W18 = CH, N
W15 = none, C, CH2, O, $NR_a$
W16 = O, none
Ra = H, alkyl, $SO_2$(alyl), $SO_2$(aryl)
Rc = alkyl or halo or CN or $CF_3$
o11 = O - 4
Of the positions W11, W12, W13 and W14, only two may be N

n11, n12 = 1, 2, 3

and n11 + n12 < 5
unless n11 = n12 = 2, V11 and V12 cannot be N at the same time

(continued)

| | |
|---|---|
| **when V13 = 0 and V14 = none, V12 = V15 = CH** | **when W15 = C, W16 = O** |
| **when V13 = V14 = none, V12 and V15 cannot be N at the same time** | **when W15 = CH2, W16 = none** |
| | **when W15 = O and W16 = none, W17 = CH** |

**9.** The protein degrader according to claim 6, wherein the target protein ligand, namely the POI ligand in Formula II, is one or more selected from:

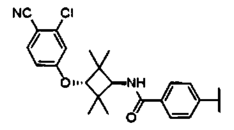
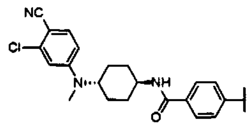
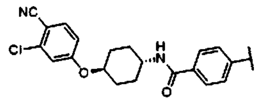
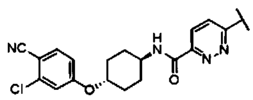
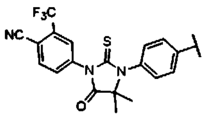
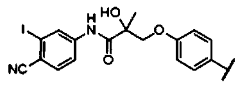
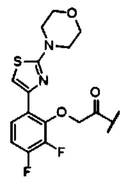
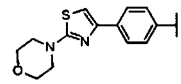
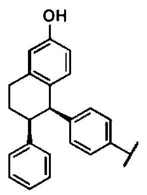
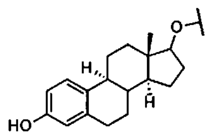
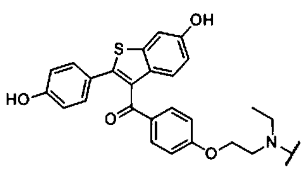
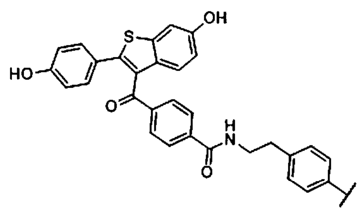
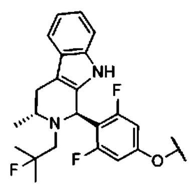
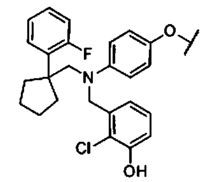
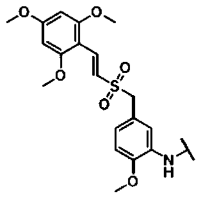
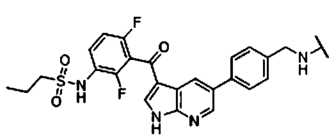
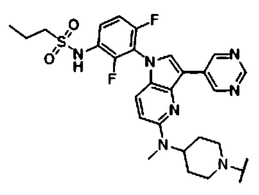
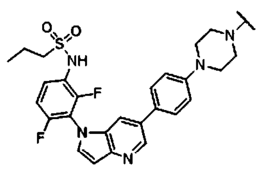
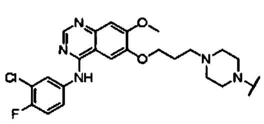
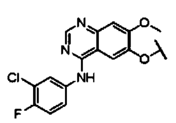
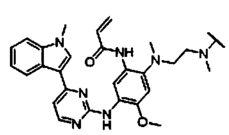
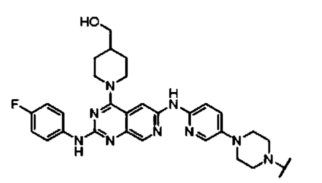

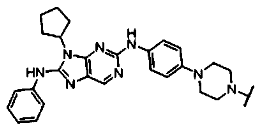
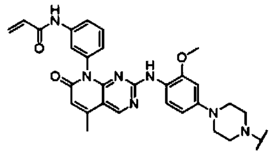
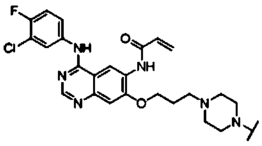

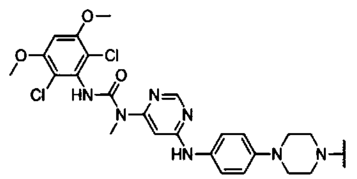
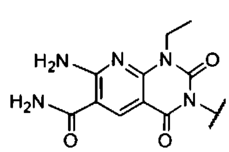
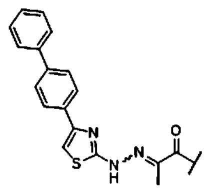
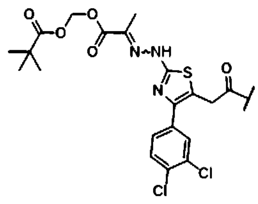

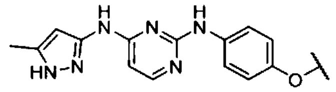
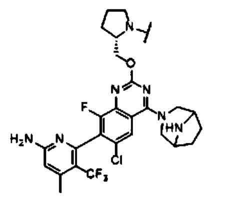
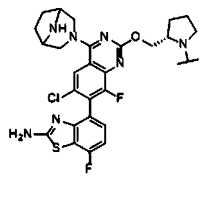

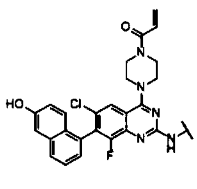
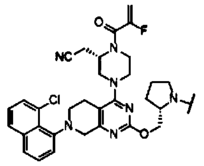
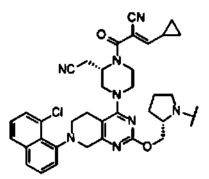
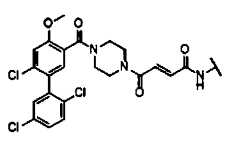

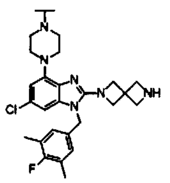
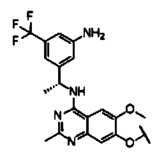
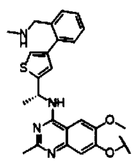
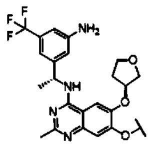
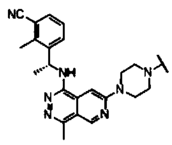

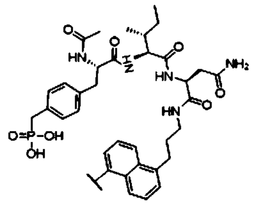
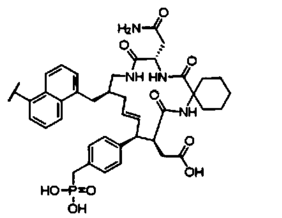
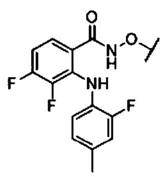
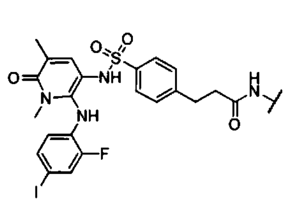

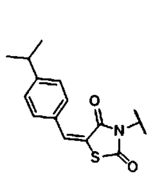
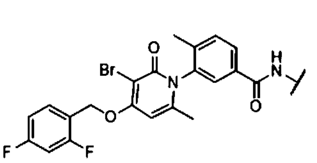
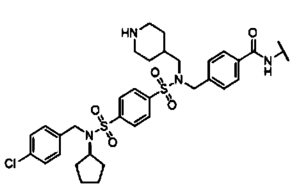
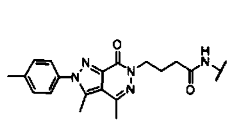

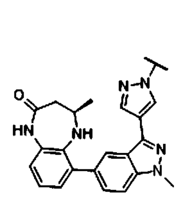
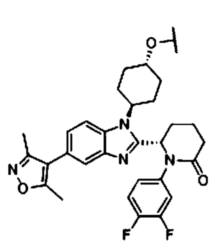
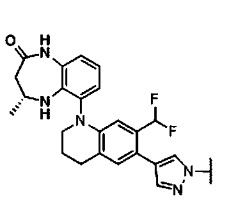
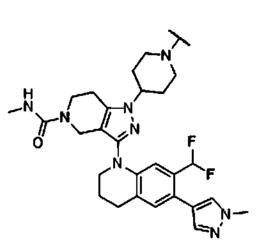

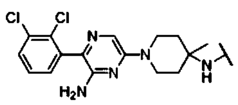
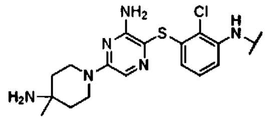
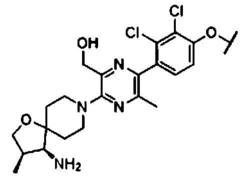
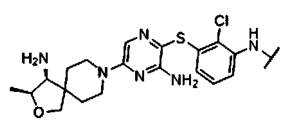

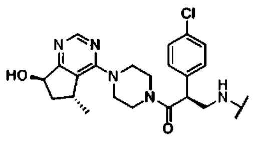
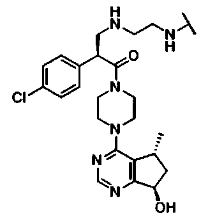

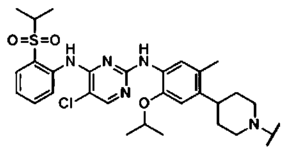
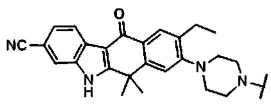
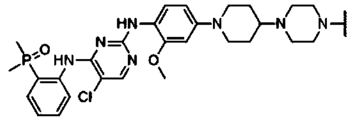

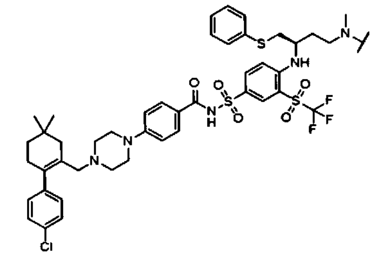
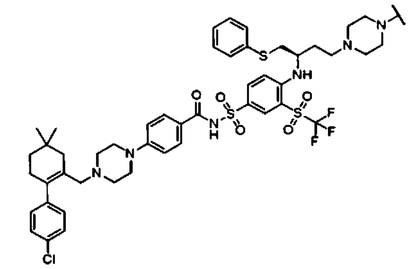

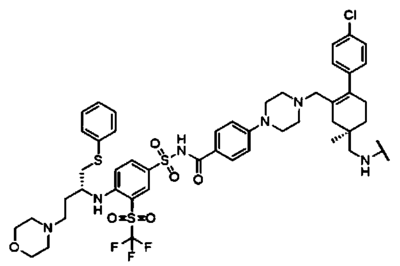
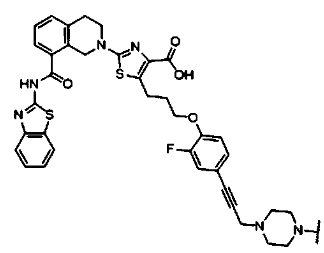

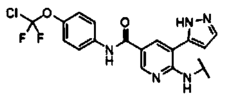
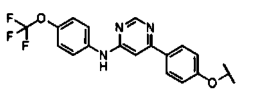
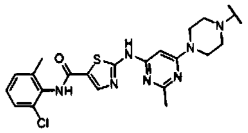
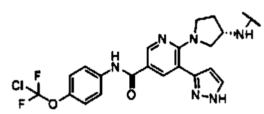

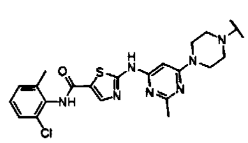
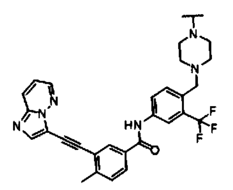
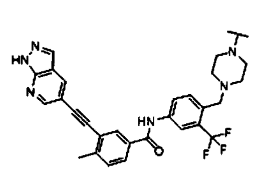

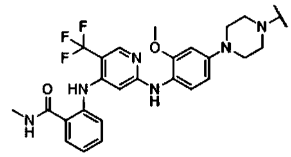
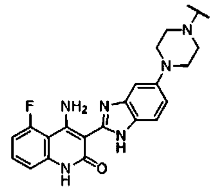
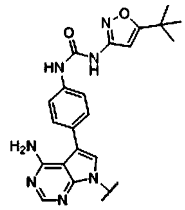

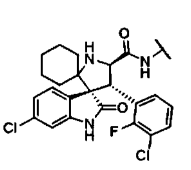
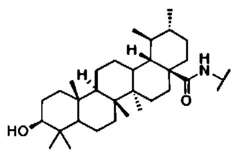
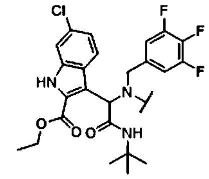
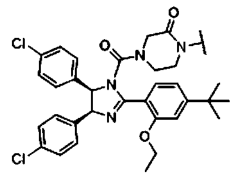

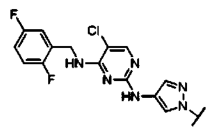
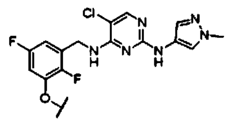
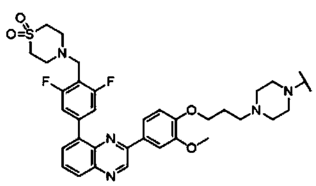
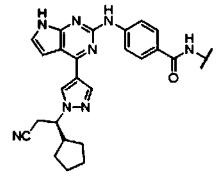

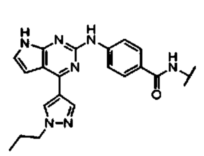
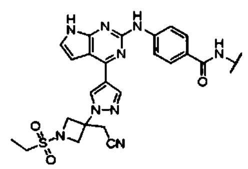

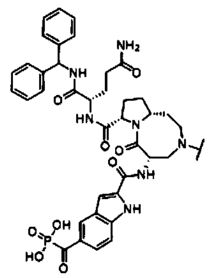
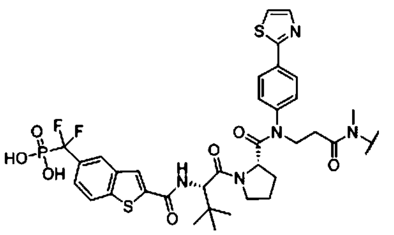
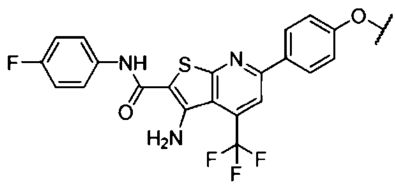

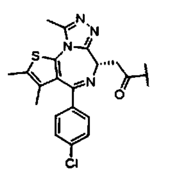
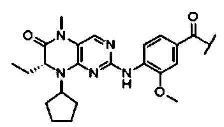
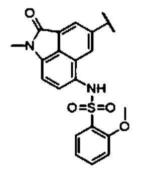
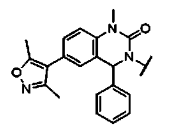
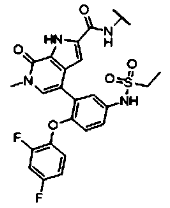

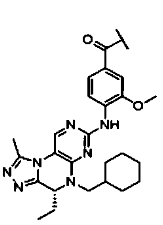

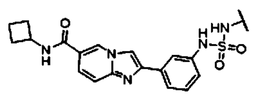
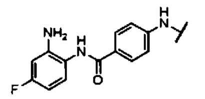
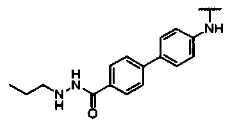
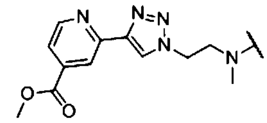

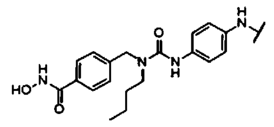
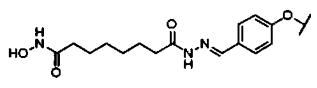
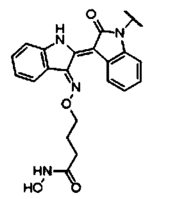
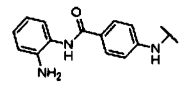

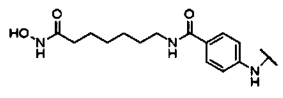

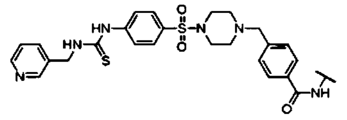
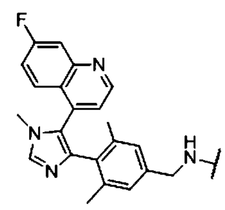
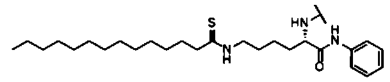

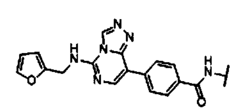
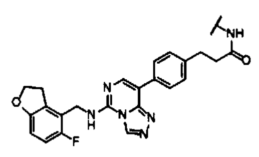
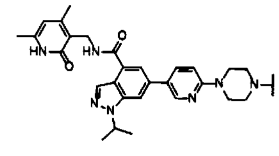
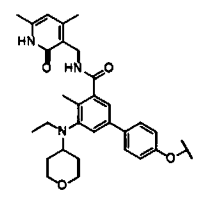

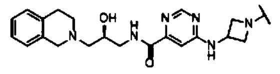

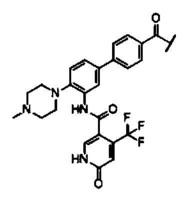
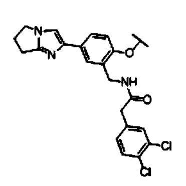
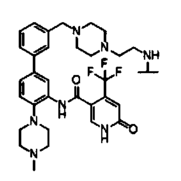
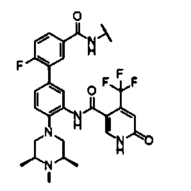

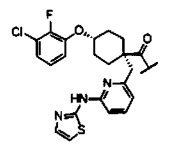
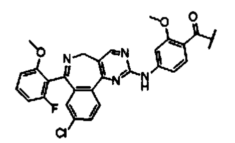
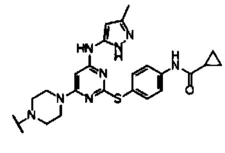
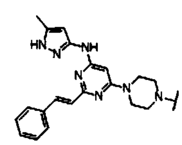

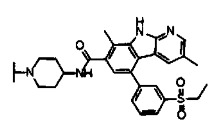
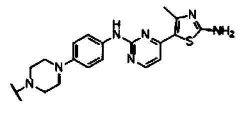
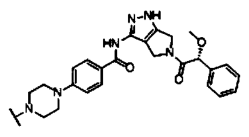
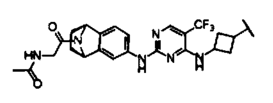

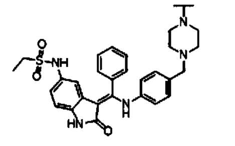
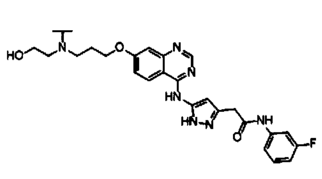
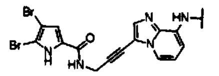

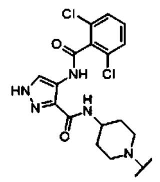
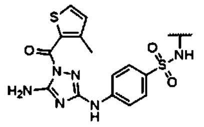
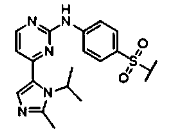

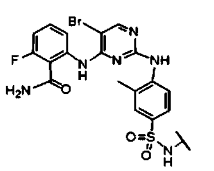
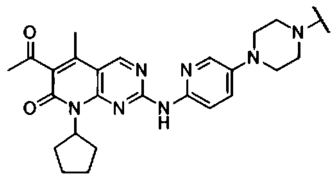
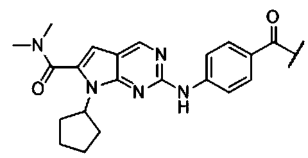

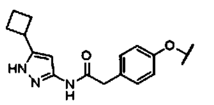
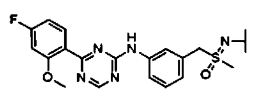
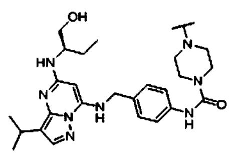
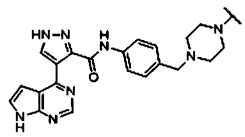

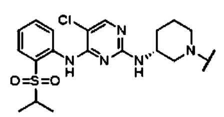
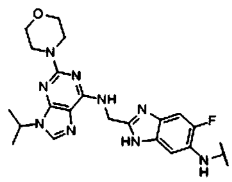
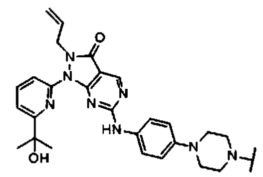

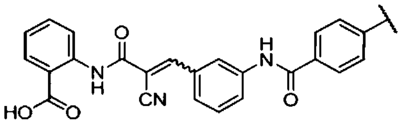
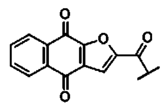

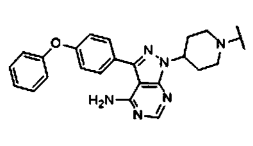
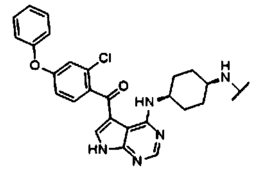
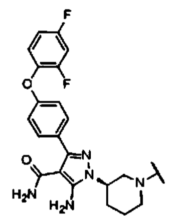
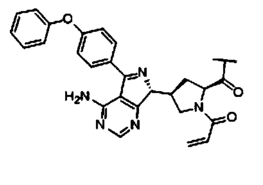

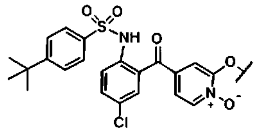
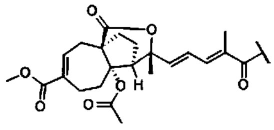
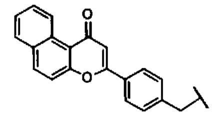

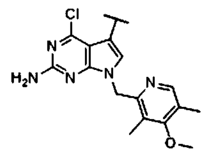
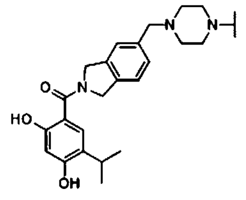
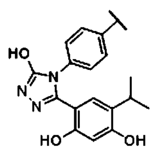

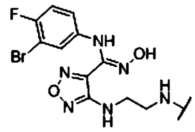
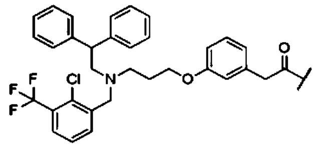
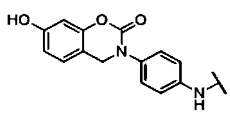

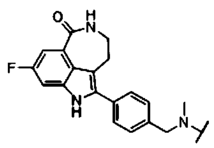
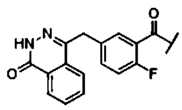
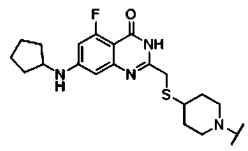

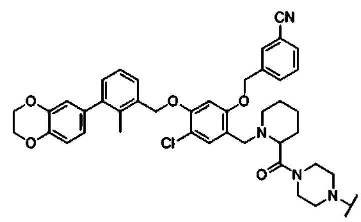
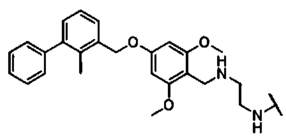
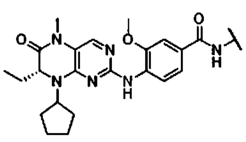

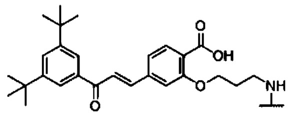
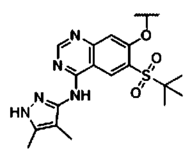
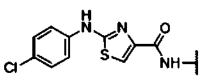
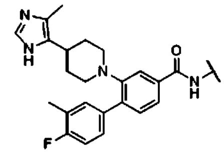

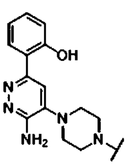
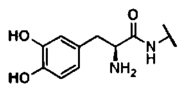
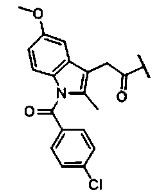
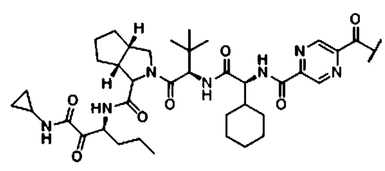

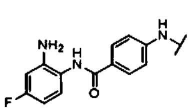
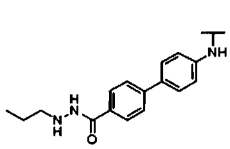
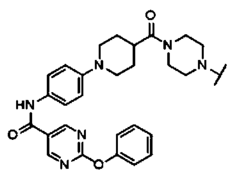
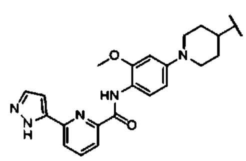

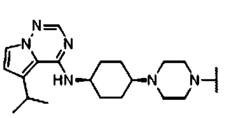

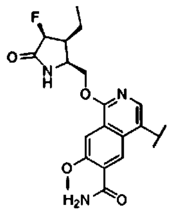
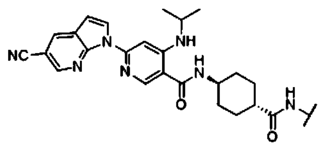
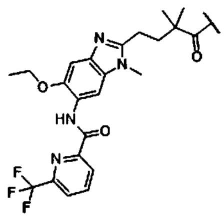

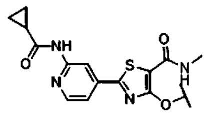
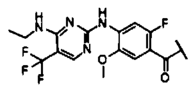
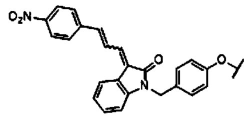
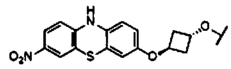

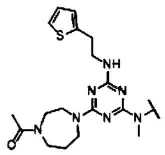
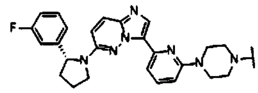
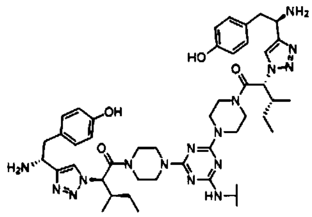

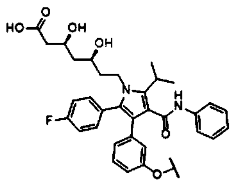
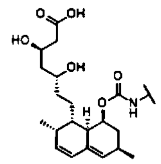
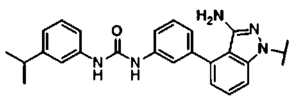
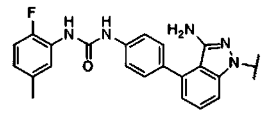
.

**10.** A pharmaceutical composition, comprising a combination of the compound of Formula (I) or the pharmaceutically acceptable salt thereof according to the first aspect and a pharmaceutically acceptable diluent or carrier.

**11.** A pharmaceutical composition for the targeted degradation of a protein, wherein the pharmaceutical composition comprises a combination of the protein degrader (II) or the pharmaceutically acceptable salt thereof according to the second aspect of the present disclosure and a pharmaceutically acceptable diluent or carrier.

**12.** The pharmaceutical composition according to claim 9 or 10, wherein the pharmaceutical composition further comprises a combination of an enantiomer, a diastereomer, a stereoisomer or a pharmaceutically acceptable salt of the compound of Formula (I) and a pharmaceutically acceptable diluent or carrier.

**13.** A regulator for regulating a transcription regulatory factor, wherein the regulator comprises a compound of Formula (1) or a pharmaceutically acceptable salt or a protein degrader (II) thereof, or a pharmaceutically acceptable salt, a hydrate, a solvate, a prodrug, a stereoisomer or a tautomer thereof.

**14.** A method for treating or preventing pathological conditions or symptoms of a disease caused by limiting or inhibiting the expression of IRF4 and Myc, comprising administering to a patient in need thereof an effective amount of at least one of a compound of Formula I or Formula II, or a pharmaceutically acceptable salt, a hydrate, a solvate, a prodrug, a stereoisomer or a tautomer thereof.

**INTERNATIONAL SEARCH REPORT**

International application No. **PCT/CN2024/108163**

**A. CLASSIFICATION OF SUBJECT MATTER**

C07D 401/04(2006.01)i; C07D 401/14(2006.01)i; C07D 487/10(2006.01)i; A61K 31/501(2006.01)i; C07D 495/14(2006.01)i; A61K 31/551(2006.01)i; A61K 31/506(2006.01)i; A61P 19/08(2006.01)i; A61P 7/06(2006.01)i; A61P 37/02(2006.01)i; A61P 31/00(2006.01)i; A61P 29/00(2006.01)i; A61P 13/12(2006.01)i; A61P 3/14(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS; DWPI; CNKI; ISI Web of science; REGISTRY (STN); CAPLUS (STN): 中国海洋大学, 秦冲, 王霄, CRBN蛋白, 降解剂, 哒嗪, pyridazine, protein, degradation

**C. DOCUMENTS CONSIDERED TO BE RELEVANT**

| Category* | Citation of document, with indication, where appropriate, of the relevant passages | Relevant to claim No. |
|---|---|---|
| X | WO 2023086399 A1 (NURIX THERAPEUTICS, INC. et al.) 19 May 2023 (2023-05-19) abstract, claims 1 and 34, and description, pages 11, 191 and 425 | 1-8, 10-14 |
| Y | WO 2023086399 A1 (NURIX THERAPEUTICS, INC. et al.) 19 May 2023 (2023-05-19) abstract, claims 1 and 34, and description, pages 11, 191 and 425 | 9 |
| Y | CN 116003418 A (GLUETACS THERAPEUTICS (SHANGHAI) CO., LTD.) 25 April 2023 (2023-04-25) abstract, and claims 1, 20 and 23 | 9 |
| X | CN 115485278 A (BEIGENE LTD.) 16 December 2022 (2022-12-16) claims 1-34 | 1-8, 10-14 |
| X | CN 116261458 A (UPTRA CO., LTD.) 13 June 2023 (2023-06-13) claims 1-17 | 1-8, 10-14 |
| PX | CN 117126133 A (OCEAN UNIVERSITY OF CHINA) 28 November 2023 (2023-11-28) claims 1-14 | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 October 2024** | **01 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/108163**

**C. DOCUMENTS CONSIDERED TO BE RELEVANT**

| Category* | Citation of document, with indication, where appropriate, of the relevant passages | Relevant to claim No. |
|---|---|---|
| PX | WO 2024020084 A1 (BRISTOL-MYERS SQUIBB CO. et al.) 25 January 2024 (2024-01-25)<br>claim 1, description, page 114, and compound P-423 | 1-14 |
| E | WO 2024188209 A1 (GLUETACS THERAPEUTICS (SHANGHAI) CO., LTD.) 19 September 2024 (2024-09-19)<br>claim 1, and description, pages 194-658 and 673-702 | 1-14 |
| A | CN 113166076 A (OTSUKA PHARMACEUTICAL CO., LTD.) 23 July 2021 (2021-07-23)<br>claim 7 | 1-14 |
| A | ACS. "RN2925081-68-9 etc."<br>*STN Registry*, 01 June 2023 (2023-06-01),<br>pp. 1-2 | 1-9 |

Form PCT/ISA/210 (second sheet) (July 2022)

**INTERNATIONAL SEARCH REPORT**

International application No.
**PCT/CN2024/108163**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

   The technical solution of claim 14 includes a disease treatment method as defined in PCT Rule 39.1(iv). The present search is carried out on the basis of the technical solution of the use for the preparation of corresponding pharmaceuticals.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/108163**

| Patent document cited in search report | Publication date (day/month/year) | Patent family member(s) | Publication date (day/month/year) |
|---|---|---|---|
| WO 2023086399 A1 | 19 May 2023 | US 2024018162 A1 | 18 January 2024 |
| | | US 12091426 B2 | 17 September 2024 |
| | | TW 202334170 A | 01 September 2023 |
| | | KR 20240101654 A | 02 July 2024 |
| | | EP 4430052 A1 | 18 September 2024 |
| CN 116003418 A | 25 April 2023 | AU 2022370021 A1 | 09 May 2024 |
| | | CA 3235512 A1 | 27 April 2023 |
| | | KR 20240110576 A | 15 July 2024 |
| | | WO 2023066350 A1 | 27 April 2023 |
| | | EP 4421071 A1 | 28 August 2024 |
| CN 115485278 A | 16 December 2022 | JP 2023523130 A | 02 June 2023 |
| | | KR 20230003161 A | 05 January 2023 |
| | | EP 4146655 A1 | 15 March 2023 |
| | | EP 4146655 A4 | 31 July 2024 |
| | | TW 202142542 A | 16 November 2021 |
| | | US 2023167118 A1 | 01 June 2023 |
| | | WO 2021219070 A1 | 04 November 2021 |
| CN 116261458 A | 13 June 2023 | WO 2023017446 A1 | 16 February 2023 |
| | | KR 20230024250 A | 20 February 2023 |
| | | KR 102604801 B1 | 22 November 2023 |
| | | EP 4384520 A1 | 19 June 2024 |
| | | JP 2023540728 A | 26 September 2023 |
| | | JP 7555155 B2 | 24 September 2024 |
| | | EP 4157850 A1 | 05 April 2023 |
| | | KR 20240060721 A | 08 May 2024 |
| | | US 2023242541 A1 | 03 August 2023 |
| | | US 11939334 B2 | 26 March 2024 |
| | | JP 2024530495 A | 21 August 2024 |
| | | KR 20230024267 A | 20 February 2023 |
| | | KR 102604803 B1 | 22 November 2023 |
| | | US 2023219966 A1 | 13 July 2023 |
| | | US 11912710 B2 | 27 February 2024 |
| | | JP 2023540729 A | 26 September 2023 |
| | | JP 7555156 B2 | 24 September 2024 |
| | | EP 4384521 A1 | 19 June 2024 |
| | | KR 20230024251 A | 20 February 2023 |
| | | KR 102604802 B1 | 22 November 2023 |
| | | WO 2023018238 A1 | 16 February 2023 |
| | | KR 20230024302 A | 20 February 2023 |
| | | KR 102662205 B1 | 30 April 2024 |
| | | EP 4157849 A1 | 05 April 2023 |
| | | WO 2023018237 A1 | 16 February 2023 |
| | | WO 2023018236 A1 | 16 February 2023 |
| | | JP 2024530195 A | 16 August 2024 |
| | | WO 2023017442 A1 | 16 February 2023 |
| CN 117126133 A | 28 November 2023 | None | |
| WO 2024020084 A1 | 25 January 2024 | US 2024109881 A1 | 04 April 2024 |
| | | TW 202412785 A | 01 April 2024 |
| WO 2024188209 A1 | 19 September 2024 | None | |

Form PCT/ISA/210 (patent family annex) (July 2022)

**INTERNATIONAL SEARCH REPORT**
**Information on patent family members**

International application No.
**PCT/CN2024/108163**

| Patent document cited in search report | Publication date (day/month/year) | Patent family member(s) | Publication date (day/month/year) |
|---|---|---|---|
| CN 113166076 A | 23 July 2021 | WO 2020111263 A1 | 04 June 2020 |
| | | TW 202426426 A | 01 July 2024 |
| | | TW 202039446 A | 01 November 2020 |
| | | EP 3887358 A1 | 06 October 2021 |
| | | KR 20210097148 A | 06 August 2021 |
| | | JP 2022509848 A | 24 January 2022 |
| | | JP 7539377 B2 | 23 August 2024 |
| | | US 2021403456 A1 | 30 December 2021 |
| | | CA 3118071 A1 | 04 June 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JOHN LB** ; **WARD AC**. The Ikaros gene family: transcriptional regulators of hematopoiesis and immunity [J. *Molecular Immunology*, 2011, vol. 48 (9), 1272-1278
- **DIJON M** ; **BARDIN F** ; **MURATI A et al.** The role of Ikaros in human erythroid differentiation [J. *Blood*, 2008, vol. 111 (3), 1138-1146